# EUROPEAN PATENT SPECIFICATION

(11) **EP 3 921 420 B1**
(45) Date of publication and mention of the grant of the patent: **08.04.2026**
(21) Application number: 20752549.4
(22) Date of filing: 10.02.2020
(51) Int. Cl.: A61K 9/00, A61K 47/34, A61K 47/36, C12N 15/13, C07H 21/02, C07K 16/22, A61K 39/395, C08B 37/08, C08L 5/08, C08L 89/00, D01D 5/00

(54) **DRUG DELIVERY COMPOSITIONS FOR OCULAR ADMINISTRATION OF THERAPEUTICS AND METHODS OF USE THEREOF**
ARZNEIMITTELABGABEZUSAMMENSETZUNGEN ZUR OKULAREN VERABREICHUNG VON THERAPEUTIKA UND VERWENDUNGSVERFAHREN DAFÜR
COMPOSITIONS D'ADMINISTRATION DE MÉDICAMENT POUR L'ADMINISTRATION OCULAIRE D'AGENTS THÉRAPEUTIQUES ET LEURS MÉTHODES D'UTILISATION

(30) Priority: 08.02.2019 US 201962803388 P
(43) Date of publication of application: 15.12.2021
(73) Proprietor: Ohio State Innovation Foundation, Columbus, OH 43210 (US)
(72) Inventor: JIANG, Pengfei, Columbus, Ohio 43220 (US); REILLY, Katelyn Elizabeth, Arlington, Ohio 43220 (US); OHR, Matthew P., Powell, Ohio 43065 (US); LANNUTTI, John, Grove City, OH 43123 (US)
(74) Representative: Berggren Oy
(86) International application number: PCT/US2020/017523
(87) International publication number: WO 2020/163871

(56) References cited:
- US-A- 5 516 522
- US-A1- 2011 038 939
- US-A1- 2011 264 190
- US-A1- 2017 196 982
- US-B2- 7 838 037
- JIANG PENGFEI ET AL: "Injectable biodegradable bi-layered capsule for sustained delivery of bevacizumab in treating wet age-related macular degeneration", JOURNAL OF CONTROLLED RELEASE, ELSEVIER, AMSTERDAM, NL, vol. 320, 23 January 2020 (2020-01-23), pages 442 - 456, XP086115489, ISSN: 0168-3659, [retrieved on 20200123], DOI: 10.1016/J.JCONREL.2020.01.036
- NABA ELSAID ET AL: "PLGA Microparticles Entrapping Chitosan-Based Nanoparticles for the Ocular Delivery of Ranibizumab", MOLECULAR PHARMACEUTICS, vol. 13, no. 9, 22 June 2016 (2016-06-22), US, pages 2923 - 2940, XP055563509, ISSN: 1543-8384, DOI: 10.1021/acs.molpharmaceut.6b00335
- DE SOUZA SARAH OLIVEIRA ET AL: "Biodegradable core-shell electrospun nanofibers containing bevacizumab to treat age-related macular degeneration", JOURNAL OF MATERIALS SCIENCE: MATERIALS IN MEDICINE, SPRINGER US, NEW YORK, vol. 29, no. 11, 3 November 2018 (2018-11-03), pages 1 - 11, XP036652818, ISSN: 0957-4530, [retrieved on 20181103], DOI: 10.1007/S10856-018-6187-5

## Description

### CROSS REFERENCE TO RELATED APPLICATIONS

This application claims the benefit of priority to United States Provisional Patent Application No. 62/803,388, filed February 8, 2019.

### TECHNICAL FIELD

This disclosure related drug delivery compositions, and more particularly to compositions containing one or more multi-layered drug delivery capsules for delivery of therapeutic agents to the eye.

### BACKGROUND

Age-related macular degeneration (AMD) is the fourth most common cause of blindness after cataracts, preterm birth, and glaucoma in the world. There are more than 11 million people diagnosed with wet AMD in the United States. It is estimated that this number will double in 30 years. Accordingly, much work has been done understanding disease pathogenesis and developing therapeutic methods. It is widely noted that overexpression of vascular endothelial growth factor (VEGF) along with aging stimulates neovascularization in the choroid, which leads to irreversible damage to the retina during bleeding and scarring of newly formed blood vessels. The current gold standard treatment for wet AMD is a monthly intravitreal injection of anti-VEGF such as bevacizumab or ranibizumab to inhibit VEGF and to prevent angiogenesis. However, frequent injections often lead to infection, elevated intraocular pressure and rhegmatogenous retinal detachment, as well as issues with patient compliance.

Recently, there have been reports of novel devices such as implants and micro/nanoparticles for a long-term drug delivery in the eye. Unfortunately, such implants require surgical procedures for implantation and removal. Moreover, the presently known implant devices tend to be off-target and lower the drug efficacy. Although microparticles or nanoparticles have a relatively small size appropriate for injection into the eye with a 30-gauge needle, currently described microparticles or nanoparticles release therapeutic agents such as anti-VEGF therapeutics over a rapid window of release due to the biodegradation of known particle compositions in the first three months.

US 5,516,522 discloses a porous drug delivery device for controllably releasing a pharmacological agent which comprises a hollow tube closed at both ends, the tube formed of a mixture of polycaprolactone and a pore-creating agent, and a pharmacological agent filled into the hollow tube.

Accordingly, despite significant efforts directed to treatment of AMD or other ophthalmological disorders, there remains a scarcity of methods and compositions that minimize deleterious side-effects of currently available treatment regiments. Moreover, there is a need for drug delivery systems and compositions that can be biodegradable and control the drug release up to nine months or more after an intravitreal injection. There remains a need for improved therapeutic approaches for the treatment of AMD and other ocular diseases requiring delivery of therapeutic agents to directly to the eye. These needs and other needs are satisfied by the present disclosure.

### SUMMARY

In accordance with the purpose(s) of the present disclosure, as embodied and broadly described herein, the disclosure, in one aspect, relates to compositions, devices, and processes for delivery of protein therapeutics, e.g., intravitreal delivery of a protein therapeutic to the eye. The disclosed drug delivery compositions comprise a capsule having a bi-layered wall and a therapeutic agent contained therein. In a further aspect, the present disclosure relates to the drug delivery compositions for use in therapy, e.g. in methods of treating an ophthalmological disease or disorder.

Thus in one aspect, a drug delivery composition is provided comprising:
one or more capsules each having a tubular shape with two ends that are closed, wherein each of the one or more capsules independently comprises a bi-layered wall and at least one luminal compartment; and
one or more therapeutic agents each present within one or more of the at least one luminal compartments;
wherein each bi-layered wall comprises an inner-layer and an outer layer;
wherein the inner layer comprises chitosan; and
wherein the outer layer independently comprises poly(ε-caprolactone) (PCL); and
wherein the one or more therapeutic agents have a net negative charge at any pH within pH 6.0 to pH 7.4.

In some embodiments, the drug delivery composition may comprise two or more capsules. In some embodiments, a different therapeutic agent is initially present within each of the two or more capsules. In other embodiments, the same therapeutic agent is initially present within each of the two or more capsules.

In some embodiments, at least one of the one or more capsules comprises two or more luminal compartments. In some embodiments, a different therapeutic agent is initially present within each of the two or more luminal compartments. In other embodiments, the same therapeutic agent is initially present within each of the two or more luminal compartments.

In some aspects, the first polymer may additionally comprise a polyethyleneimine, a protamine, a polypropylimine, a poly-L-lysine, a poly-L-arginine, a poly-D-lysine, a poly-D-arginine, a cellulose, a dextran, a poly(amidoamine), poly(2-(dimethylamino)ethyl methacrylate), derivatives thereof, or combinations thereof. In some embodiments, the first polymer comprises fibers having an average diameter from about 50 nm to about 1000 nm.

In some aspects, the second polymer may additionally comprise a poly-lactic acid (PLA), a poly-glycolic acid (PGA), a poly-lactide-co-glycolide (PLGA), a polyester, a poly(ortho ester), a poly(phosphazine), a poly(phosphate ester), a gelatin, a collagen, a polyethylene glycol (PEG), derivatives thereof, or combinations thereof. In some embodiments, the second polymer comprises fibers having an average diameter from about 100 nm to about 2000 nm.

In some aspects, the one or more capsules each independently have a length from about 0.1 cm to about 5 cm. In some embodiments, the one or more capsules each independently have an inner diameters from about 100 µm to about 2000 µm. In some embodiments, the one or more capsules each independently have an outer diameter from about 50 µm to about 300 µm greater than the inner diameter of the same capsule. In some embodiments, each bi-layered wall has a wall thickness from about 25 µm to about 150 µm. In some embodiments, each outer layer may further comprise pores having an average pore diameter from about 100 nm to about 10000 nm.

In some embodiments, each of the one or more drug delivery capsules have a surface charge measured as a zeta potential at pH 7.4 of from about -25 mV to about 25 mV. The one or more therapeutic agents each have a net negative charge within a pH range from about 6.0 to about 7.4.

In some embodiments, at least one of the one or more therapeutic agents is an anti-VEGF agent. In some embodiments, the anti-VEGF agent is a therapeutic antibody, for example bevacizumab, ranibizumab, IBI305, or combinations thereof. In some embodiments, the anti-VEGF agent is a VEGF decoy receptor, for example aflibercept. In some embodiments, the anti-VEGF agent is a tyrosine kinase inhibitor, for example lapatinib, sunitinib, axitinib, pazopanib, or combinations thereof.

In some embodiments, the one or more therapeutic agents may comprise an anti-inflammatory agent, such as cyclosporine, a steroid, or a non-steroidal anti-inflammatory drug, an antimicrobial agent, an immunomodulating drug, an ocular hypotensive agent, a neuroprotective agent, a gene therapy, a viral vector therapy, an alpha-adrenergic agonist, a beta-adrenergic agonist, or combinations thereof.

In another aspect, the composition is for use in a method for treating an ophthalmological disorder in a subject in need thereof comprising injecting into the eye of the subject a therapeutically effective amount of the drug delivery composition described herein. In some embodiments, the ophthalmological disorder may comprise acute macular neuroretinopathy; Behcet's disease; neovascularization, including choroidal neovascularization; diabetic uveitis; histoplasmosis; infections, such as fungal or viral-caused infections; macular degeneration, such as acute macular degeneration (AMD), including wet AMD, non-exudative AMD and exudative AMD; edema, such as macular edema, cystoid macular edema and diabetic macular edema; multifocal choroiditis; ocular trauma which affects a posterior ocular site or location; ocular tumors; retinal disorders, such as central retinal vein occlusion, diabetic retinopathy (including proliferative diabetic retinopathy), proliferative vitreoretinopathy (PVR), retinal arterial occlusive disease, retinal detachment, uveitic retinal disease; sympathetic ophthalmia; Vogt Koyanagi-Harada (VKH) syndrome; uveal diffusion; a posterior ocular condition caused by or influenced by an ocular laser treatment; posterior ocular conditions caused by or influenced by a photodynamic therapy, photocoagulation, radiation retinopathy, epiretinal membrane disorders, branch retinal vein occlusion, anterior ischemic optic neuropathy, non-retinopathy diabetic retinal dysfunction, retinitis pigmentosa, a cancer, and glaucoma. In some embodiments, the ophthalmological disorder comprises wet age-related macular degeneration (wet AMD), neovascularization, or macular edema. In some embodiments, injecting the described compositions into the eye of a subject comprises injecting into the vitreous chamber of the eye. In other embodiments, injecting the described compositions into the eye of a subject comprises an intravitreal injection, a subconjunctival injection, a subtenon injection, a retrobulbar injection, or a suprachoroidal injection.

Also provided are methods of creating the one or more capsules as used in the drug delivery compositions described herein, the method comprising:
forming a first layer of the first polymer on a conductive rod, wherein forming the first layer comprises electrospinning using a first solution comprising the first polymer in at least one organic solvent, and wherein electrospinning is performed using a voltage difference of about 10 kV to about 30 kV; and
forming a second layer of the second polymer on the first layer, wherein forming the second layer comprises electrospinning onto the formed first layer a second solution comprising the first polymer and optionally a porogen, and wherein electrospinning is performed using a voltage difference of about 10 kV to about 30 kV.

Also disclosed are kits comprising one of: (a) a disclosed drug delivery composition; (b) a disclosed drug delivery composition in a sterile package; or (c) a pre-filled syringe or needle comprising a disclosed drug delivery composition, and instructions for administering the drug delivery composition to treat an ophthalmological disease or disorder as described herein.

### BRIEF DESCRIPTION OF THE FIGURES

Many aspects of the present disclosure can be better understood with reference to the following drawings. The components in the drawings are not necessarily to scale, emphasis instead being placed upon clearly illustrating the principles of the present disclosure. Moreover, in the drawings, like reference numerals designate corresponding parts throughout the several views.

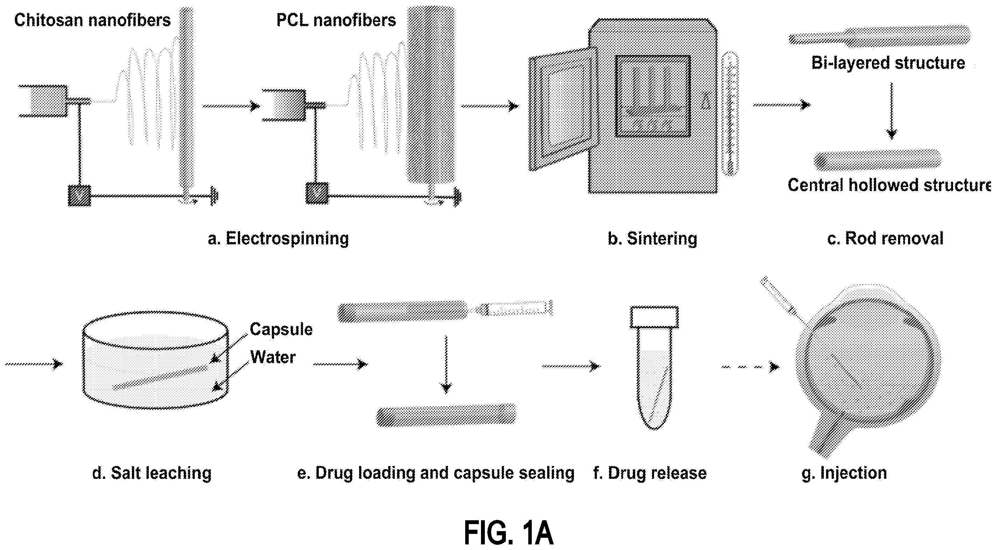

**FIG. 1A** shows a representative disclosed process comprising the following steps: a) two layers of chitosan and PCL nanofibers collected on the rotary rod using electrospinning; b) bi-layered coated rod sintered at 100 °C in the vacuum oven for about 3 hours; c) rod removal to create a central hollowed cylinder; d) porous structure in PCL layer generated by salt leaching; and e) therapeutic loading to the capsule followed by end sealing. The prepared bi-layered capsules can then be utilized in studies, such as (such as step f) assessment of drug release into an appropriate buffer, e.g., PBS at 37 °C; or used for delivery of a loaded drug to a suitable target, e.g., the eye via (such as step g) intravitreal injection.

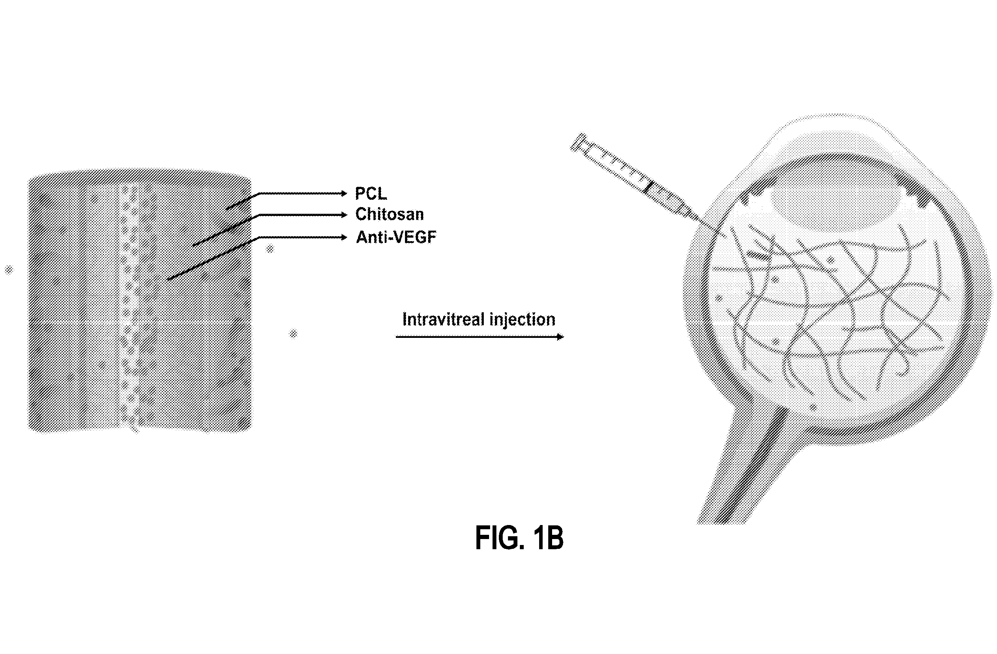

**FIG. 1B** shows a schematic cross-sectional representation of a disclosed bi-layer capsule and a schematic representation of intra-vitreal injection of a disclosed bi-layer capsule.

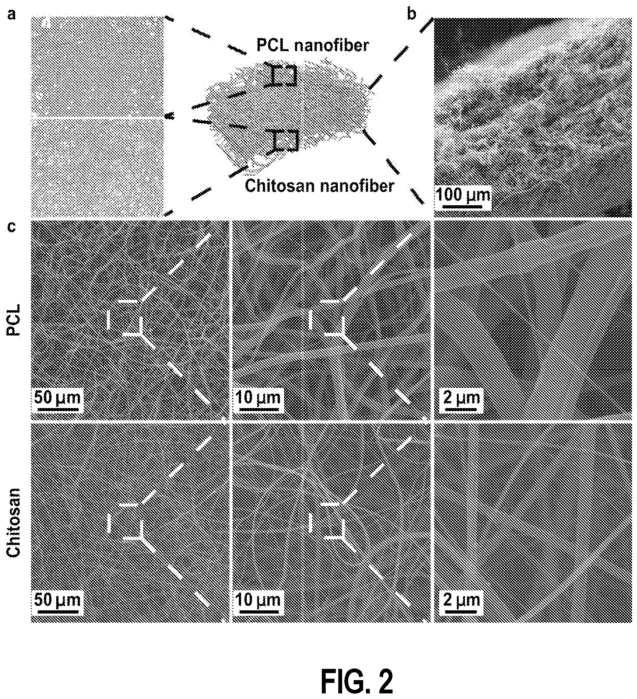

**FIG. 2** shows a representative schematic representation of a chitosan and PCL fibrous mat formed using the disclosed techniques (see panel A). The FIG. also shows representative scanning electron micrograph (SEM) images as follows: (panel B) representative SEM image of cross-section of bi-layered chitosan-PCL fibrous mat; and (panel C) representative SEM images of PCL and chitosan nanofibrous layer with diameter of 932.57 ± 399.42 nm and 331.61 ± 186.19 nm, respectively.

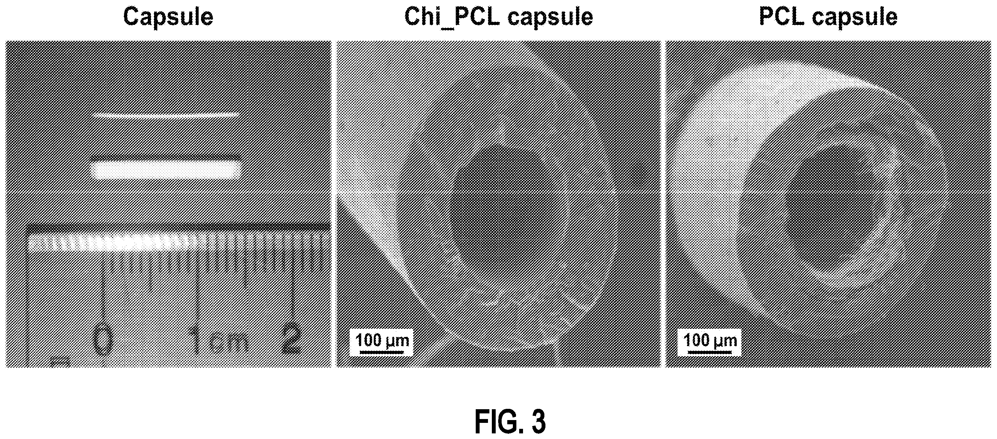

**FIG. 3** shows representative photographic images of disclosed bi-layer capsules. The left pane of the FIG. shows a photograph image of two capsules, one having a diameter of 1.645 mm and the other having a diameter of 260 µm. The middle pane of the FIG. shows a representative SEM image of 260 µm inner diameter PCL mono-layered capsule. The right pane shows a representative SEM image of a chitosan-PCL bi-layered capsule with 89.85 ± 4.27 µm membrane thickness. The image in right pane shows in this representative example that a layer of chitosan fibrous mat is attached to the PCL outer layer, and that the chitosan layer takes approximately 25% of whole thickness of wall.

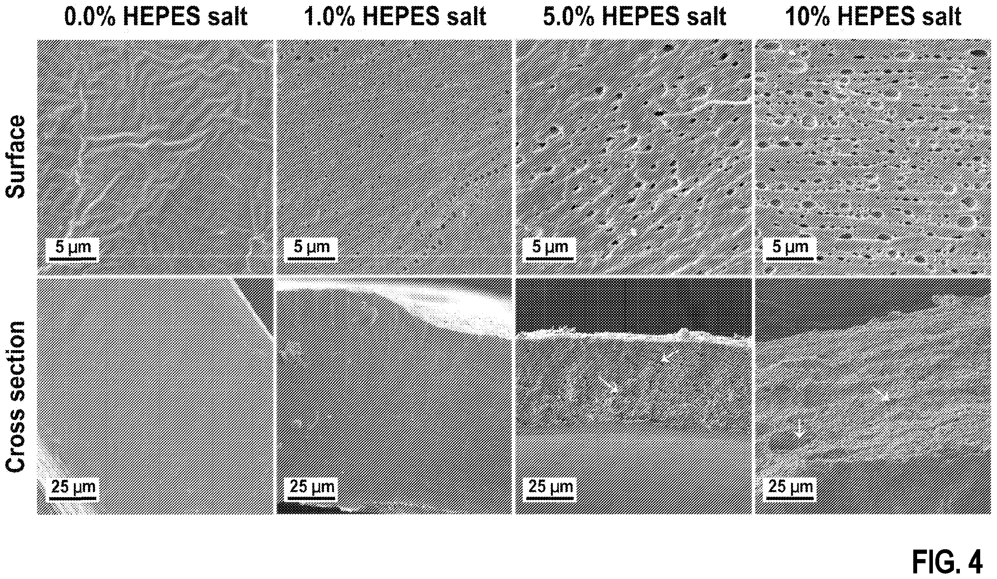

**FIG. 4** shows representative images of disclosed PCL membranes prepared using the indicated concentrations of HEPES salt, with the images showing the surface or cross-sectional view of a disclosed PCL membrane as indicated. The images show that increasing the ratio of HEPES sodium salt resulted in larger pores on PCL membrane. Interconnecting pores can be overserved inside the membrane with salt concentration above 5.0%. Arrow: characteristic interconnecting pores inside the PCL films after salt leaching. Each image has a scalar bar show in the lower left corner of the image.

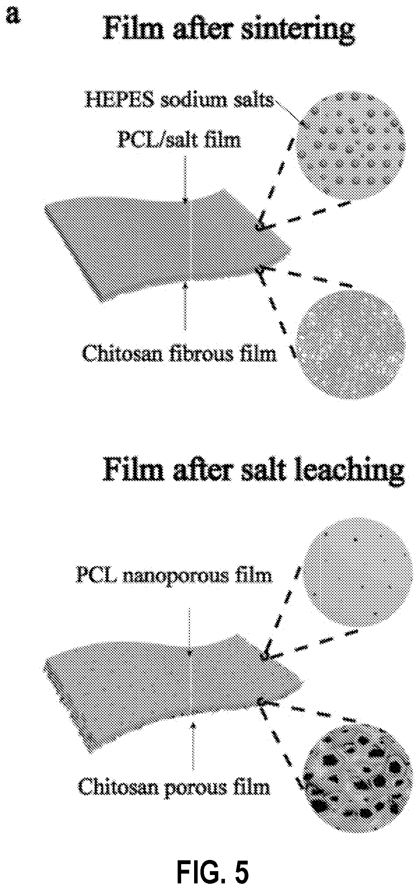

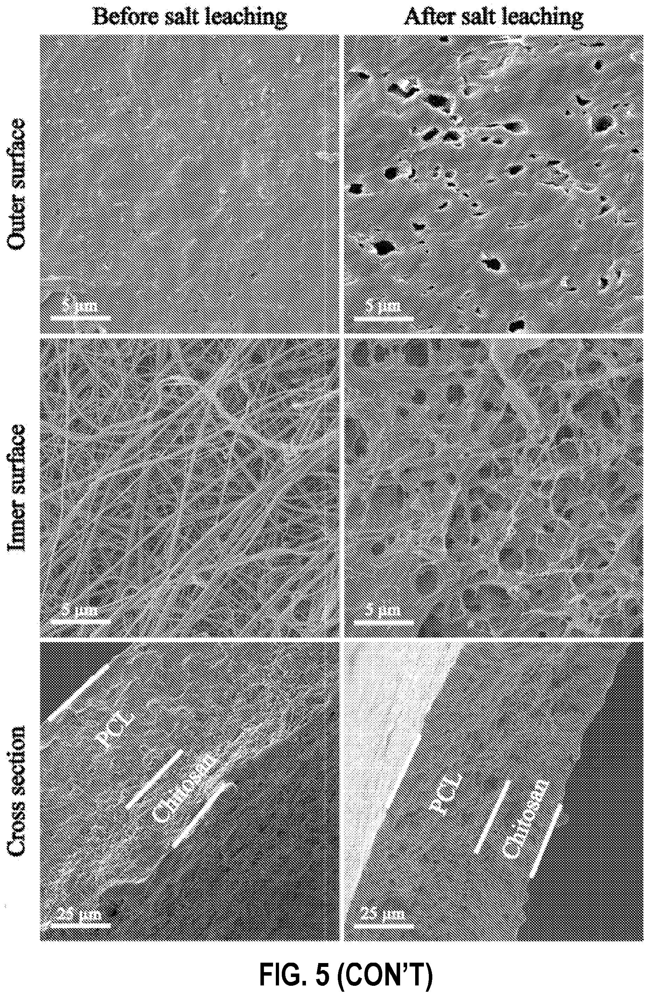

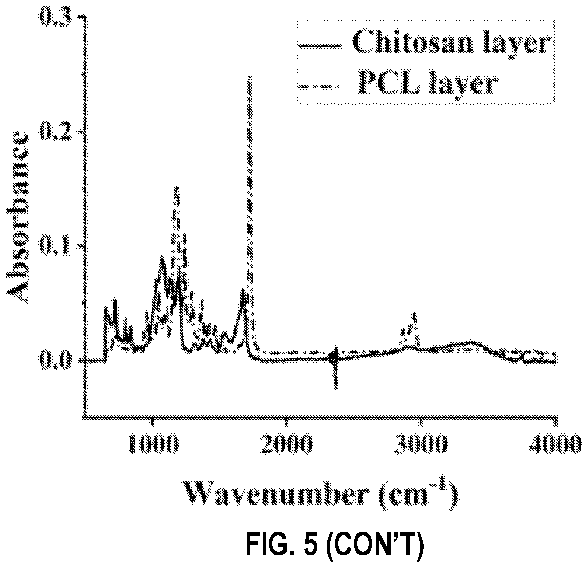

**FIG. 5** shows images and data pertaining to characterization of a disclosed bi-layer capsule. Panel **a** shows a representative scheme of a bi-layered structure after salt leaching and washing. Panel **b** shows a representative SEM image of a disclosed bi-layered membrane before and after salt leaching. As shown, a porous structure was generated by salt leaching and chitosan fibrous structure lost after washing with saturated sodium bicarbonate solution. A porous bi-layered structure was observed in its cross-section. Panel **c** shows a representative FTIR spectrum of chitosan layer and PCL layer after salt leaching. As shown, a significant peak at 1752 cm⁻¹ was assigned to the carbonyl group in PCL. A broad group at 3478 cm⁻¹ was the hydroxyl group in chitosan.

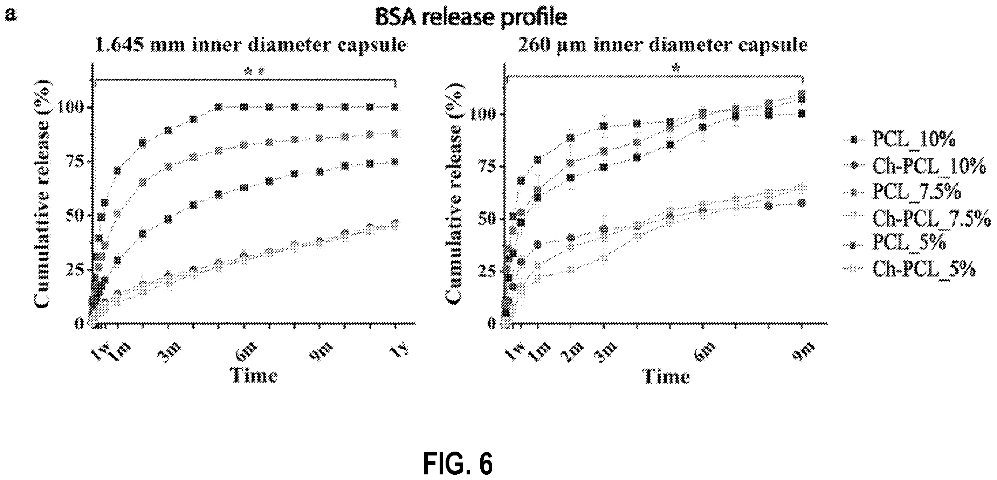

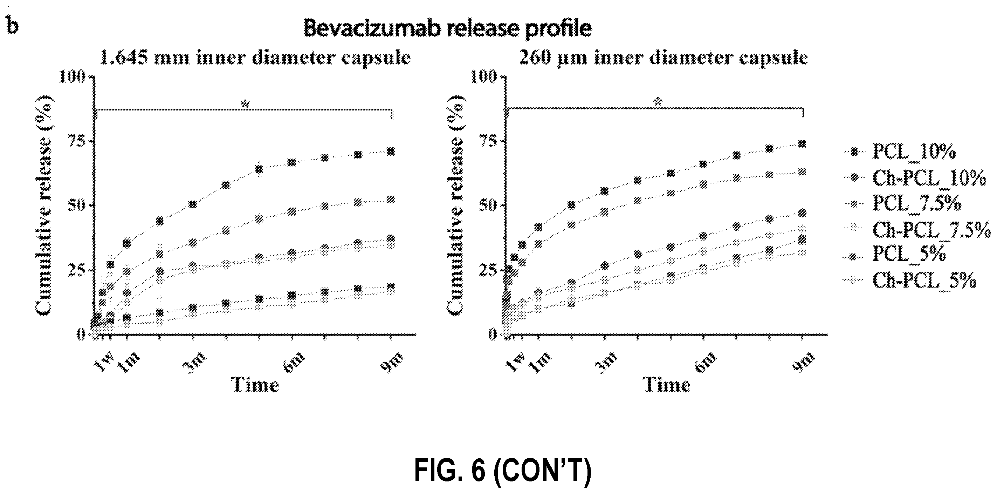

**FIG. 6** shows data pertaining to the effect of porous and bi-layered structure on protein release from a disclosed bi-layered capsule. The data were obtained as described herein below from a representative disclosed chitosan-PCL bi-layered capsule (labeled as Ch-PCL in the graph legend) and PCL mono-layered capsules (labeled as PCL in the graph legend) encapsulating BSA or bevacizumab as described herein determined from incubation in PBS. The percent values show with the "Ch-PCL" or "PCL" labels in the graph legend indicate the w/v% used to prepare the bi-layered or mono-layered capsule. Panel **a** shows a representative BSA release profile from a 1.645 mm inner diameter bi-layered capsule and a representative BSA release profile of profile from a 260 µm inner diameter bi-layered capsule. Panel **b** shows a representative bevacizumab release profile from a 1.645 mm inner diameter bi-layered capsule and a representative bevacizumab release profile of profile from a 260 µm inner diameter bi-layered capsule. Drug delivery compositions show a lower cumulative release than mono-layered capsules at each time point. (# = p ≤ 0.05). The data also show that increasing salt concentration was associated with increased cumulative release at each time point (*= p ≤ 0.05).

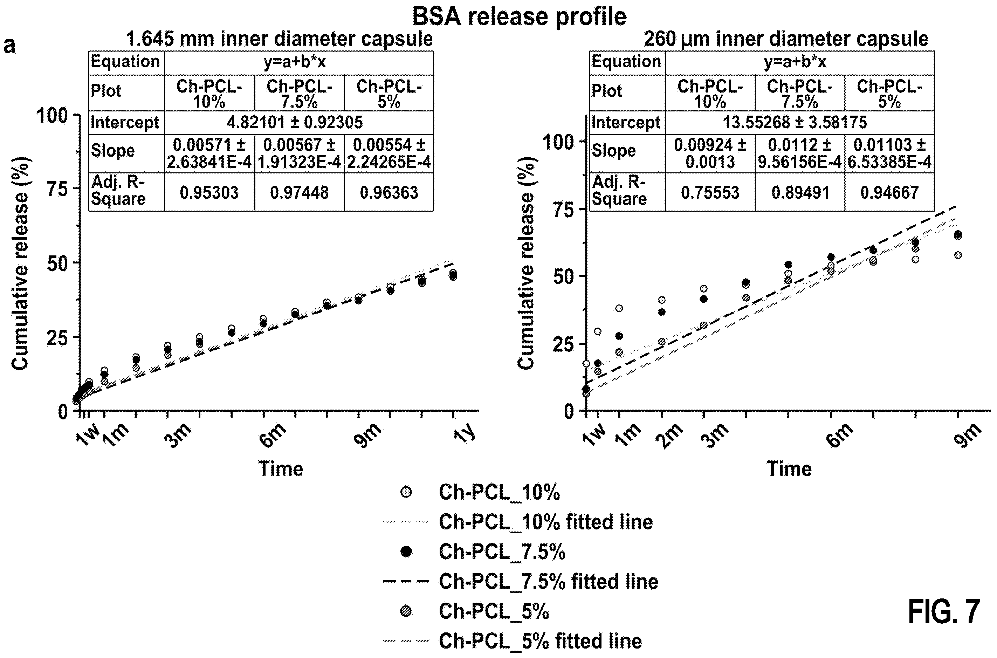

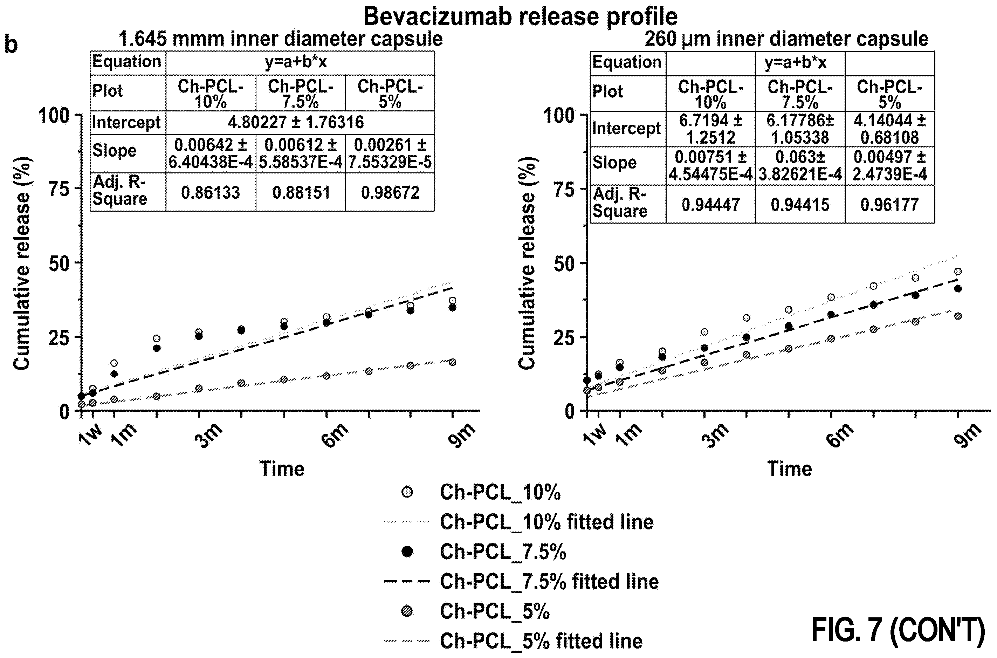

**FIG. 7** shows data pertaining to the effect of porous and bi-layered structure on protein release from a disclosed bi-layered capsule. In this figure, trendlines were fit to the data with the fit parameters as shown. The data were obtained as described herein below from a representative disclosed chitosan-PCL bi-layered capsule (labeled as Ch-PCL in the graph legend) and PCL mono-layered capsules (labeled as PCL in the graph legend) encapsulating BSA or bevacizumab as described herein determined from incubation in PBS. The percent values show with the "Ch-PCL" or "PCL" labels in the graph legend indicate the w/v% used to prepare the bi-layered or mono-layered capsule. Panel **a** shows a representative BSA release profile from a 1.645 mm inner diameter bi-layered capsule and a representative BSA release profile of profile from a 260 µm inner diameter bi-layered capsule. Panel **b** shows a representative bevacizumab release profile from a 1.645 mm inner diameter bi-layered capsule and a representative bevacizumab release profile of profile from a 260 µm inner diameter bi-layered capsule. The data show that the disclosed bi-layer capsules can achieve nearly zero-order release kinetics.

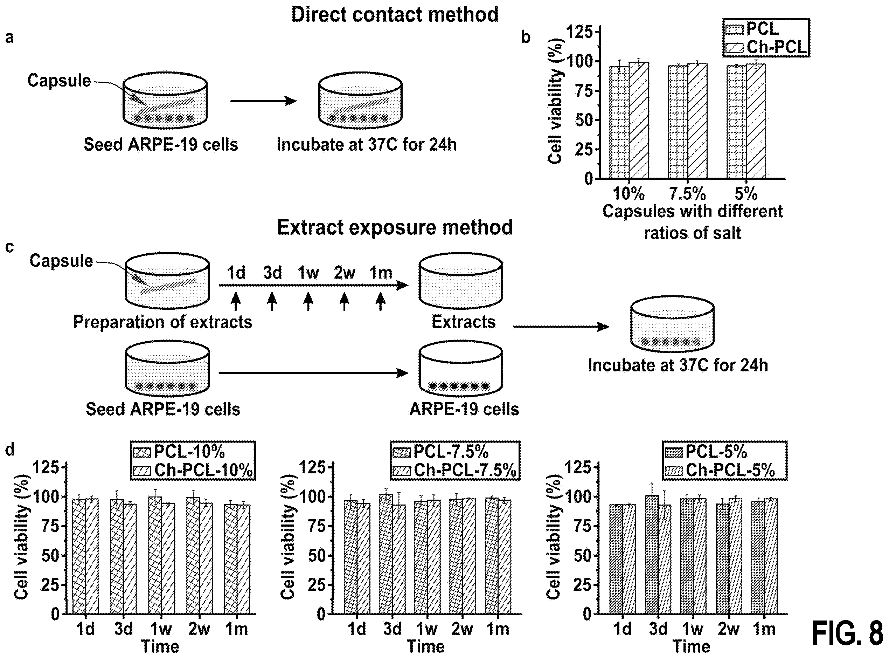

**FIG. 8** shows an assay scheme to assess potential toxicity of a disclosed capsule, and data obtained from the assay. Panel a shows an assay scheme for assessing *in vitro* cytotoxicity using ARPE-19 cells by a direct contact method. Panel **b** shows *in vitro* toxicity data for capsules prepared with 10.0% HEPES salt, 7.5% HEPES salt, and 5.0% HEPES salt by the direct contact method. Panel c shows an assay scheme for assessing *in vitro* cytotoxicity using ARPE-19 cells by an extract exposure method. Panel **d** shows the *in vitro* cytotoxicity of extracts of capsules prepared with different conditions. Each bar at different time point and salt concentration represents the mean measurement of three independent samples. Error bars show the standard deviation. As described above, the data were obtained using representative disclosed chitosan-PCL bi-layered capsule (labeled as Ch-PCL in the graph legend) and PCL mono-layered capsules (labeled as PCL in the graph legend). The data show no significant difference on cell viability between the cells treated with PCL or Chitosan-PCL extract (p > 0.05), as well as no significant observed difference over time ( p > 0.05).

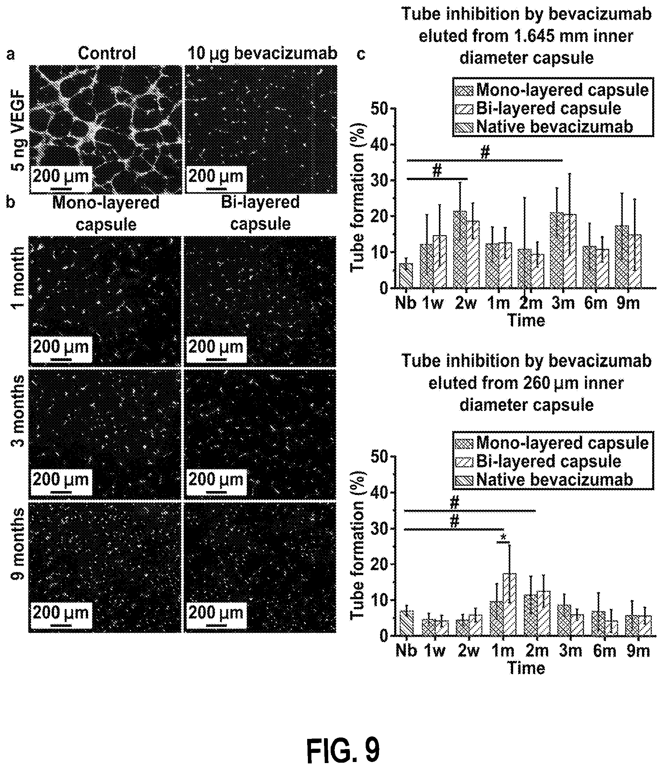

**FIG. 9** shows representative fluorescent micrograph images and data pertaining to inhibition of cell-tubule length in VEGF-treated HUVEC cells exposed to bevacizumab delivered using a PCL mono-layered capsule or a disclosed bi-layered capsule. Cells were labeled using Calcien AM. Panel **a** shows representative fluorescent images showing HUVECs treated to 5 ng VEGF in the absence (left) and presence (right) of 10 mg native bevacizumab in cell culture media. The data show that a significant disruption of cell tubules in cells in the presence of bevacizumab compared to the control group. Panel **b** shows representative fluorescent images showing the inhibition of cell tubules in cells exposed to 10 mg bevacizumab released from 260 µm diameter PCL mono-layered and chitosan-PCL drug delivery devices for 1 week, 1 month, 3 months, and 9 months exposure as indicated. Panel c shows mean tube length inhibition in the indicated groups quantitatively analyzed using ImageJ software. Data are presented as the mean ± SD, n = 3. The data show that a significant difference of HUVECS tube length of the group treated with eluted bevacizumab from the mono-layered capsule was noted compared to that from the bi-layered capsules (* = p ≤ 0.05). The data also show that a significant different of HUVECS tube inhibition capability of eluted bevacizumab was observed over time as compared to that of free native bevacizumab (# = p ≤ 0.05).

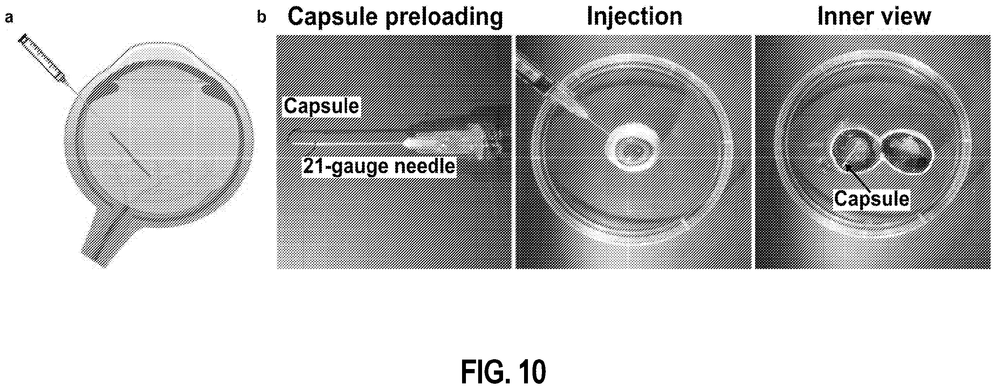

**FIG. 10** shows results obtained from studies assessing the injection of a representative disclosed bi-layered capsule into an *ex vivo* porcine eye model. Panel **a** shows a schematic representation of injection of a bi-layered capsule into the vitreous humor via a hypodermic needle. Panel **b** shows a preloaded capsule in 21-gauge needle, which was injected at 3 mm posterior to the limbus in the *ex vivo* porcine eye (see middle pane). Following injection, the *ex vivo* porcine eye was dissection, and the intact capsule was observed to be intact in the vitreous humor of the *ex vivo* porcine eye (see right pane).

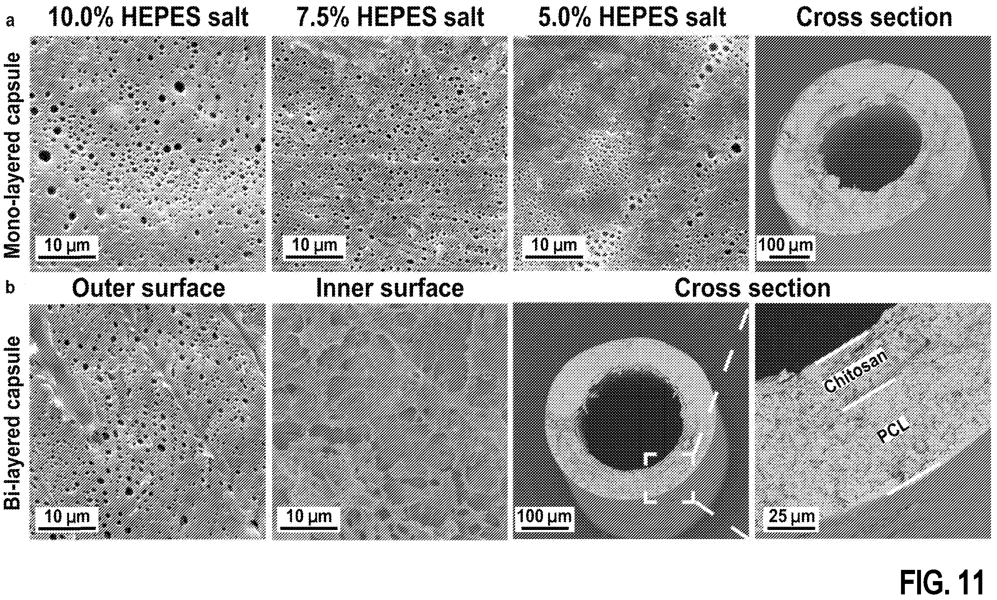

**FIG. 11** shows a comparison of biodegradation of PCL mono-layered capsules and the bi-layered capsules as described herein over one year of incubation. Panel **a** shows representative scanning electron micrograph (SEM) images prepared with different salt concentration. Increased pore size on PCL membranes was observed in all samples after nine months. The cross-section image (see right pane) shows the capsule remained intact over a one-year period. Panel **b** shows representative SEM images of bi-layered capsules. The fibrous framework could be observed in the porous chitosan layer. The intact bi-layered structure is shown from the cross-section image (see right pane).µ

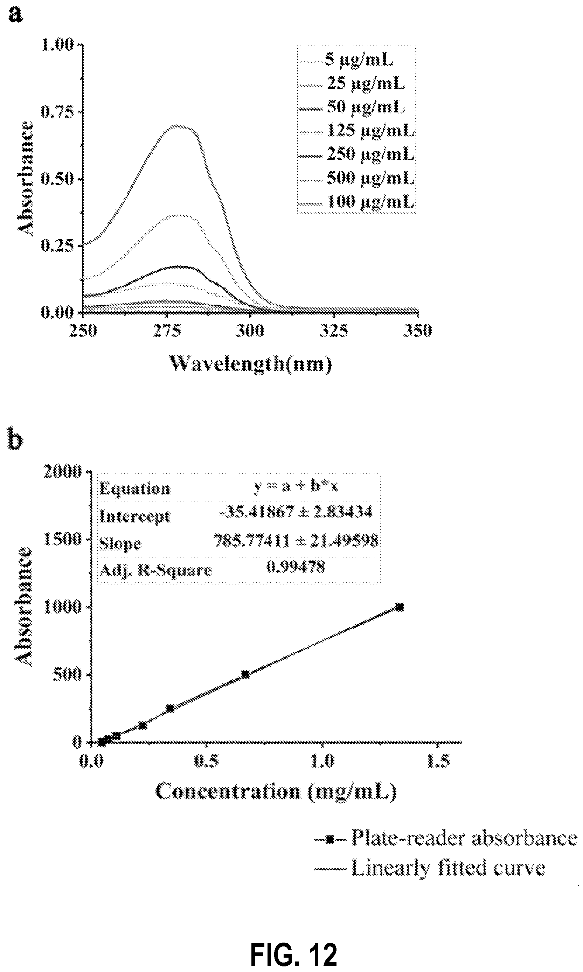

**FIG. 12** shows the UV-visible absorption spectrum of bevacizumab diluted in PBS at different concentrations. Panel **a** shows the absorbance of diluted bevacizumab measured by UV-Vis spectroscopy. Panel **b** shows the standard curve of bevacizumab measured by a plate-reader. The minimal concentrate which can be detected by UV-Vis spectroscopy and the plate reader is 5 µg/mL.

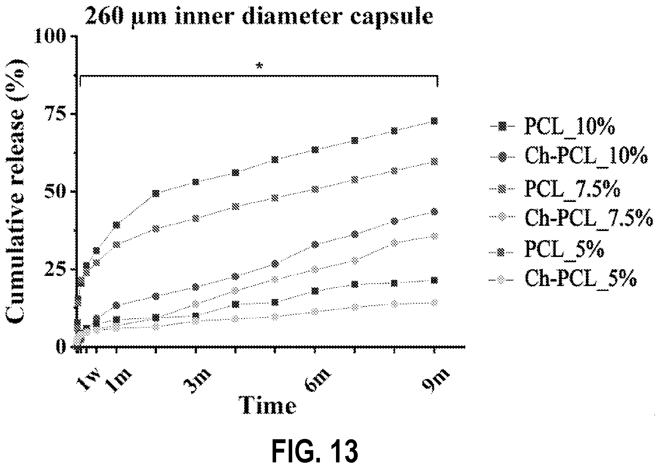

**FIG. 13** shows the effect of the porous and bi-layered structure of the capsules described herein on bevacizumab release as assessed by ELISA. The bi-layered capsules described herein and PCL mono-layered capsules encapsulating bevacizumab were incubated in PBS. The bevacizumab release profile for 260 µm inner diameter capsules was obtained. The disclosed bi-layered capsules effectively retained the protein inside the capsule for at least nine months and have lower cumulative release that mono-layered capsules at each time point (# = p ≤ 0.05). Increasing the salt concentration also increase cumulative release at each time point (* = p ≤ 0.05). The release profile acquired by ELISA is consistent with the results determined by UV-Vis spectroscopy.

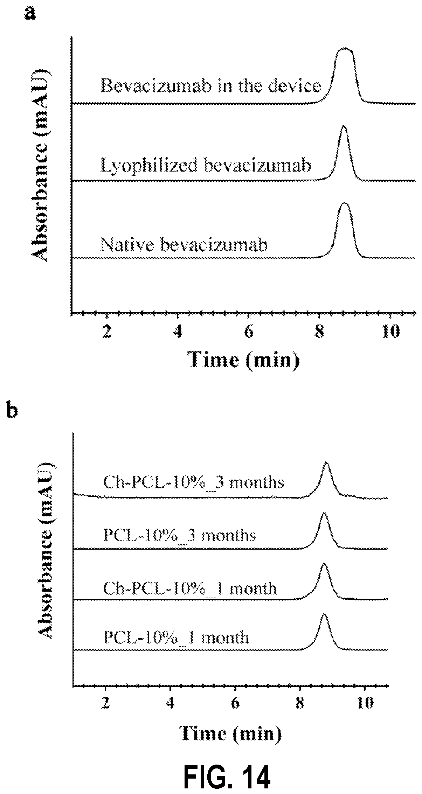

**FIG. 14** shows the stability of free native bevacizumab before and after lyophilization and eluted bevacizumab from the mono-layered capsule and the bi-layered capsule described herein over the first three months. Panel **a** shows SEC-HPLC chromatograms of the free native bevacizumab, lyophilized bevacizumab, and bevacizumab in the device. Panel **b** shows SEC-HPLC chromatograms of eluted bevacizumab from the mono-layered capsule and bi-layered capsule incubated at physiological temperature for one and three months.

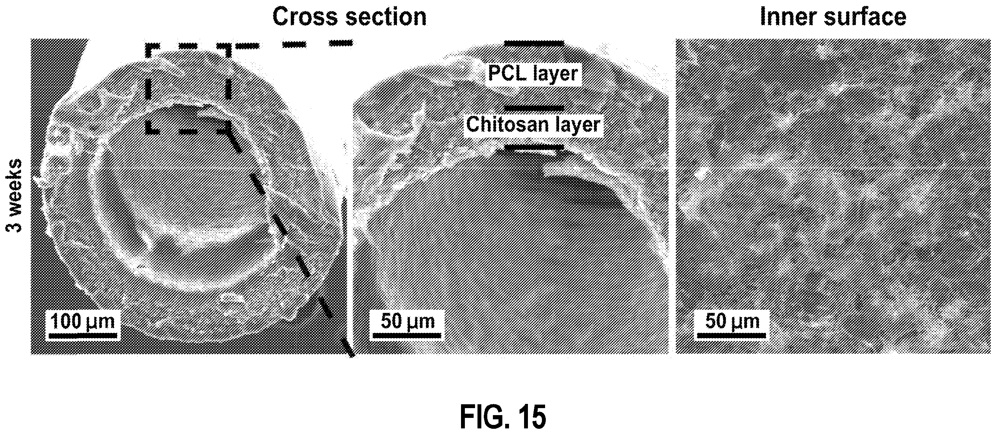

**FIG. 15** shows the biodegradation of chitosan-PCL bi-layered capsules exposed to PBS over three weeks. Representative SEM images are provided of 260 µm inner diameter bi-layered capsules prepared with 10% HEPES salts, representing the most porous structure. The cross-section and inner images show that the capsule lost its inner chitosan layer when it was directed exposed to PBS after three weeks, whereas biodegradation was not significant when the chitosan layer was coated with PCL. The thickness of the bi-layered capsule was 73.23 ± 3.62 µm.

Additional advantages of the disclosure will be set forth in part in the description which follows, and in part will be obvious from the description, or can be learned by practice of the disclosure. The advantages of the disclosure will be realized and attained by means of the elements and combinations particularly pointed out in the appended claims. It is to be understood that both the foregoing general description and the following detailed description are exemplary and explanatory only and are not restrictive of the disclosure, as claimed.

### DETAILED DESCRIPTION

Unless defined otherwise, all technical and scientific terms used herein have the same meaning as commonly understood by one of ordinary skill in the art to which the disclosed compositions and methods belong. It will be further understood that terms, such as those defined in commonly used dictionaries, should be interpreted as having a meaning that is consistent with their meaning in the context of the specification and relevant art and should not be interpreted in an idealized or overly formal sense unless expressly defined herein.

Prior to describing the various aspects of the present disclosure, the following definitions are provided and should be used unless otherwise indicated. Additional terms may be defined elsewhere in the present disclosure.

### Definitions

As used herein, "comprising" is to be interpreted as specifying the presence of the stated features, integers, steps, or components as referred to, but does not preclude the presence or addition of one or more features, integers, steps, or components, or groups thereof. Moreover, each of the terms "by", "comprising," "comprises", "comprised of," "including," "includes," "included," "involving," "involves," "involved," and "such as" are used in their open, non-limiting sense and may be used interchangeably. Further, the term "comprising" is intended to include examples and aspects encompassed by the terms "consisting essentially of" and "consisting of." Similarly, the term "consisting essentially of" is intended to include examples encompassed by the term "consisting of.

As used in the specification and the appended claims, the singular forms "a," "an" and "the" include plural referents unless the context clearly dictates otherwise. Thus, for example, reference to "a drug delivery composition," "a therapeutic agent," or "a clinical condition," includes, but is not limited to, two or more such drug delivery compositions, therapeutic agents, or clinical conditions, and the like.

It should be noted that ratios, concentrations, amounts, and other numerical data can be expressed herein in a range format. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. Ranges can be expressed herein as from "about" one particular value, and/or to "about" another particular value. Similarly, when values are expressed as approximations, by use of the antecedent "about," it will be understood that the particular value forms a further aspect. For example, if the value "about 10" is disclosed, then "10" is also disclosed.

When a range is expressed, a further aspect includes from the one particular value and/or to the other particular value. For example, where the stated range includes one or both of the limits, ranges excluding either or both of those included limits are also included in the disclosure, e.g. the phrase "x to y" includes the range from 'x' to 'y' as well as the range greater than 'x' and less than 'y'. The range can also be expressed as an upper limit, e.g. 'about x, y, z, or less' and should be interpreted to include the specific ranges of 'about x', 'about y', and 'about z' as well as the ranges of 'less than x', less than y', and 'less than z'. Likewise, the phrase 'about x, y, z, or greater' should be interpreted to include the specific ranges of 'about x', 'about y', and 'about z' as well as the ranges of 'greater than x', greater than y', and 'greater than z'. In addition, the phrase "about 'x' to 'y''', where 'x' and 'y' are numerical values, includes "about 'x' to about 'y'".

As used herein, the terms "about," "approximate," "at or about," and "substantially" mean that the amount or value in question can be the exact value or a value that provides equivalent results or effects as recited in the claims or taught herein. That is, it is understood that amounts, sizes, formulations, parameters, and other quantities and characteristics are not and need not be exact, but may be approximate and/or larger or smaller, as desired, reflecting tolerances, conversion factors, rounding off, measurement error and the like, and other factors known to those of skill in the art such that equivalent results or effects are obtained. In some circumstances, the value that provides equivalent results or effects cannot be reasonably determined. It is understood that where "about," "approximate," or "at or about" is used before a quantitative value, the parameter also includes the specific quantitative value itself, unless specifically stated otherwise.

As used herein, "effective amount" can refer to the amount of a disclosed compound or pharmaceutical composition provided herein that is sufficient to effect beneficial or desired biological, emotional, medical, or clinical response of a cell, tissue, system, animal, or human. An effective amount can be administered in one or more administrations, applications, or dosages. The term can also include within its scope amounts effective to enhance or restore to substantially normal physiological function.

As used herein, the term "therapeutically effective amount" refers to an amount that is sufficient to achieve the desired therapeutic result or to have an effect on undesired symptoms but is generally insufficient to cause adverse side effects. The specific therapeutically effective dose level for any particular patient will depend upon a variety of factors including the disorder being treated and the severity of the disorder; the specific composition employed; the age, body weight, general health, sex and diet of the patient; the time of administration; the route of administration; the rate of excretion of the specific compound employed; the duration of the treatment; drugs used in combination or coincidental with the specific compound employed and like factors within the knowledge and expertise of the health practitioner and which may be well known in the medical arts. In the case of treating a particular disease or condition, in some instances, the desired response can be inhibiting the progression of the disease or condition. This may involve only slowing the progression of the disease temporarily. However, in other instances, it may be desirable to halt the progression of the disease permanently. This can be monitored by routine diagnostic methods known to one of ordinary skill in the art for any particular disease. The desired response to treatment of the disease or condition also can be delaying the onset or even preventing the onset of the disease or condition.

For example, it is well within the skill of the art to start doses of a compound at levels lower than those required to achieve the desired therapeutic effect and to gradually increase the dosage until the desired effect is achieved. If desired, the effective daily dose can be divided into multiple doses for purposes of administration. Consequently, single dose compositions can contain such amounts or submultiples thereof to make up the daily dose. The dosage can be adjusted by the individual physician in the event of any contraindications. It is generally preferred that a maximum dose of the pharmacological agents of the invention (alone or in combination with other therapeutic agents) be used, that is, the highest safe dose according to sound medical judgment. It will be understood by those of ordinary skill in the art however, that a patient may insist upon a lower dose or tolerable dose for medical reasons, psychological reasons or for virtually any other reasons.

A response to a therapeutically effective dose of a disclosed drug delivery composition can be measured by determining the physiological effects of the treatment or medication, such as the decrease or lack of disease symptoms following administration of the treatment or pharmacological agent. Other assays will be known to one of ordinary skill in the art and can be employed for measuring the level of the response. The amount of a treatment may be varied for example by increasing or decreasing the amount of a disclosed compound and/or pharmaceutical composition, by changing the disclosed compound and/or pharmaceutical composition administered, by changing the route of administration, by changing the dosage timing and so on. Dosage can vary, and can be administered in one or more dose administrations daily, for one or several days. Guidance can be found in the literature for appropriate dosages for given classes of pharmaceutical products.

As used herein, the term "prophylactically effective amount" refers to an amount effective for preventing onset or initiation of a disease or condition.

As used herein, the term "prevent" or "preventing" refers to precluding, averting, obviating, forestalling, stopping, or hindering something from happening, especially by advance action. It is understood that where reduce, inhibit or prevent are used herein, unless specifically indicated otherwise, the use of the other two words is also expressly disclosed.

As used herein, the terms "optional" or "optionally" means that the subsequently described event or circumstance can or cannot occur, and that the description includes instances where said event or circumstance occurs and instances where it does not.

As used herein, "therapeutic agent" can refer to any substance, compound, molecule, and the like, which can be biologically active or otherwise can induce a pharmacologic, immunogenic, biologic and/or physiologic effect on a subject to which it is administered to by local and/or systemic action. A therapeutic agent can be a primary active agent, or in other words, the component(s) of a composition to which the whole or part of the effect of the composition is attributed. A therapeutic agent can be a secondary therapeutic agent, or in other words, the component(s) of a composition to which an additional part and/or other effect of the composition is attributed. The term therefore encompasses those compounds or chemicals traditionally regarded as drugs, vaccines, and biopharmaceuticals including molecules such as proteins, peptides, hormones, nucleic acids, gene constructs and the like. Examples of therapeutic agents are described in well-known literature references such as the Merck Index (14th edition), the Physicians' Desk Reference (64th edition), and The Pharmacological Basis of Therapeutics (12th edition), and they include, without limitation, medicaments; vitamins; mineral supplements; substances used for the treatment, prevention, diagnosis, cure or mitigation of a disease or illness; substances that affect the structure or function of the body, or pro-drugs, which become biologically active or more active after they have been placed in a physiological environment. For example, the term "therapeutic agent" includes compounds or compositions for use in all of the major therapeutic areas including, but not limited to, adjuvants; anti-infectives such as antibiotics and antiviral agents; analgesics and analgesic combinations, anorexics, anti-inflammatory agents, anti-epileptics, local and general anesthetics, hypnotics, sedatives, antipsychotic agents, neuroleptic agents, antidepressants, anxiolytics, antagonists, neuron blocking agents, anticholinergic and cholinomimetic agents, antimuscarinic and muscarinic agents, antiadrenergics, antiarrhythmics, antihypertensive agents, hormones, and nutrients, antiarthritics, antiasthmatic agents, anticonvulsants, antihistamines, antinauseants, antineoplastics, antipruritics, antipyretics; antispasmodics, cardiovascular preparations (including calcium channel blockers, beta-blockers, beta-agonists and antiarrythmics), antihypertensives, diuretics, vasodilators; central nervous system stimulants; cough and cold preparations; decongestants; diagnostics; hormones; bone growth stimulants and bone resorption inhibitors; immunosuppressives; muscle relaxants; psychostimulants; sedatives; tranquilizers; proteins, peptides, and fragments thereof (whether naturally occurring, chemically synthesized or recombinantly produced); and nucleic acid molecules (polymeric forms of two or more nucleotides, either ribonucleotides (RNA) or deoxyribonucleotides (DNA) including both double- and single-stranded molecules, gene constructs, expression vectors, antisense molecules and the like), small molecules (e.g., doxorubicin) and other biologically active macromolecules such as, for example, proteins and enzymes. The agent may be a biologically active agent used in medical, including veterinary, applications and in agriculture, such as with plants, as well as other areas. The term therapeutic agent also includes without limitation, medicaments; vitamins; mineral supplements; substances used for the treatment, prevention, diagnosis, cure or mitigation of disease or illness; or substances which affect the structure or function of the body; or pro- drugs, which become biologically active or more active after they have been placed in a predetermined physiological environment.

It is understood that disclosure herein of a therapeutic agent also disclosed pharmaceutically acceptable salt, pharmaceutically acceptable ester, pharmaceutically acceptable amide, prodrug forms, and derivates of the therapeutic agent.

The term "pharmaceutically acceptable salts", as used herein, means salts of the active principal agents which are prepared with acids or bases that are tolerated by a biological system or tolerated by a subject or tolerated by a biological system and tolerated by a subject when administered in a therapeutically effective amount. When compounds of the present disclosure contain relatively acidic functionalities, base addition salts can be obtained by contacting the neutral form of such compounds with a sufficient amount of the desired base, either neat or in a suitable inert solvent. Examples of pharmaceutically acceptable base addition salts include, but are not limited to; sodium, potassium, calcium, ammonium, organic amino, magnesium salt, lithium salt, strontium salt or a similar salt. When compounds of the present disclosure contain relatively basic functionalities, acid addition salts can be obtained by contacting the neutral form of such compounds with a sufficient amount of the desired acid, either neat or in a suitable inert solvent. Examples of pharmaceutically acceptable acid addition salts include, but are not limited to; those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like.

The term "pharmaceutically acceptable ester" refers to esters of compounds of the present disclosure which hydrolyze in vivo and include those that break down readily in the human body to leave the parent compound or a salt thereof. Examples of pharmaceutically acceptable, non-toxic esters of the present disclosure include C 1 -to-C 6 alkyl esters and C 5 -to-C 7 cycloalkyl esters, although C 1 -to-C 4 alkyl esters are preferred. Esters of disclosed compounds can be prepared according to conventional methods. Pharmaceutically acceptable esters can be appended onto hydroxy groups by reaction of the compound that contains the hydroxy group with acid and an alkylcarboxylic acid such as acetic acid, or with acid and an arylcarboxylic acid such as benzoic acid. In the case of compounds containing carboxylic acid groups, the pharmaceutically acceptable esters are prepared from compounds containing the carboxylic acid groups by reaction of the compound with base such as triethylamine and an alkyl halide, for example with methyl iodide, benzyl iodide, cyclopentyl iodide or alkyl triflate. They also can be prepared by reaction of the compound with an acid such as hydrochloric acid and an alcohol such as ethanol or methanol.

The term "pharmaceutically acceptable amide" refers to non-toxic amides of the present disclosure derived from ammonia, primary C 1 -to-C 6 alkyl amines and secondary C 1 -to-C 6 dialkyl amines. In the case of secondary amines, the amine can also be in the form of a 5- or 6-membered heterocycle containing one nitrogen atom. Amides derived from ammonia, C 1 -to-C 3 alkyl primary amides and C 1 -to-C 2 dialkyl secondary amides are preferred. Amides of disclosed compounds can be prepared according to conventional methods. Pharmaceutically acceptable amides can be prepared from compounds containing primary or secondary amine groups by reaction of the compound that contains the amino group with an alkyl anhydride, aryl anhydride, acyl halide, or aroyl halide. In the case of compounds containing carboxylic acid groups, the pharmaceutically acceptable amides are prepared from compounds containing the carboxylic acid groups by reaction of the compound with base such as triethylamine, a dehydrating agent such as dicyclohexyl carbodiimide or carbonyl diimidazole, and an alkyl amine, dialkylamine, for example with methylamine, diethylamine, and piperidine. They also can be prepared by reaction of the compound with an acid such as sulfuric acid and an alkylcarboxylic acid such as acetic acid, or with acid and an arylcarboxylic acid such as benzoic acid under dehydrating conditions such as with molecular sieves added. The composition can contain a compound of the present disclosure in the form of a pharmaceutically acceptable prodrug.

The term "pharmaceutically acceptable prodrug" or "prodrug" represents those prodrugs of the compounds of the present disclosure which are, within the scope of sound medical judgment, suitable for use in contact with the tissues of humans and lower animals without undue toxicity, irritation, allergic response, and the like, commensurate with a reasonable benefit/risk ratio, and effective for their intended use. Prodrugs of the present disclosure can be rapidly transformed in vivo to a parent compound having a structure of a disclosed compound, for example, by hydrolysis in blood. A thorough discussion is provided in T. Higuchi and V. Stella, Pro-drugs as Novel Delivery Systems, V. 14 of the A.C.S. Symposium Series, and in Edward B. Roche, ed., Bioreversible Carriers in Drug Design, American Pharmaceutical Association and Pergamon Press (1987).

As used herein, "kit" means a collection of at least two components constituting the kit. Together, the components constitute a functional unit for a given purpose. Individual member components may be physically packaged together or separately. For example, a kit comprising an instruction for using the kit may or may not physically include the instruction with other individual member components. Instead, the instruction can be supplied as a separate member component, either in a paper form or an electronic form which may be supplied on computer readable memory device or downloaded from an internet website, or as recorded presentation.

As used herein, "instruction(s)" means documents describing relevant materials or methodologies pertaining to a kit. These materials may include any combination of the following: background information, list of components and their availability information (purchase information, etc.), brief or detailed protocols for using the kit, trouble-shooting, references, technical support, and any other related documents. Instructions can be supplied with the kit or as a separate member component, either as a paper form or an electronic form which may be supplied on computer readable memory device or downloaded from an internet website, or as recorded presentation. Instructions can comprise one or multiple documents and are meant to include future updates.

As used interchangeably herein, "subject," "individual," or "patient" can refer to a vertebrate organism, such as a mammal (e.g. human). "Subject" can also refer to a cell, a population of cells, a tissue, an organ, or an organism, preferably to human and constituents thereof.

As used herein, the terms "treating" and "treatment" can refer generally to obtaining a desired pharmacological and/or physiological effect. The effect can be, but does not necessarily have to be, prophylactic in terms of preventing or partially preventing a disease, symptom or condition thereof, such as an ophthalmological disorder. The effect can be therapeutic in terms of a partial or complete cure of a disease, condition, symptom or adverse effect attributed to the disease, disorder, or condition. The term "treatment" as used herein can include any treatment of ophthalmological disorder in a subject, particularly a human and can include any one or more of the following: (a) preventing the disease from occurring in a subject which may be predisposed to the disease but has not yet been diagnosed as having it; (b) inhibiting the disease, i.e., arresting its development; and (c) relieving the disease, i.e., mitigating or ameliorating the disease and/or its symptoms or conditions. The term "treatment" as used herein can refer to both therapeutic treatment alone, prophylactic treatment alone, or both therapeutic and prophylactic treatment. Those in need of treatment (subjects in need thereof) can include those already with the disorder and/or those in which the disorder is to be prevented. As used herein, the term "treating", can include inhibiting the disease, disorder or condition, e.g., impeding its progress; and relieving the disease, disorder, or condition, e.g., causing regression of the disease, disorder and/or condition. Treating the disease, disorder, or condition can include ameliorating at least one symptom of the particular disease, disorder, or condition, even if the underlying pathophysiology is not affected, e.g., such as treating the pain of a subject by administration of an analgesic agent even though such agent does not treat the cause of the pain.

As used herein, "dose," "unit dose," or "dosage" can refer to physically discrete units suitable for use in a subject, each unit containing a predetermined quantity of a disclosed compound and/or a pharmaceutical composition thereof calculated to produce the desired response or responses in association with its administration.

As used herein, "therapeutic" can refer to treating, healing, and/or ameliorating a disease, disorder, condition, or side effect, or to decreasing in the rate of advancement of a disease, disorder, condition, or side effect.

As used herein, nomenclature for compounds, including organic compounds, can be given using common names, IUPAC, IUBMB, or CAS recommendations for nomenclature. When one or more stereochemical features are present, Cahn-Ingold-Prelog rules for stereochemistry can be employed to designate stereochemical priority, E/Z specification, and the like. One of skill in the art can readily ascertain the structure of a compound if given a name, either by systemic reduction of the compound structure using naming conventions, or by commercially available software, such as CHEMDRAW^{™} (Cambridgesoft Corporation, U.S.A.).

Unless otherwise specified, temperatures referred to herein are based on atmospheric pressure (i.e. one atmosphere).

Described herein are drug delivery compositions that have therapeutic or clinical utility. Also described herein are methods of preparing or making the disclosed drug delivery compositions. Also described herein are the disclosed drug delivery compositions for use in methods of administering the disclosed drug delivery compositions to a subject in need thereof. In some aspects, the subject can have a clinical condition or pathology such as an ophthalmological disorder. Other compositions, compounds, methods, features, and advantages of the present disclosure will be or become apparent to one having ordinary skill in the art upon examination of the following drawings, detailed description, and examples.

### Drug Delivery Compositions

Vascular endothelial growth factor (VEGF) is an essential regulator involved in the abnormal angiogenesis; it assists in the rapid tumor growth and in the formation of wet age-related macular degeneration (AMD) (see Holmes, D.I.R. and I. Zachary, The vascular endothelial growth factor (VEGF) family: angiogenic factors in health and disease. Genome biology, 2005. 6(2): p. 209-209; Shibuya, M., Vascular Endothelial Growth Factor (VEGF) and Its Receptor (VEGFR) Signaling in Angiogenesis: A Crucial Target for Anti- and Pro-Angiogenic Therapies. Genes & cancer, 2011. 2(12): p. 1097-1105; and Ferrara, N., Role of vascular endothelial growth factor in the regulation of angiogenesis. Kidney International, 1999. 56(3): p. 794-814). Anti-angiogenesis strategies have been proposed to slow wet AMD (see Ferrara, N., et al., Discovery and development of bevacizumab, an anti-VEGF antibody for treating cancer. 2004. 3(5): p. 391; and Niu, G. and X. Chen, Vascular endothelial growth factor as an anti-angiogenic target for cancer therapy. Current drug targets, 2010. 11(8): p. 1000-1017). The humanized monoclonal antibody, anti-VEGF, has been used in ophthalmology for the off-label treatment of wet AMD (see Ferrara, N., et al., Discovery and development of bevacizumab, an anti-VEGF antibody for treating cancer. 2004. 3(5): p. 391; and Presta, L.G., et al., Humanization of an anti-vascular endothelial growth factor monoclonal antibody for the therapy of solid tumors and other disorders. 1997. 57(20): p. 4593-4599).

Treatment of this age-associated retinal disease currently relies on the use of anti-angiogenesis agents to slow or halt progression. Intravitreal injection of anti-VEGF therapeutics, such as bevacizumab and ranibizumab, constitute the current gold standard treatment for wet AMD and prevent VEGF from initiating subretinal choroidal neovascularization (CNV) and irreversible retinal damage caused by bleeding and scarring of newly formed blood vessels (see Delplace, V., S. Payne, and M.J.J.o.C.R. Shoichet, Delivery strategies for treatment of age-related ocular diseases: From a biological understanding to biomaterial solutions. 2015. 219: p. 652-668; and Ohr, M. and P.K.J.E.o.o.p. Kaiser, Intravitreal aflibercept injection for neovascular (wet) age-related macular degeneration. 2012. 13(4): p. 585-591). Bevacizumab, for example, has been widely used for treating wet AMD because of its relatively low cost. However, the short half-life of these protein therapeutics in the vitreous humor often requires frequent, up to monthly, intravitreal injections to maintain effectiveness in the eye (see Hård, A.L. and A.J.A.p. Hellström, On safety, pharmacokinetics and dosage of bevacizumab in ROP treatment a review. 2011. 100(12): p. 1523-1527; and Stewart, M.W., et al., Pharmacokinetic rationale for dosing every 2 weeks versus 4 weeks with intravitreal ranibizumab, bevacizumab, and aflibercept (vascular endothelial growth factor Trap-eye). Retina, 2012. 32(3): p. 434-457). Unfortunately, this modality frequently results in side effects that include pain, infection, endophthalmitis, elevated intraocular pressure, inflammation, retinal detachment, and cataract formation (see Sampat, K.M. and S.J.J.C.o.i.o. Garg, Complications of intravitreal injections. 2010. 21(3): p. 178-183). The primary barrier to treatment is the high cost associated with each injection, placing the burden on patients and families to receive treatment monthly (see Heimes, B., et al., Compliance von Patienten mit altersabhängiger Makuladegeneration unter Anti-VEGF-Therapie. Der Ophthalmologe, 2016. 113(11): p. 925-932). Therefore, a clear need for more facile and efficient treatment for wet AMD exists.

Conventional delivery systems, in the form of implants and particles, have been developed to achieve controlled release for potential AMD treatment (see Delplace, V., S. Payne, and M.J.J.o.C.R. Shoichet, Delivery strategies for treatment of age-related ocular diseases: From a biological understanding to biomaterial solutions. 2015. 219: p. 652-668; Radhakrishnan, K., et al., Protein delivery to the back of the eye: barriers, carriers and stability of anti-VEGF proteins. 2017. 22(2): p. 416-423; Imperiale, J.C., G.B. Acosta, and A.J.J.o.C.R. Sosnik, Polymer-based carriers for ophthalmic drug delivery. 2018; and Lee, S.S., et al., Biodegradable implants for sustained drug release in the eye. 2010. 27(10): p. 2043-2053). As compared to micro/nanoparticle-based systems, implants have higher stability and drug payload due to their larger size (see Kim, Y.C., et al., Ocular delivery of macromolecules. Journal of Controlled Release, 2014. 190: p. 172-181). However, most implant-based treatments are accompanied by difficulties in injection; additional surgeries for implantation and removal are required for nonbiodegradable intraocular implants (see Silva, G.R.d., et al., Implants as drug delivery devices for the treatment of eye diseases. Brazilian Journal of Pharmaceutical Sciences, 2010. 46: p. 585-595). These are associated with postoperative complications as well as increased cost. In addition, long-term sustained release from either particles or implants has been challenging due to insufficient physical and chemical drug retention. For example, poly(lactic-co-glycolic acid) (PLGA), one the most commonly used polymers for drug delivery is characterized by rapid hydrolytic degradation , often leading to a maximum of 90-day therapeutic release (see Li, F., et al., Controlled release of bevacizumab through nanospheres for extended treatment of age-related macular degeneration. 2012. 6: p. 54; and Sousa, F., et al., A new paradigm for antiangiogenic therapy through controlled release of bevacizumab from PLGA nanoparticles. 2017. 7(1): p. 3736). Additional drawbacks include the formation of acidic byproducts that can induce inflammation and aggravate the foreign body reaction (see Lu, L., M.J. Yaszemski, and A.G.J.B. Mikos, Retinal pigment epithelium engineering using synthetic biodegradable polymers. 2001. 22(24): p. 3345-3355).

Herein, a drug delivery composition comprising injectable, biodegradable and multi-layered capsules loaded with a therapeutic agent, e.g., bevacizumab, is disclosed for achieving a higher drug loading rate and a longer-term drug release duration than conventionally available injectable drug delivery devices. To achieve highly sustainable and controllable drug release, disclosed herein, for example, are drug delivery compositions comprising a nanoporous PCL outer-shell and chitosan inner-layer to achieve physical trapping and electrostatic-based chemoabsorption, respectively. To load enough therapeutic sufficient for long-term drug release to at least one year, a hollow structure encapsulated by the bi-layer hybrid shell was utilized. More specifically, the whole drug delivery composition is prepared by combining materials processing technologies including electrospinning, sintering and salt leaching. The disclosed methods provide a central hollow cylindrical microrod with high aspect ratios to enable injection feasibility via 21-gauge or smaller needle for intravitreal implant delivery. By optimizing the chemical and physical structures of the capsules using the disclosed methods a stable and controlled release of protein therapeutics for over ten months using the disclosed drug delivery composition can be obtained. By reducing the frequency of injections through a small gauge needle, the disclosed drug delivery composition can potentially improve the quality of life of patients with wet AMD.

Thus in one aspect, a drug delivery composition is provided comprising:
one or more capsules each having a tubular shape with two ends that are closed, wherein each of the one or more capsules independently comprises a bi-layered wall and at least one luminal compartment; and
one or more therapeutic agents each present within one or more of the at least one luminal compartment;
wherein each bi-layered wall comprises an inner layer and an outer layer;
wherein each inner layer comprises chitosan; and
wherein each outer layer independently comprises a poly(ε-caprolactone) (PCL);
wherein the one or more therapeutic agents have a net negative charge at any pH within pH 6.0 to pH 7.4.

In some embodiments, the drug delivery composition may comprise two or more capsules, for example two capsules, three capsules, four capsules, five capsules, six capsules, seven capsules, eight capsules, nine capsules, ten capsules, or more. In such embodiments, the two or more capsules may comprise the same composition for the bi-layered wall of each capsule or may differ in their composition. In some embodiments, the same therapeutic agent or a different therapeutic agent may be initially present within each of the two or more capsules.

In some embodiments, each capsule in the drug delivery composition may independently comprise two or more luminal compartments, for example two luminal compartments, three luminal compartments, for luminal compartments, or more. In some embodiments, the same therapeutic agent or a different therapeutic agent may be initially present within each of the two or more luminal compartments within a single capsule.

In some embodiments, each capsule independently has a length from about 0.1 cm to about 5 cm, for example from 0.5 cm to about 3 cm or from 1 cm to about 3 cm. In some embodiments, each capsule independently has a length from about 0.1 cm to 5 cm, from 0.5 cm to 5 cm, from 1 cm to 5 cm, from 2 cm to 5 cm, from 3 cm to 5 cm, from 4 cm to 5 cm, from 0.1 cm to 4 cm, from 0.5 to 4 cm, from 1 cm to 4 cm, from 2 cm to 4 cm, from 3 cm to 4 cm, from 0.1 cm to 3 cm, from 0.5 cm to 3 cm, from 1 cm to 3 cm, from 2 cm to 3 cm, from 0.1 cm to 2 cm, from 0.5 cm to 2 cm, from 1 cm to 2 cm, from 0.1 cm to 1 cm, from 0.5 to 1 cm, or from 0.1 to 0.5 cm.

In some embodiments, the bi-layered wall has a wall thickness from about 25 µm to about 150 µm, for example from about 70 µm to about 100 µm, from about 75 µm to about 95 µm, or from about 80 µm to about 90 µm. In some embodiments, the multi-layered wall has a wall thickness from about 50 µm to 150 µm, from about 55 µm to 150 µm, from about 60 µm to about 150 µm, from about 65 µm to about 150 µm, from about 70 µm to about 150 µm, from about 75 µm to about 150 µm, from about 80 µm to about 150 µm, from about 90 µm to about 150 µm, from about 95 µm to about 150 µm, from about 100 µm to about 150 µm, from about 110 µm to about 150 µm, from about 125 µm to about 150 µm, from about 140 µm to about 150 µm, from about 50 µm to 140 µm, from about 55 µm to 140 µm, from about 60 µm to about 140 µm, from about 65 µm to about 140 µm, from about 70 µm to about 140 µm, from about 75 µm to about 140 µm, from about 80 µm to about 140 µm, from about 90 µm to about 140 µm, from about 95 µm to about 140 µm, from about 100 µm to about 140 µm, from about 110 µm to about 140 µm, from about 125 µm to about 140 µm, from about 50 µm to 125 µm, from about 55 µm to 125 µm, from about 60 µm to about 125 µm, from about 65 µm to about 125 µm, from about 70 µm to about 125 µm, from about 75 µm to about 125 µm, from about 80 µm to about 125 µm, from about 90 µm to about 125 µm, from about 95 µm to about 125 µm, from about 100 µm to about 125 µm, from about 110 µm to about 125 µm, from about 50 µm to 110 µm, from about 55 µm to 110 µm, from about 60 µm to about 110 µm, from about 65 µm to about 110 µm, from about 70 µm to about 110 µm, from about 75 µm to about 110 µm, from about 80 µm to about 110 µm, from about 90 µm to about 110 µm, from about 95 µm to about 110 µm, from about 100 µm to about 110 µm, from about 50 µm to 100 µm, from about 55 µm to 100 µm, from about 60 µm to about 100 µm, from about 65 µm to about 100 µm, from about 70 µm to about 100 µm, from about 75 µm to about 100 µm, from about 80 µm to about 100 µm, from about 90 µm to about 100 µm, from about 95 µm to about 100 µm, from about 50 µm to 95 µm, from about 55 µm to 95 µm, from about 60 µm to about 95 µm, from about 65 µm to about 95 µm, from about 70 µm to about 95 µm, from about 75 µm to about 95 µm, from about 80 µm to about 95 µm, from about 90 µm to about 95 µm, from about 50 µm to 90 µm, from about 55 µm to 90 µm, from about 60 µm to about 90 µm, from about 65 µm to about 90 µm, from about 70 µm to about 90 µm, from about 75 µm to about 90 µm, from about 80 µm to about 90 µm, from about 50 µm to 80 µm, from about 55 µm to 80 µm, from about 60 µm to about 80 µm, from about 65 µm to about 80 µm, from about 70 µm to about 80 µm, from about 75 µm to about 80 µm, from about 50 µm to 75 µm, from about 55 µm to 75 µm, from about 60 µm to about 75 µm, from about 65 µm to about 75 µm, from about 70 µm to about 75 µm, from about 50 µm to 70 µm, from about 55 µm to 70 µm, from about 60 µm to about 70 µm, from about 65 µm to about 70 µm, from about 50 µm to 65 µm, from about 55 µm to 65 µm, from about 60 µm to about 65 µm, from about 50 µm to 60 µm, from about 55 µm to 60 µm, and from about 50 µm to about 55 µm.

In some embodiments, the thickness of the inner layer may range from about 1 µm to about 100 µm. In some embodiments, the thickness of the inner layer may range from about 100 nm to about 990 nm, for example about 100 nm, 200 nm, 300 nm, 400 nm, 500 nm, 600 nm, 700 nm, 800 nm, 900 nm, or 990 nm.

In some embodiments, the thickness of the outer layer may range from about 1 µm to about 100 µm. In some embodiments, the thickness of the outer layer may range from about 100 nm to about 990 nm, for example about 100 nm, 200 nm, 300 nm, 400 nm, 500 nm, 600 nm, 700 nm, 800 nm, 900 nm, or 990 nm.

In some embodiments, the tubular shape of the drug delivery capsule has an inner diameter from about 100 µm to about 1000 µm, for example from about 100 µm to about 1000 µm, from about 100 µm to about 500 µm, or from 100 µm to about 300 µm. In some embodiments, the tubular shape of the drug delivery capsule has an inner diameter from about 100 µm to about 2000 µm, from 200 µm to about 2000 µm, from about 300 µm to about 2000 µm, from about 400 µm to about 2000 µm, from about 500 µm to about 2000 µm, from about 600 µm to about 2000 µm, from about 700 µm to about 2000 µm, from about 800 µm to about 2000 µm, from about 900 µm to about 2000 µm, from about 1000 µm to about 2000 µm, from about 1500 µm to about 2000 µm, from about 100 µm to about 1500 µm, from 200 µm to about 1500 µm, from about 300 µm to about 1500 µm, from about 400 µm to about 1500 µm, from about 500 µm to about 1500 µm, from about 600 µm to about 1500 µm, from about 700 µm to about 1500 µm, from about 800 µm to about 1500 µm, from about 900 µm to about 1500 µm, from about 1000 µm to about 1500 µm, from about 100 µm to about 1000 µm, from 200 µm to about 1000 µm, from about 300 µm to about 1000 µm, from about 400 µm to about 1000 µm, from about 500 µm to about 1000 µm, from about 600 µm to about 1000 µm, from about 700 µm to about 1000 µm, from about 800 µm to about 1000 µm, from about 900 µm to about 1000 µm, from about 100 µm to about 900 µm, from 200 µm to about 900 µm, from about 300 µm to about 900 µm, from about 400 µm to about 900 µm, from about 500 µm to about 900 µm, from about 600 µm to about 900 µm, from about 700 µm to about 900 µm, from about 800 µm to about 900 µm, from about 100 µm to about 800 µm, from 200 µm to about 800 µm, from about 300 µm to about 800 µm, from about 400 µm to about 800 µm, from about 500 µm to about 800 µm, from about 600 µm to about 800 µm, from about 700 µm to about 800 µm, from about 100 µm to about 700 µm, from 200 µm to about 700 µm, from about 300 µm to about 700 µm, from about 400 µm to about 700 µm, from about 500 µm to about 700 µm, from about 600 µm to about 700 µm, from about 100 µm to about 600 µm, from 200 µm to about 600 µm, from about 300 µm to about 600 µm, from about 400 µm to about 600 µm, from about 500 µm to about 600 µm, from about 100 µm to about 500 µm, from 200 µm to about 500 µm, from about 300 µm to about 500 µm, from about 400 µm to about 500 µm, from about 100 µm to about 400 µm, from 200 µm to about 400 µm, from about 300 µm to about 400 µm, from about 100 µm to about 300 µm, from 200 µm to about 300 µm, and from about 100 µm to about 200 µm. In some embodiments, the tubular shape has an outer diameter from about 100 µm to about 300 µm greater than the inner diameter, for example from about 100 µm to about 300 µm, from 150 µm to about 300 µm, from 200 µm to about 300 µm, from about 250 µm to about 300 µm, from about 100 µm to about 250 µm, from about 150 µm to about 250 µm, from about 200 µm to about 250 µm, from about 100 µm to about 200 µm, from about 150 µm to about 200 µm, or from about 100 µm to about 150 µm greater than the inner diameter.

In some embodiments, the first polymer may additionally comprise a polyethyleneimine, a protamine, a polypropylenimine, a poly-L-lysine, a poly-L-arginine, a poly-D-lysine, a poly-D-arginine, a cellulose, a dextran, a poly(amidoamine), poly(2-(dimethylamino)ethyl methacrylate, derivatives thereof, or combinations thereof.

The chitosan can have a degree of deacetylation of about 60% to about 90%; a degree of deacetylation of at least about 70%, at least about 75%, at least about 80%.

In some embodiments, the first polymer has a molecular weight of from about 50 kDa to about 500 kDa, for example from about 100 kDa to about 500 kDa, from about 100 kDa to about 400 kDa, from about 200 kDa to about 400 kDa, from about 300 kDa to about 400 kDa, or from about 310 kDa to about 375 kDa. In some embodiments, the first polymer has a molecular weight of about 10 kDa or more, for example about 15 kDa or more, about 20 kDa or more, about 30 kDa or more, about 40 kDa or more, about 50 kDa or more, about 60 kDa or more, about 70 kDa or more, about 90 kDa or more, about 90 kDa or more, or about 100 kDa or more.

In some embodiments, the first polymer as used in the inner layer comprises fibers. In some embodiments, the fibers can have a diameter from about 50 nm to about 1000 nm, for example from about 100 nm to about 400 nm. In some embodiments, the fibers can have a diameter from about 50 nm to about 1000 nm, from about 100 nm to about 1000 nm, from about 200 nm to about 1000 nm, from about 400 nm to about 1000 nm, from about 600 nm to about 1000 nm, from about 800 nm to about 1000 nm, from about 50 nm to about 800 nm, from about 100 nm to about 800 nm, from about 200 nm to about 800 nm, from about 400 nm to about 800 nm, from about 600 nm to about 800 nm, from about 50 nm to about 600 nm, from about 100 nm to about 600 nm, from about 200 nm to about 600 nm, from about 400 nm to about 600 nm, from about 50 nm to about 400 nm, from about 100 nm to about 400 nm, from about 200 nm to about 400 nm, from about 50 nm to about 200 nm, from about 100 nm to about 200 nm, or from about 50 nm to about 100 nm.

In some embodiments, the second polymer may additionally comprise a poly-lactic acid (PLA), a poly-glycolic acid (PGA), a poly-lactide-co-glycolide (PLGA), a polyester, a poly(other ester), a poly(phosphazine), a poly(phosphate ester), a gelatin, a collagen, a polyethylene glycol (PEG), derivatives thereof, and combinations thereof. In other embodiments, the second polymer may additionally comprise PLGA, PLA, PGA, PEG, polysorbate, poly(ε-caprolactone-thioethyl ethylene phosphate) (PCLEEP), polyvinyl alcohol (PVA), or combinations thereof. In some embodiments, the second polymer additionally comprises PLGA, PLA, PGA, or combinations thereof. In some embodiments, the second polymer may additionally comprise PLGA, PLA, or combinations thereof.

In some embodiments, the second polymer has a molecular weight of from about 50 kDa to about 500 kDa, for example from about 100 kDa to about 500 kDa, from about 100 kDa to about 400 kDa, from about 200 kDa to about 400 kDa, from about 300 kDa to about 400 kDa, or from about 310 kDa to about 375 kDa. In some embodiments, the second polymer has a molecular weight of about 10 kDa or more, for example about 15 kDa or more, about 20 kDa or more, about 30 kDa or more, about 40 kDa or more, about 50 kDa or more, about 60 kDa or more, about 70 kDa or more, about 90 kDa or more, about 90 kDa or more, or about 100 kDa or more.

In some embodiments, the second polymer is biodegradable *in vivo* and well tolerated throughout the duration of the presence and degradation of the composition. In some embodiments, under physiological conditions the second polymer degrades by random chain scission, which gives rise to a two-phase degradation. Initially, as molecular weight decreases the physical structure is not significantly affected. Degradation takes places throughout the polymer material, and proceeds until a critical molecular weight is reached, when degradation products become small enough to be solubilized. At this point, the structure starts to become significantly more porous and hydrated. In some embodiments, the second polymer has a molecular weight of about 90 kDa or more and does not degrade until after 6 months or more in the eye of a subject. In some embodiments, the molecular weight of the biodegradable polymer is selected so as to tune the degradation time of the material in vivo.

In some embodiments, the second polymer may comprise a blend of a high molecular weight polymer and a low molecular weight polymer. In some embodiments, the high molecular weight polymer may be of about 25 kDa or more (for example, about 30 kDa or more, 40 kDa or more, 50 kDa or more, 60 kDa or more, 70 kDa or more, 80 kDa or more, 90 kDa or more, or 100 kDa or more) and the low molecular weight polymer may be of about 20 kDa or less (for example 15 kDa or less, 10 kDa or less, 8 kDa or less, 6 kDa or less, or 4 kDa or less). In some embodiments, the ratio of high molecular weight polymer to lower molecular weight polymer is between about 1:9 to about 9:1, for example between about 2:8 to about 8:2, between about 2:8 to about 6:4, or between about 2:8 to about 1:1.

In some embodiments, the outer layer of the second polymer as used in the outer layer comprises fibers. In some embodiments, the fibers can have a diameter from about 100 nm to about 2000 nm, for example from about 500 nm to about 1000 nm. In some embodiments, the fibers can have a diameter from about 100 nm to about 2000 nm, from about 250 nm to about 2000 nm, from about 500 nm to about 2000 nm, from about 750 nm to about 2000 nm, from about 1000 nm to about 2000 nm, from about 1500 nm to about 2000 nm, from about 100 nm to about 1500 nm, from about 250 nm to about 1500 nm, from about 500 nm to about 1500 nm, from about 750 nm to about 1500 nm, from about 1000 nm to about 1500 nm, from about 100 nm to about 1000 nm, from about 250 nm to about 1000 nm, from about 500 nm to about 1000 nm, from about 750 nm to about 1000 nm, from about 100 nm to about 750 nm, from about 250 nm to about 750 nm, from about 500 nm to about 750 nm, from about 100 nm to about 500 nm, from about 250 nm to about 500 nm, or from about 100 nm to about 250 nm.

In some embodiments, the outer layer may further comprise pores. In other embodiments, the outer layer does not comprise pores. In some embodiments, the outer layer comprises pores having an average pore diameter from about 1 nm to about 990 nm, for example from about 1 nm to about 100 nm, from about 2 nm to about 700 nm, from about 3 nm to about 400 nm, from about 5 nm to about 200 nm, or from about 7 nm to about 50 nm. In some embodiments, the outer layer comprises pores having an average pore diameter from about 100 nm to 1000 nm, for example from 350 nm to 650 nm. In some embodiments, the outer layer comprises pores having an average pore diameter from about 100 nm to about 1000 nm, from 200 nm to about 1000 nm, from 300 nm to about 1000 nm, from about 400 nm to about 1000 nm, from about 450 nm to about 1000 nm, from about 500 nm to about 1000 nm, from about 550 nm to about 1000 nm, from about 600 nm to about 1000 nm, from about 650 nm to about 1000 nm, from about 700 nm to about 1000 nm, from about 800 nm to about 1000 nm, from about 900 nm to about 1000 nm, from about 100 nm to about 900 nm, from 200 nm to about 900 nm, from 300 nm to about 900 nm, from about 400 nm to about 900 nm, from about 450 nm to about 900 nm, from about 500 nm to about 900 nm, from about 550 nm to about 900 nm, from about 600 nm to about 900 nm, from about 650 nm to about 900 nm, from about 700 nm to about 900 nm, from about 800 nm to about 900 nm, from about 100 nm to about 800 nm, from 200 nm to about 800 nm, from 300 nm to about 800 nm, from about 400 nm to about 800 nm, from about 450 nm to about 800 nm, from about 500 nm to about 800 nm, from about 550 nm to about 800 nm, from about 600 nm to about 800 nm, from about 650 nm to about 800 nm, from about 700 nm to about 800 nm, from about 100 nm to about 700 nm, from 200 nm to about 700 nm, from 300 nm to about 700 nm, from about 400 nm to about 700 nm, from about 450 nm to about 700 nm, from about 500 nm to about 700 nm, from about 550 nm to about 700 nm, from about 600 nm to about 700 nm, from about 650 nm to about 700 nm, from about 100 nm to about 650 nm, from 200 nm to about 650 nm, from 300 nm to about 650 nm, from about 400 nm to about 650 nm, from about 450 nm to about 650 nm, from about 500 nm to about 650 nm, from about 550 nm to about 650 nm, from about 600 nm to about 650 nm, from about 100 nm to about 600 nm, from 200 nm to about 600 nm, from 300 nm to about 600 nm, from about 400 nm to about 600 nm, from about 450 nm to about 600 nm, from about 500 nm to about 600 nm, from about 550 nm to about 600 nm, from about 100 nm to about 550 nm, from 200 nm to about 550 nm, from 300 nm to about 550 nm, from about 400 nm to about 550 nm, from about 450 nm to about 550 nm, from about 500 nm to about 550 nm, from about 100 nm to about 500 nm, from 200 nm to about 500 nm, from 300 nm to about 500 nm, from about 400 nm to about 500 nm, from about 450 nm to about 500 nm, from about 100 nm to about 450 nm, from 200 nm to about 450 nm, from 300 nm to about 450 nm, from about 400 nm to about 450 nm, from about 100 nm to about 400 nm, from 200 nm to about 400 nm, from 300 nm to about 400 nm, from about 100 nm to about 300 nm, from about 200 nm to about 300 nm, and from about 100 nm to about 200 nm. In some embodiments, the average pore size is similar to the size of the therapeutic agent such that the one or more therapeutic agents diffuse via single file diffusion or hindered diffusion through nanopores. Pores may not be necessary when the desired therapeutic agent is of sufficient small size (for example, having a molecular weight of less than 500) that it may readily diffuse through the outer layer of the capsule.

In some embodiments, the composition of the first polymer or the second polymer may provide a melting temperature between about 50 °C to about 70 °C. In some embodiments, the composition of the first polymer or the second polymer is selected to provide a glass transition temperature (T_{g}) of between about -50 °C to about -80 °C.

In some embodiments, each of the one or more capsules may independently have a surface charge measures as a zeta potential at pH 7.5 of from about -25 mV to about 25 mV, for example from about -20 mV to about 20 mV, from about -15 mV to about 15 mV, from about -10 mV to about 10 mV, from about -5 mV to about 5 mV, from about -1 mV to about 1 mV, from about - 0.5 mV to about 0.5 mV, or from about -0.1 mV to about 0.1 mV.

In some embodiments, the composition of the first polymer and second polymer are selected such that 50% of the mass for one or more of the layers remains after at least three months when subjected to physiological conditions. If desirably, the degradation rate of either one or more of the layers may be accelerated by tuning such aspects in the manufacture of the capsules such as the thickness or porosity of the layer or by increasing the hydrophilicity of the polymer composition used to manufacture the one or more layers.

### Therapeutic Agents

In a further aspect, the present disclosure also provides one or more therapeutic agents that can be used in the compositions disclosed herein.

The one or more therapeutic agents each have a net negative charge within a pH range from about 6.0 to about 7.4.

As used herein, a "therapeutic agent" refers to one or more therapeutic agents, active ingredients, or substances that can be used to treat a medical condition of the eye or a cancer. The therapeutic agents are typically ophthalmically acceptable and are provided in a form that does not cause adverse reactions when the compositions disclosed herein are placed in an eye. As discussed herein, the therapeutic agents can be released from the disclosed compositions in a biologically active form. For example, the therapeutic agents may retain their three-dimensional structure when released from the system into an eye.

It is further understood, that as used herein, the terms "therapeutic agent" includes any synthetic or naturally occurring biologically active compound or composition of matter which, when administered to an organism (human or nonhuman animal), induces a desired pharmacologic, immunogenic, and/or physiologic effect by local and/or systemic action. The term therefore encompasses those compounds or chemicals traditionally regarded as drugs, vaccines, and biopharmaceuticals including molecules such as proteins, peptides, hormones, nucleic acids, gene constructs and the like. Examples of therapeutic agents are described in well-known literature references such as the Merck Index (14th edition), the Physicians' Desk Reference (64th edition), and The Pharmacological Basis of Therapeutics (12th edition), and they include, without limitation, medicaments; vitamins; mineral supplements; substances used for the treatment, prevention, diagnosis, cure or mitigation of a disease or illness; substances that affect the structure or function of the body, or pro-drugs, which become biologically active or more active after they have been placed in a physiological environment. For example, the term "therapeutic agent" includes compounds or compositions for use in all of the major therapeutic areas including, but not limited to, adjuvants; anti-infectives such as antibiotics and antiviral agents; analgesics and analgesic combinations, anorexics, anti- inflammatory agents, anti-epileptics, local and general anesthetics, hypnotics, sedatives, antipsychotic agents, neuroleptic agents, antidepressants, anxiolytics, antagonists, neuron blocking agents, anticholinergic and cholinomimetic agents, antimuscarinic and muscarinic agents, antiadrenergics, antiarrhythmics, antihypertensive agents, hormones, and nutrients, antiarthritics, antiasthmatic agents, anticonvulsants, antihistamines, antinauseants, antineoplastics, antipruritics, antipyretics; antispasmodics, cardiovascular preparations (including calcium channel blockers, beta-blockers, beta-agonists and antiarrythmics), antihypertensives, diuretics, vasodilators; central nervous system stimulants; cough and cold preparations; decongestants; diagnostics; hormones; bone growth stimulants and bone resorption inhibitors; immunosuppressives; muscle relaxants; psychostimulants; sedatives; tranquilizers; proteins, peptides, and fragments thereof (whether naturally occurring, chemically synthesized or recombinantly produced); and nucleic acid molecules (polymeric forms of two or more nucleotides, either ribonucleotides (RNA) or deoxyribonucleotides (DNA) including both double- and single-stranded molecules, gene constructs, expression vectors, antisense molecules and the like), small molecules (*e.g.,* doxorubicin) and other biologically active macromolecules such as, for example, proteins and enzymes. The agent may be a biologically active agent used in medical, including veterinary, applications and in agriculture, such as with plants, as well as other areas. The term therapeutic agent also includes without limitation, medicaments; vitamins; mineral supplements; substances used for the treatment, prevention, diagnosis, cure or mitigation of disease or illness; or substances which affect the structure or function of the body; or pro- drugs, which become biologically active or more active after they have been placed in a predetermined physiological environment.

In some embodiments, the therapeutic agent may comprise an agent useful in the treatment of an ophthalmological disorder or an eye disease such as: beta-blockers including timolol, betaxolol, levobetaxolol, and carteolol; miotics including pilocarpine; carbonic anhydrase inhibitors; serotonergics; muscarinics; dopaminergic agonists; adrenergic agonists including apraclonidine and brimonidine; anti- angiogenesis agents; anti-infective agents including quinolones such as ciprofloxacin and aminoglycosides such as tobramycin and gentamicin; non-steroidal and steroidal anti- inflammatory agents, such as suprofen, diclofenac, ketorolac, rimexolone and tetrahydrocortisol; growth factors, such as EGF; immunosuppressant agents; and anti-allergic agents including olopatadine; prostaglandins such as latanoprost; 15-keto latanoprost; travoprost; and unoprostone isopropyl.

In some embodiments, the therapeutic agent is selected from the group consisting of an anti-inflammatory agent, a calcineurin inhibitor, an antibiotic, a nicotinic acetylcholine receptor agonist, and an anti-lymphangiogenic agent. In some embodiments, the anti-inflammatory agent may be cyclosporine. In some embodiments, the calcineurin inhibitor may be voclosporin. In some embodiments, the antibiotic may be selected from the group consisting of amikacin, gentamycin, kanamycin, neomycin, netilmicin, streptomycin, tobramycin, teicoplanin, vancomycin, azithromycin, clarithromycin, dirithromycin, erythromycin, roxithromycin, troleandomycin, amoxicillin, ampicillin, azlocillin, carbenicillin, cloxacillin, dicloxacillin, flucloxacillin, mezlocillin, nafcillin, penicillin, piperacillin, ticarcillin, bacitracin, colistin, polymyxin B, ciprofloxacin, enoxacin, gatifloxacin, levofloxacin, lomefloxacin, moxifloxacin, norfloxacin, ofloxacin, trovafloxacin, mafenide, sulfacetamide, sulfamethizole, sulfasalazine, sulfisoxazole, trimethoprim, cotrimoxazole, demeclocycline, doxycycline, minocycline, oxytetracycline, and tetracycline. In some embodiments, the nicotinic acetylcholine receptor agonist may be any of pilocarpine, atropine, nicotine, epibatidine, lobeline, or imidacloprid. In some embodiments, the anti- lymphangiogenic agent may be a vascular endothelial growth factor C (VEGF-C) antibody, a VEGF-D antibody or a VEGF-3 antibody.

In some aspects, the therapeutic agent may be selected from: a beta-blocker, including levobunolol (BETAGAN), timolol (BETIMOL, TIMOPTIC), betaxolol (BETOPTIC) and metipranolol (OPTIPRANOLOL); alpha-agonists, such as apraclonidine (IOPIDINE) and brimonidine (ALPHAGAN); carbonic anhydrase inhibitors, such as acetazolamide, methazolamide, dorzolamide (TRUSOPT) and brinzolamide (AZOPT); prostaglandins or prostaglandin analogs such as latanoprost (XALATAN), bimatoprost (LUMIGAN) and travoprost (TRAVATAN); miotic or cholinergic agents, such as pilocarpine (ISOPTO CARPINE, PILOPINE) and carbachol (ISOPTO CARBACHOL); epinephrine compounds, such as dipivefrin (PROPINE); forskolin; or neuroprotective compounds, such as brimonidine and memantine; a steroid derivative, such as 2-methoxyestradiol or analogs or derivatives thereof; or an antibiotic.

The term "VEGF" refers to a vascular endothelial growth factor that induces angiogenesis or an angiogenic process, including, but not limited to, increased permeability. As used herein, the term "VEGF" includes the various subtypes of VEGF (also known as vascular permeability factor (VPF) and VEGF-A) that arise by, e.g., alternative splicing of the VEGF-A/VPF gene including VEGF121, VEGF165 and VEGF189. Further, as used herein, the term "VEGF" includes VEGF-related angiogenic factors such as PIGF (placental growth factor), VEGF-B, VEGF-C, VEGF-D and VEGF-E, which act through a cognate VEFG receptor (i.e., VEGFR) to induce angiogenesis or an angiogenic process. The term "VEGF" includes any member of the class of growth factors that binds to a VEGF receptor such as VEGFR-1 (Flt-1), VEGFR-2 (KDR/Flk-1), or VEGFR-3 (FLT-4). The term "VEGF" can be used to refer to a "VEGF" polypeptide or a "VEGF" encoding gene or nucleic acid.

The term "anti-VEGF agent" refers to an agent that reduces, or inhibits, either partially or fully, the activity or production of a VEGF. An anti-VEGF agent can directly or indirectly reduce or inhibit the activity or production of a specific VEGF such as VEGF165. Furthermore, "anti-VEGF agents" include agents that act on either a VEGF ligand or its cognate receptor so as to reduce or inhibit a VEGF-associated receptor signal. Non-limiting examples of "anti- VEGF agents" include antisense molecules, ribozymes or RNAi that target a VEGF nucleic acid; anti-VEGF aptamers, anti-VEGF antibodies to VEGF itself or its receptor, or soluble VEGF receptor decoys that prevent binding of a VEGF to its cognate receptor; antisense molecules, ribozymes, or RNAi that target a cognate VEGF receptor (VEGFR) nucleic acid; anti-VEGFR aptamers or anti-VEGFR antibodies that bind to a cognate VEGFR receptor; and VEGFR tyrosine kinase inhibitors.

In some embodiments, the therapeutic agent may comprise an anti-VEGF agent. Representative examples of anti-VEGF agents include ranibizumab, bevacizumab, aflibercept, KH902 VEGF receptor-Fc, fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, beta-, hydroxyisovalerylshikonin, ganglioside GM3, DC101 antibody, Mab25 antibody, Mab73 antibody, 4A5 antibody, 4E10 antibody, 5F12 antibody, VA01 antibody, BL2 antibody, VEGF-related protein, sFLT01, sFLT02, Peptide B3, TG100801, sorafenib, G6-31 antibody, a fusion antibody and an antibody that binds to an epitope of VEGF. Additional non-limiting examples of anti-VEGF agents useful in the present methods include a substance that specifically binds to one or more of a human vascular endothelial growth factor-A (VEGF-A), human vascular endothelial growth factor-B (VEGF-B), human vascular endothelial growth factor-C (VEGF-C), human vascular endothelial growth factor-D (VEGF-D) and human vascular endothelial growth, factor-E (VEGF-E), and an antibody that binds, to an epitope of VEGF.

In various aspects, the anti-VEGF agent is the antibody ranibizumab or a pharmaceutically acceptable salt thereof. Ranibizumab is commercially available under the trademark LUCENTIS. In another embodiment, the anti-VEGF agent is the antibody bevacizumab or a pharmaceutically acceptable salt thereof. Bevacizumab is commercially available under the trademark AVASTIN. In another embodiment, the anti-VEGF agent is aflibercept or a pharmaceutically acceptable salt thereof. Aflibercept is commercially available under the trademark EYLEA. In one embodiment, the anti-VEGF agent is pegaptanib or a pharmaceutically acceptable salt thereof. Pegaptinib is commercially available under the trademark MACUGEN. In another embodiment, the anti-VEGF agent is an antibody or an antibody fragment that binds to an epitope of VEGF, such as an epitope of VEGF-A, VEGF- B, VEGF-C, VEGF-D, or VEGF-E. In some embodiments, the VEGF antagonist binds to an epitope of VEGF such that binding of VEGF and VEGFR are inhibited. In one embodiment, the epitope encompasses a component of the three dimensional structure of VEGF that is displayed, such that the epitope is exposed on the surface of the folded VEGF molecule. In one embodiment, the epitope is a linear amino acid sequence from VEGF.

In various aspects, the therapeutic agent may comprise an agent that blocks or inhibits VEGF-mediated activity, e.g., one or more VEGF antisense nucleic acids. The present disclosure provides the therapeutic or prophylactic use of nucleic acids comprising at least six nucleotides that are antisense to a gene or cDNA encoding VEGF or a portion thereof. As used herein, a VEGF "antisense" nucleic acid refers to a nucleic acid capable of hybridizing by virtue of some sequence complementarity to a portion of an RNA (preferably mRNA) encoding VEGF. The antisense nucleic acid may be complementary to a coding and/or noncoding region of an mRNA encoding VEGF. Such antisense nucleic acids have utility as compounds that prevent VEGF expression, and can be used in the treatment of diabetes. The antisense nucleic acids of the disclosure are double-stranded or single-stranded oligonucleotides, RNA or DNA or a modification or derivative thereof, and can be directly administered to a cell or produced intracellularly by transcription of exogenous, introduced sequences.

The VEGF antisense nucleic acids are of at least six nucleotides and are preferably oligonucleotides ranging from 6 to about 50 oligonucleotides. In specific aspects, the oligonucleotide is at least 10 nucleotides, at least 15 nucleotides, at least 100 nucleotides, or at least 200 nucleotides. The oligonucleotides can be DNA or RNA or chimeric mixtures or derivatives or modified versions thereof and can be single-stranded or double-stranded. In addition, the antisense molecules may be polymers that are nucleic acid mimics, such as PNA, morpholino oligos, and LNA. Other types of antisense molecules include short double-stranded RNAs, known as siRNAs, and short hairpin RNAs, and long dsRNA (>50 bp but usually ≧500 bp).

In various aspects, the therapeutic agent may comprise one or more ribozyme molecule designed to catalytically cleave gene mRNA transcripts encoding VEGF, preventing translation of target gene mRNA and, therefore, expression of the gene product.

Ribozymes are enzymatic RNA molecules capable of catalyzing the specific cleavage of RNA. The mechanism of ribozyme action involves sequence-specific hybridization of the ribozyme molecule to complementary target RNA, followed by an endonucleolytic cleavage event. The composition of ribozyme molecules must include one or more sequences complementary to the target gene mRNA and must include the well-known catalytic sequence responsible for mRNA cleavage. For this sequence, see, e.g., U.S. Pat. No. 5,093,246. While ribozymes that cleave mRNA at site-specific recognition sequences can be used to destroy mRNAs encoding VEGF, the use of hammerhead ribozymes is preferred. Hammerhead ribozymes cleave mRNAs at locations dictated by flanking regions that form complementary base pairs with the target mRNA. The sole requirement is that the target mRNA has the following sequence of two bases: 5'-UG-3'. The construction and production of hammerhead ribozymes is well known in the art. The ribozymes of the present disclosure also include RNA endoribonucleases (hereinafter "Cech-type ribozymes") such as the one that occurs naturally in Tetrahymena thermophila (known as the IVS, or L-19 IVS RNA). The Cech-type ribozymes have an eight base pair active site that hybridizes to a target RNA sequence where after cleavage of the target RNA takes place. The disclosure encompasses those Cech-type ribozymes that target eight base-pair active site sequences that are present in the gene encoding VEGF.

In further aspects, the therapeutic agent may comprise an antibody that inhibits VEGF such as bevacizumab or ranibizumab. In still further aspects, therapeutic agent may comprise an agent that inhibits VEGF activity such as a tyrosine kinases stimulated by VEGF, examples of which include, but are not limited to lapatinib, sunitinib, sorafenib, axitinib, and pazopanib.

The term "anti-RAS agent" or "anti-Renin Angiotensin System agent" refers to refers to an agent that reduces, or inhibits, either partially or fully, the activity or production of a molecule of the renin angiotensin system (RAS). Non-limiting examples of "anti-RAS" or "anti- Renin Angiotensin System" molecules are one or more of an angiotensin-converting enzyme (ACE) inhibitor, an angiotensin-receptor blocker, and a renin inhibitor.

In some embodiments, the therapeutic agent may comprise a renin angiotensin system (RAS) inhibitor. In some embodiments, the renin angiotensin system (RAS) inhibitor is one or more of an angiotensin-converting enzyme (ACE) inhibitor, an angiotensin-receptor blocker, and a renin inhibitor.

Non limiting examples of angiotensin-converting enzyme (ACE) inhibitors which are useful in the present invention include, but are not limited to: alacepril, alatriopril, altiopril calcium, ancovenin, benazepril, benazepril hydrochloride, benazeprilat, benzazepril, benzoylcaptopril, captopril, captoprilcysteine, captoprilglutathione, ceranapril, ceranopril, ceronapril, cilazapril, cilazaprilat, converstatin, delapril, delaprildiacid, enalapril, enalaprilat, enalkiren, enapril, epicaptopril, foroxymithine, fosfenopril, fosenopril, fosenopril sodium, fosinopril, fosinopril sodium, fosinoprilat, fosinoprilic acid, glycopril, hemorphin-4, idapril, imidapril, indolapril, indolaprilat, libenzapril, lisinopril, lyciumin A, lyciumin B, mixanpril, moexipril, moexiprilat, moveltipril, muracein A, muracein B, muracein C, pentopril, perindopril, perindoprilat, pivalopril, pivopril, quinapril, quinapril hydrochloride, quinaprilat, ramipril, ramiprilat, spirapril, spirapril hydrochloride, spiraprilat, spiropril, spirapril hydrochloride, temocapril, temocapril hydrochloride, teprotide, trandolapril, trandolaprilat, utibapril, zabicipril, zabiciprilat, zofenopril, zofenoprilat, pharmaceutically acceptable salts thereof, and mixtures thereof.

Non limiting examples of angiotensin-receptor blockers which are useful in the present invention include, but are not limited to: irbesartan (U.S. Pat. No. 5,270,317), candesartan (U.S. Pat. Nos. 5,196,444 and 5,705,517), and losartan (U.S. Pat. No. 5,138,069).

Non limiting examples of renin inhibitors which may be used as therapeutic agents include, but are not limited to: aliskiren, ditekiren, enalkiren, remikiren, terlakiren, ciprokiren and zankiren, pharmaceutically acceptable salts thereof, and mixtures thereof.

The term "steroid" refers to compounds belonging to or related to the following illustrative families of compounds: corticosteroids, mineralicosteroids, and sex steroids (including, for example, potentially androgenic or estrogenic or anti-androgenic and anti- estrogenic molecules). Included among these are, for example, prednisone, prednisolone, methyl-prednisolone, triamcinolone, fluocinolone, aldosterone, spironolactone, danazol (otherwise known as OPTINA), and others. In some embodiments, the therapeutic agent may comprise a steroid.

The terms "peroxisome proliferator-activated receptor gamma agent," or "PPAR-γ agent," or "PPARG agent," or "PPAR-gamma agent" refers to agents which directly or indirectly act upon the peroxisome proliferator-activated receptor. This agent may also influence PPAR-alpha, "PPARA" activity.

In some embodiments, the therapeutic agent may comprise a modulator of macrophage polarization. Illustrative modulators of macrophage polarization include peroxisome proliferator activated receptor gamma (PPAR-g) modulators, including, for example, agonists, partial agonists, antagonists or combined PPAR-gamma/alpha agonists. In some embodiments, the therapeutic agent may comprise a PPAR gamma modulator, including PPAR gamma modulators that are full agonists or a partial agonists. In some embodiments, the PPAR gamma modulator is a member of the drug class of thiazolidinediones (TZDs, or glitazones). By way of non-limiting example, the PPAR gamma modulator may be one or more of rosiglitazone (AVANDIA), pioglitazone (ACTOS), troglitazone (REZULIN), netoglitazone, rivoglitazone, ciglitazone, rhodanine. In some embodiments, the PPAR gamma modulator is one or more of irbesartan and telmesartan. In some embodiments, the PPAR gamma modulator is a nonsteroidal anti-inflammatory drug (NSAID, such as, for example, ibuprofen) or an indole. Known inhibitors include the experimental agent GW-9662. Further examples of PPAR gamma modulators are described in WIPO Publication Nos. WO/1999/063983, WO/2001/000579, Nat Rev Immunol. 2011 Oct. 25; 11(11):750-61, or agents identified using the methods of WO/2002/068386.

In some embodiments, the PPAR gamma modulator is a "dual," or "balanced," or "pan" PPAR modulator. In some embodiments, the PPAR gamma modulator is a glitazar, which bind two or more PPAR isoforms, e.g., muraglitazar (Pargluva) and tesaglitazar (Galida) and aleglitazar.

In some embodiments, the therapeutic agent may comprise semapimod (CNI-1493) as described in Bianchi, et al. (March 1995). Molecular Medicine (Cambridge, Mass.) 1 (3): 254-266.

In some embodiments, the therapeutic agent may comprise a migration inhibitory factor (MIF) inhibitor. Illustrative MIF inhibitors are described in WIPO Publication Nos. WO 2003/104203, WO 2007/070961, WO 2009/117706 and U.S. Pat. Nos. 7,732,146 and 7,632,505, and 7,294,753 7,294,753. In some embodiments, the MIF inhibitor is (S,R)- 3-(4-hydroxyphenyl)-4,5-dihydro-5-isoxazole acetic acid methyl ester (ISO-1), isoxazoline, p425 (J. Biol. Chem., 287, 30653-30663), epoxyazadiradione, or vitamin E.

In some embodiments, the therapeutic agent may comprise a chemokine receptor 2 (CCR2) inhibitor as described in, for example, U.S. patent and Patent Publication Nos.: U.S. Pat. No. 7,799,824, U.S. Pat. No. 8,067,415, US 2007/0197590, US 2006/0069123, US 2006/0058289, and US 2007/0037794. In some embodiments, the CCR2) inhibitor is Maraviroc, cenicriviroc, CD192, CCX872, CCX140, 2-((Isopropylaminocarbonyl)amino)-N- (2-((cis-2-((4-(methylthio)benzoyl)amino)cyclohexyl)amino)-2-oxoethyl)-5-(trifluoromethyl)- benzamide, vicriviroc, SCH351125, TAK779, Teijin, RS-504393, compound 2, compound 14, or compound 19 (Plos ONE 7(3): e32864).

In some embodiments, the therapeutic agent may comprise an agent that modulates autophagy, microautophagy, mitophagy or other forms of autophagy. In some embodiments, the therapeutic agent may comprise sirolimus, tacrolimis, rapamycin, everolimus, bafilomycin, chloroquine, hydroxychloroquine, spautin-1, metformin, perifosine, resveratrol, trichostatin, valproic acid, Z-VAD-FMK, or others known to those in the art. Without wishing to be bound by theory, agent that modulates autophagy, microautophagy, mitophagy or other forms of autophagy may alter the recycling of intra-cellular components, for example, but not limited to, cellular organelles, mitochondria, endoplasmic reticulum, lipid or others. Without further wishing to be bound by theory, this agent may or may not act through microtubule-associated protein 1A/1Blight chain 3 (LC3).

In some embodiments, the therapeutic agent may comprise an agent used to treat cancer, i.e., a cancer drug or anti-cancer agent. Exemplary cancer drugs can be selected from antimetabolite anti- cancer agents and antimitotic anti-cancer agents, and combinations thereof, to a subject. Various antimetabolite and antimitotic anti-cancer agents, including single such agents or combinations of such agents, may be employed in the methods and compositions described herein.

Antimetabolic anti-cancer agents typically structurally resemble natural metabolites, which are involved in normal metabolic processes of cancer cells such as the synthesis of nucleic acids and proteins. The antimetabolites, however, differ enough from the natural metabolites such that they interfere with the metabolic processes of cancer cells. In the cell, antimetabolites are mistaken for the metabolites they resemble, and are processed by the cell in a manner analogous to the normal compounds. The presence of the "decoy" metabolites prevents the cells from carrying out vital functions and the cells are unable to grow and survive. For example, antimetabolites may exert cytotoxic activity by substituting these fraudulent nucleotides into cellular DNA, thereby disrupting cellular division, or by inhibition of critical cellular enzymes, which prevents replication of DNA.

In one aspect, therefore, the antimetabolite anti-cancer agent is a nucleotide or a nucleotide analog. In certain aspects, for example, the antimetabolite agent may comprise purine (e.g., guanine or adenosine) or analogs thereof, or pyrimidine (cytidine or thymidine) or analogs thereof, with or without an attached sugar moiety.

Suitable antimetabolite anti-cancer agents for use in the present disclosure may be generally classified according to the metabolic process they affect, and can include, but are not limited to, analogues and derivatives of folic acid, pyrimidines, purines, and cytidine. Thus, in one aspect, the antimetabolite agent(s) is selected from the group consisting of cytidine analogs, folic acid analogs, purine analogs, pyrimidine analogs, and combinations thereof.

In one particular aspect, for example, the antimetabolite agent is a cytidine analog. According to this aspect, for example, the cytidine analog may be selected from the group consisting of cytarabine (cytosine arabinodside), azacitidine (5-azacytidine), and salts, analogs, and derivatives thereof.

In another particular aspect, for example, the antimetabolite agent is a folic acid analog. Folic acid analogs or antifolates generally function by inhibiting dihydrofolate reductase (DHFR), an enzyme involved in the formation of nucleotides; when this enzyme is blocked, nucleotides are not formed, disrupting DNA replication and cell division. According to certain aspects, for example, the folic acid analog may be selected from the group consisting of denopterin, methotrexate (amethopterin), pemetrexed, pteropterin, raltitrexed, trimetrexate, and salts, analogs, and derivatives thereof.

In another particular aspect, for example, the antimetabolite agent is a purine analog. Purine-based antimetabolite agents function by inhibiting DNA synthesis, for example, by interfering with the production of purine containing nucleotides, adenine and guanine which halts DNA synthesis and thereby cell division. Purine analogs can also be incorporated into the DNA molecule itself during DNA synthesis, which can interfere with cell division. According to certain aspects, for example, the purine analog may be selected from the group consisting of acyclovir, allopurinol, 2-aminoadenosine, arabinosyl adenine (ara-A), azacitidine, azathiprine, 8-aza-adenosine, 8-fluoro-adenosine, 8-methoxy-adenosine, 8-oxo-adenosine, cladribine, deoxycoformycin, fludarabine, gancylovir, 8-aza-guanosine, 8-fluoro-guanosine, 8- methoxy-guanosine, 8-oxo-guanosine, guanosine diphosphate, guanosine diphosphate-beta- L-2-aminofucose, guanosine diphosphate-D-arabinose, guanosine diphosphate-2- fluorofucose, guanosine diphosphate fucose, mercaptopurine (6-MP), pentostatin, thiamiprine, thioguanine (6-TG), and salts, analogs, and derivatives thereof.

In yet another particular aspect, for example, the antimetabolite agent is a pyrimidine analog. Similar to the purine analogs discussed above, pyrimidine-based antimetabolite agents block the synthesis of pyrimidine-containing nucleotides (cytosine and thymine in DNA; cytosine and uracil in RNA). By acting as "decoys," the pyrimidine-based compounds can prevent the production of nucleotides, and/or can be incorporated into a growing DNA chain and lead to its termination. According to certain aspects, for example, the pyrimidine analog may be selected from the group consisting of ancitabine, azacitidine, 6-azauridine, bromouracil (e.g., 5-bromouracil), capecitabine, carmofur, chlorouracil (e.g. 5-chlorouracil), cytarabine (cytosine arabinoside), cytosine, dideoxyuridine, 3'-azido-3'-deoxythymidine, 3'- dideoxycytidin-2'-ene, 3'-deoxy-3'-deoxythymidin-2'-ene, dihydrouracil, doxifluridine, enocitabine, floxuridine, 5-fluorocytosine, 2-fluorodeoxycytidine, 3-fluoro-3'-deoxythymidine, fluorouracil (e.g., 5-fluorouracil (also known as 5-FU), gemcitabine, 5-methylcytosine, 5- propynylcytosine, 5-propynylthymine, 5-propynyluracil, thymine, uracil, uridine, and salts, analogs, and derivatives thereof. In one aspect, the pyrimidine analog is other than 5- fluorouracil. In another aspect, the pyrimidine analog is gemcitabine or a salt thereof.

In certain aspects, the antimetabolite agent is selected from the group consisting of 5-fluorouracil, capecitabine, 6-mercaptopurine, methotrexate, gemcitabine, cytarabine, fludarabine, pemetrexed, and salts, analogs, derivatives, and combinations thereof. In other aspects, the antimetabolite agent is selected from the group consisting of capecitabine, 6- mercaptopurine, methotrexate, gemcitabine, cytarabine, fludarabine, pemetrexed, and salts, analogs, derivatives, and combinations thereof. In one particular aspect, the antimetabolite agent is other than 5-fluorouracil. In a particularly preferred aspect, the antimetabolite agent is gemcitabine or a salt or thereof (e.g., gemcitabine HCl (Gemzar^{®})).

Other antimetabolite anti-cancer agents may be selected from, but are not limited to, the group consisting of acanthifolic acid, aminothiadiazole, brequinar sodium, Ciba-Geigy CGP-30694, cyclopentyl cytosine, cytarabine phosphate stearate, cytarabine conjugates, Lilly DATHF, Merrel Dow DDFC, dezaguanine, dideoxycytidine, dideoxyguanosine, didox, Yoshitomi DMDC, Wellcome EHNA, Merck & Co. EX-015, fazarabine, fludarabine phosphate, N-(2'-furanidyl)-5-fluorouracil, Daiichi Seiyaku FO-152, 5-FU-fibrinogen, isopropyl pyrrolizine, Lilly LY-188011; Lilly LY-264618, methobenzaprim, Wellcome MZPES, norspermidine, NCI NSC-127716, NCI NSC-264880, NCI NSC-39661, NCI NSC-612567, Warner-Lambert PALA, pentostatin, piritrexim, plicamycin, Asahi Chemical PL-AC, Takeda TAC-788, tiazofurin, Erbamont TIF, tyrosine kinase inhibitors, Taiho UFT and uricytin, among others.

In one aspect, the antimitotic agent is a microtubule inhibitor or a microtubule stabilizer. In general, microtubule stabilizers, such as taxanes and epothilones, bind to the interior surface of the beta-microtubule chain and enhance microtubule assembly by promoting the nucleation and elongation phases of the polymerization reaction and by reducing the critical tubulin subunit concentration required for microtubules to assemble. Unlike mictrotubule inhibitors, such as the vinca alkaloids, which prevent microtubule assembly, the microtubule stabilizers, such as taxanes, decrease the lag time and dramatically shift the dynamic equilibrium between tubulin dimers and microtubule polymers towards polymerization. In one aspect, therefore, the microtubule stabilizer is a taxane or an epothilone. In another aspect, the microtubule inhibitor is a vinca alkaloid.

In some embodiments, the therapeutic agent may comprise a taxane or derivative or analog thereof. The taxane may be a naturally derived compound or a related form, or may be a chemically synthesized compound or a derivative thereof, with antineoplastic properties. The taxanes are a family of terpenes, including, but not limited to paclitaxel (Taxol^{®}) and docetaxel (Taxotere^{®}), which are derived primarily from the Pacific yew tree, Taxus brevifolia, and which have activity against certain tumors, particularly breast and ovarian tumors. In one aspect, the taxane is docetaxel or paclitaxel. Paclitaxel is a preferred taxane and is considered an antimitotic agent that promotes the assembly of microtubules from tubulin dimers and stabilizes microtubules by preventing depolymerization. This stability results in the inhibition of the normal dynamic reorganization of the microtubule network that is essential for vital interphase and mitotic cellular functions.

Also included are a variety of known taxane derivatives, including both hydrophilic derivatives, and hydrophobic derivatives. Taxane derivatives include, but are not limited to, galactose and mannose derivatives described in International Patent Application No. WO 99/18113; piperazino and other derivatives described in WO 99/14209; taxane derivatives described in WO 99/09021, WO 98/22451, and U.S. Pat. No. 5,869,680; 6-thio derivatives described in WO 98/28288; sulfenamide derivatives described in U.S. Pat. No. 5,821,263; deoxygenated paclitaxel compounds such as those described in U.S. Pat. No. 5,440,056; and taxol derivatives described in U.S. Pat. No. 5,415,869. As noted above, it further includes prodrugs of paclitaxel including, but not limited to, those described in WO 98/58927; WO 98/13059; and U.S. Pat. No. 5,824,701. The taxane may also be a taxane conjugate such as, for example, paclitaxel-PEG, paclitaxel-dextran, paclitaxel-xylose, docetaxel-PEG, docetaxel- dextran, docetaxel-xylose, and the like. Other derivatives are mentioned in "Synthesis and Anticancer Activity of Taxol Derivatives," D. G. I. Kingston et al., Studies in Organic Chemistry, vol. 26, entitled "New Trends in Natural Products Chemistry" (1986), Atta-ur-Rabman, P. W. le Quesne, Eds. (Elsevier, Amsterdam 1986), among other references.

Various taxanes may be readily prepared utilizing techniques known to those skilled in the art (see also WO 94/07882, WO 94/07881, WO 94/07880, WO 94/07876, WO 93/23555, WO 93/10076; U.S. Pat. Nos. 5,294,637; 5,283,253; 5,279,949; 5,274,137; 5,202,448; 5,200,534; 5,229,529; and EP 590,267) or obtained from a variety of commercial sources, including for example, Sigma-Aldrich Co., St. Louis, Mo.

Alternatively, the antimitotic agent can be a microtubule inhibitor; in one preferred aspect, the microtubule inhibitor is a vinca alkaloid. In general, the vinca alkaloids are mitotic spindle poisons. The vinca alkaloid agents act during mitosis when chromosomes are split and begin to migrate along the tubules of the mitosis spindle towards one of its poles, prior to cell separation. Under the action of these spindle poisons, the spindle becomes disorganized by the dispersion of chromosomes during mitosis, affecting cellular reproduction. According to certain aspects, for example, the vinca alkaloid is selected from the group consisting of vinblastine, vincristine, vindesine, vinorelbine, and salts, analogs, and derivatives thereof.

The antimitotic agent can also be an epothilone. In general, members of the epothilone class of compounds stabilize microtubule function according to mechanisms similar to those of the taxanes. Epothilones can also cause cell cycle arrest at the G2-M transition phase, leading to cytotoxicity and eventually apoptosis. Suitable epithiolones include epothilone A, epothilone B, epothilone C, epothilone D, epothilone E, and epothilone F, and salts, analogs, and derivatives thereof. One particular epothilone analog is an epothilone B analog, ixabepilone (Ixempra^{™}).

In certain aspects, the antimitotic anti-cancer agent is selected from the group consisting of taxanes, epothilones, vinca alkaloids, and salts and combinations thereof. Thus, for example, in one aspect the antimitotic agent is a taxane. More preferably in this aspect the antimitotic agent is paclitaxel or docetaxel, still more preferably paclitaxel. In another aspect, the antimitotic agent is an epothilone (e.g., an epothilone B analog). In another aspect, the antimitotic agent is a vinca alkaloid.

Examples of cancer drugs that may be used in the present disclosure include, but are not limited to: thalidomide; platinum coordination complexes such as cisplatin (cis-DDP), oxaliplatin and carboplatin; anthracenediones such as mitoxantrone; substituted ureas such as hydroxyurea; methylhydrazine derivatives such as procarbazine (N- methylhydrazine, MIH); adrenocortical suppressants such as mitotane (o,p'-DDD) and aminoglutethimide; RXR agonists such as bexarotene; and tyrosine kinase inhibitors such as sunitimib and imatinib. Examples of additional cancer drugs include alkylating agents, antimetabolites, natural products, hormones and antagonists, and miscellaneous agents. Alternate names are indicated in parentheses. Examples of alkylating agents include nitrogen mustards such as mechlorethamine, cyclophosphainide, ifosfamide, melphalan sarcolysin) and chlorambucil; ethylenimines and methylmelamines such as hexamethylmelamine and thiotepa; alkyl sulfonates such as busulfan; nitrosoureas such as carmustine (BCNU), semustine (methyl-CCNU), lomustine (CCNU) and streptozocin (streptozotocin); DNA synthesis antagonists such as estramustine phosphate; and triazines such as dacarbazine (DTIC, dimethyl-triazenoimidazolecarboxamide) and temozolomide. Examples of antimetabolites include folic acid analogs such as methotrexate (amethopterin); pyrimidine analogs such as fluorouracin (5-fluorouracil, 5-FU, SFU), floxuridine (fluorodeoxyuridine, FUdR), cytarabine (cytosine arabinoside) and gemcitabine; purine analogs such as mercaptopurine (6-mercaptopurine, 6-MP), thioguanine (6-thioguanine, TG) and pentostatin (2'-deoxycoformycin, deoxycoformycin), cladribine and fludarabine; and topoisomerase inhibitors such as amsacrine. Examples of natural products include vinca alkaloids such as vinblastine (VLB) and vincristine; taxanes such as paclitaxel, protein bound paclitaxel (Abraxane) and docetaxel (Taxotere); epipodophyllotoxins such as etoposide and teniposide; camptothecins such as topotecan and irinotecan; antibiotics such as dactinomycin (actinomycin D), daunorubicin (daunomycin, rubidomycin), doxorubicin, bleomycin, mitomycin (mitomycin C), idarubicin, epirubicin; enzymes such as L-asparaginase; and biological response modifiers such as interferon alpha and interlelukin 2. Examples of hormones and antagonists include luteinising releasing hormone agonists such as buserelin; adrenocorticosteroids such as prednisone and related preparations; progestins such as hydroxyprogesterone caproate, rnedroxyprogesterone acetate and megestrol acetate; estrogens such as diethylstilbestrol and ethinyl estradiol and related preparations; estrogen antagonists such as tamoxifen and anastrozole; androgens such as testosterone propionate and fluoxymesterone and related preparations; androgen antagonists such as flutamide and bicalutamide; and gonadotropin-releasing hormone analogs such as leuprolide. Alternate names and trade-names of these and additional examples of cancer drugs, and their methods of use including dosing and administration regimens, will be known to a person versed in the art.

In some aspects, the anti-cancer agent may comprise a chemotherapeutic agent. Suitable chemotherapeutic agents include, but are not limited to, alkylating agents, antibiotic agents, antimetabolic agents, hormonal agents, plant-derived agents and their synthetic derivatives, anti-angiogenic agents, differentiation inducing agents, cell growth arrest inducing agents, apoptosis inducing agents, cytotoxic agents, agents affecting cell bioenergetics i.e., affecting cellular ATP levels and molecules/activities regulating these levels, biologic agents, e.g., monoclonal antibodies, kinase inhibitors and inhibitors of growth factors and their receptors, gene therapy agents, cell therapy, e.g., stem cells, or any combination thereof.

According to these aspects, the chemotherapeutic agent is selected from the group consisting of cyclophosphamide, chlorambucil, melphalan, mechlorethamine, ifosfamide, busulfan, lomustine, streptozocin, temozolomide, dacarbazine, cisplatin, carboplatin, oxaliplatin, procarbazine, uramustine, methotrexate, pemetrexed, fludarabine, cytarabine, fluorouracil, floxuridine, gemcitabine, capecitabine, vinblastine, vincristine, vinorelbine, etoposide, paclitaxel, docetaxel, doxorubicin, daunorubicin, epirubicin, idarubicin, mitoxantrone, bleomycin, mitomycin, hydroxyurea, topotecan, irinotecan, amsacrine, teniposide, erlotinib hydrochloride and combinations thereof. Each possibility represents a separate aspect of the invention.

According to certain aspects, the therapeutic agent may comprise a biologic drug, particularly an antibody. According to some aspects, the antibody is selected from the group consisting of cetuximab, anti-CD24 antibody, panitumumab and bevacizumab.

Therapeutic agents as used in the present disclosure may comprise peptides, proteins such as hormones, enzymes, antibodies, monoclonal antibodies, antibody fragments, monoclonal antibody fragments, and the like, nucleic acids such as aptamers, siRNA, DNA, RNA, antisense nucleic acids or the like, antisense nucleic acid analogs or the like, low-molecular weight compounds, or high-molecular-weight compounds, receptor agonists, receptor antagonists, partial receptor agonists, and partial receptor antagonists.

Additional representative therapeutic agents may include, but are not limited to, peptide drugs, protein drugs, desensitizing materials, antigens, factors, growth factors, anti-infective agents such as antibiotics, antimicrobial agents, antiviral, antibacterial, antiparasitic, antifungal substances and combination thereof, antiallergenics, steroids, androgenic steroids, decongestants, hypnotics, steroidal anti-inflammatory agents, anti-cholinergics, sympathomimetics, sedatives, miotics, psychic energizers, tranquilizers, vaccines, estrogens, progestational agents, humoral agents, prostaglandins, analgesics, antispasmodics, antimalarials, antihistamines, cardioactive agents, nonsteroidal anti-inflammatory agents, antiparkinsonian agents, anti-Alzheimer's agents, antihypertensive agents, beta-adrenergic blocking agents, alpha-adrenergic blocking agents, nutritional agents, and the benzophenanthridine alkaloids. The therapeutic agent can further be a substance capable of acting as a stimulant, a sedative, a hypnotic, an analgesic, an anticonvulsant, and the like.

Additional therapeutic agents may comprise CNS-active drugs, neuro-active drugs, inflammatory and anti-inflammatory drugs, renal and cardiovascular drugs, gastrointestinal drugs, anti-neoplastics, immunomodulators, immunosuppressants, hematopoietic agents, growth factors, anticoagulant, thrombolytic, antiplatelet agents, hormones, hormone-active agents, hormone antagonists, vitamins, ophthalmic agents, anabolic agents, antacids, anti-asthmatic agents, anti-cholesterolemic and anti-lipid agents, anti-convulsants, anti-diarrheals, anti-emetics, anti-manic agents, antimetabolite agents, anti-nauseants, anti-obesity agents, anti-pyretic and analgesic agents, anti-spasmodic agents, anti-thrombotic agents, anti-tussive agents, anti-uricemic agents, anti-anginal agents, antihistamines, appetite suppressants, biologicals, cerebral dilators, coronary dilators, bronchiodilators, cytotoxic agents, decongestants, diuretics, diagnostic agents, erythropoietic agents, expectorants, gastrointestinal sedatives, hyperglycemic agents, hypnotics, hypoglycemic agents, laxatives, mineral supplements, mucolytic agents, neuromuscular drugs, peripheral vasodilators, psychotropics, stimulants, thyroid and anti-thyroid agents, tissue growth agents, uterine relaxants, vitamins, antigenic materials, and so on. Other classes of therapeutic agents include those cited in Goodman & Gilman's The Pharmacological Basis of Therapeutics (McGraw Hill) as well as therapeutic agents included in the Merck Index and The Physicians' Desk Reference (Thompson Healthcare).

Other therapeutic agents include androgen inhibitors, polysaccharides, growth factors (e.g., a vascular endothelial growth factor-VEGF), hormones, anti-angiogenesis factors, dextromethorphan, dextromethorphan hydrobromide, noscapine, carbetapentane citrate, chlophedianol hydrochloride, chlorpheniramine maleate, phenindamine tartrate, pyrilamine maleate, doxylamine succinate, phenyltoloxamine citrate, phenylephrine hydrochloride, phenylpropanolamine hydrochloride, pseudoephedrine hydrochloride, ephedrine, codeine phosphate, codeine sulfate morphine, mineral supplements, cholestryramine, N-acetylprocainamide, acetaminophen, aspirin, ibuprofen, phenyl propanolamine hydrochloride, caffeine, guaifenesin, aluminum hydroxide, magnesium hydroxide, peptides, polypeptides, proteins, amino acids, hormones, interferons, cytokines, and vaccines.

Further examples of therapeutic agents include, but are not limited to, peptide drugs, protein drugs, desensitizing materials, antigens, anti-infective agents such as antibiotics, antimicrobial agents, antiviral, antibacterial, antiparasitic, antifungal substances and combination thereof, antiallergenics, androgenic steroids, decongestants, hypnotics, steroidal anti-inflammatory agents, anti-cholinergics, sympathomimetics, sedatives, miotics, psychic energizers, tranquilizers, vaccines, estrogens, progestational agents, humoral agents, prostaglandins, analgesics, antispasmodics, antimalarials, antihistamines, antiproliferatives, anti-VEGF agents, cardioactive agents, nonsteroidal anti-inflammatory agents, antiparkinsonian agents, antihypertensive agents, β-adrenergic blocking agents, nutritional agents, and the benzophenanthridine alkaloids. The agent can further be a substance capable of acting as a stimulant, sedative, hypnotic, analgesic, anticonvulsant, and the like.

Further representative therapeutic agents include but are not limited to analgesics such as acetaminophen, acetylsalicylic acid, and the like; anesthetics such as lidocaine, xylocaine, and the like; anorexics such as dexadrine, phendimetrazine tartrate, and the like; antiarthritics such as methylprednisolone, ibuprofen, and the like; antiasthmatics such as terbutaline sulfate, theophylline, ephedrine, and the like; antibiotics such as sulfisoxazole, penicillin G, ampicillin, cephalosporins, amikacin, gentamicin, tetracyclines, chloramphenicol, erythromycin, clindamycin, isoniazid, rifampin, and the like; antifungals such as amphotericin B, nystatin, ketoconazole, and the like; antivirals such as acyclovir, amantadine, and the like; anticancer agents such as cyclophosphamide, methotrexate, etretinate, paclitaxel, taxol, and the like; anticoagulants such as heparin, warfarin, and the like; anticonvulsants such as phenyloin sodium, diazepam, and the like; antidepressants such as isocarboxazid, amoxapine, and the like; antihistamines such as diphenhydramine HCl, chlorpheniramine maleate, and the like; hormones such as insulin, progestins, estrogens, corticoids, glucocorticoids, androgens, and the like; tranquilizers such as thorazine, diazepam, chlorpromazine HCl, reserpine, chlordiazepoxide HCl, and the like; antispasmodics such as belladonna alkaloids, dicyclomine hydrochloride, and the like; vitamins and minerals such as essential amino acids, calcium, iron, potassium, zinc, vitamin B12, and the like; cardiovascular agents such as prazosin HCl, nitroglycerin, propranolol HCl, hydralazine HCl, pancrelipase, succinic acid dehydrogenase, and the like; peptides and proteins such as LHRH, somatostatin, calcitonin, growth hormone, glucagon-like peptides, growth releasing factor, angiotensin, FSH, EGF, bone morphogenic protein (BMP), erythopoeitin (EPO), interferon, interleukin, collagen, fibrinogen, insulin, Factor VIII, Factor IX, Enbrel^{®}, Rituxam^{®}, Herceptin^{®}, alpha-glucosidase, Cerazyme/Ceredose^{®}, vasopressin, ACTH, human serum albumin, gamma globulin, structural proteins, blood product proteins, complex proteins, enzymes, antibodies, monoclonal antibodies, and the like; prostaglandins; nucleic acids; carbohydrates; fats; narcotics such as morphine, codeine, and the like, psychotherapeutics; anti-malarials, L-dopa, diuretics such as furosemide, spironolactone, and the like; antiulcer drugs such as rantidine HCl, cimetidine HCl, and the like.

The therapeutic agent can also be an immunomodulator, including, for example, cytokines, interleukins, interferon, colony stimulating factor, tumor necrosis factor, and the like; immunosuppressants such as rapamycin, tacrolimus, and the like; allergens such as cat dander, birch pollen, house dust mite, grass pollen, and the like; antigens of bacterial organisms such as Streptococcus pneumoniae, Haemophilus influenzae, Staphylococcus aureus, Streptococcus pyrogenes, Corynebacterium diphteriae, Listeria monocytogenes, Bacillus anthracis, Clostridium tetani, Clostridium botulinum, Clostridium perfringens. Neisseria meningitides, Neisseria gonorrhoeae, Streptococcus mutans. Pseudomonas aeruginosa, Salmonella typhi, Haemophilus parainfluenzae, Bordetella pertussis, Francisella tularensis, Yersinia pestis, Vibrio cholerae, Legionella pneumophila, Mycobacterium tuberculosis, Mycobacterium leprae, Treponema pallidum, Leptspirosis interrogans, Borrelia burgddorferi, Campylobacter jejuni, and the like; antigens of such viruses as smallpox, influenza A and B, respiratory synctial, parainfluenza, measles, HIV, SARS, varicella-zoster, herpes simplex 1 and 2, cytomeglavirus, Epstein-Barr, rotavirus, rhinovirus, adenovirus, papillomavirus, poliovirus, mumps, rabies, rubella, coxsackieviruses, equine encephalitis, Japanese encephalitis, yellow fever, Rift Valley fever, lymphocytic choriomeningitis, hepatitis B, and the like; antigens of such fungal, protozoan, and parasitic organisms such as Cryptococcus neoformans, Histoplasma capsulatum, Candida albicans, Candida tropicalis, Nocardia asteroids, Rickettsia ricketsii, Rickettsia typhi, Mycoplasma pneumoniae, Chlamydia psittaci, Chlamydia trachomatis, Plasmodium falciparum, Trypanasoma brucei, Entamoeba histolytica, Toxoplasma gondii, Trichomonas vaginalis, Schistosoma mansoni, and the like. These antigens may be in the form of whole killed organisms, peptides, proteins, glycoproteins, carbohydrates, or combinations thereof.

In a further specific aspect, the therapeutic agent can comprise an antibiotic. The antibiotic can be, for example, one or more of Amikacin, Gentamicin, Kanamycin, Neomycin, Netilmicin, Streptomycin, Tobramycin, Paromomycin, Ansamycins, Geldanamycin, Herbimycin, Carbacephem, Loracarbef, Carbapenems, Ertapenem, Doripenem, Imipenem/Cilastatin, Meropenem, Cephalosporins (First generation), Cefadroxil, Cefazolin, Cefalotin or Cefalothin, Cefalexin, Cephalosporins (Second generation), Cefaclor, Cefamandole, Cefoxitin, Cefprozil, Cefuroxime, Cephalosporins (Third generation), Cefixime, Cefdinir, Cefditoren, Cefoperazone, Cefotaxime, Cefpodoxime, Ceftazidime, Ceftibuten, Ceftizoxime, Ceftriaxone, Cephalosporins (Fourth generation), Cefepime, Cephalosporins (Fifth generation), Ceftobiprole, Glycopeptides, Teicoplanin, Vancomycin, Macrolides, Azithromycin, Clarithromycin, Dirithromycin, Erythromycin, Roxithromycin, Troleandomycin, Telithromycin, Spectinomycin, Monobactams, Aztreonam, Penicillins, Amoxicillin, Ampicillin, Azlocillin, Carbenicillin, Cloxacillin, Dicloxacillin, Flucloxacillin, Mezlocillin, Meticillin, Nafcillin, Oxacillin, Penicillin, Piperacillin, Ticarcillin, Polypeptides, Bacitracin, Colistin, Polymyxin B, Quinolones, Ciprofloxacin, Enoxacin, Gatifloxacin, Levofloxacin, Lomefloxacin, Moxifloxacin, Norfloxacin, Ofloxacin, Trovafloxacin, Sulfonamides, Mafenide, Prontosil (archaic), Sulfacetamide, Sulfamethizole, Sulfanilimide (archaic), Sulfasalazine, Sulfisoxazole, Trimethoprim, Trimethoprim-Sulfamethoxazole (Co-trimoxazole) (TMP-SMX), Tetracyclines, including Demeclocycline, Doxycycline, Minocycline, Oxytetracycline, Tetracycline, and others; Arsphenamine, Chloramphenicol, Clindamycin, Lincomycin, Ethambutol, Fosfomycin, Fusidic acid, Furazolidone, Isoniazid, Linezolid, Metronidazole, Mupirocin, Nitrofurantoin, Platensimycin, Pyrazinamide, Quinupristin/Dalfopristin, Rifampicin (Rifampin in U.S.), Timidazole, or a combination thereof. In one aspect, the therapeutic agent can be a combination of Rifampicin (Rifampin in U.S.) and Minocycline.

Growth factors useful as therapeutic agents include, but are not limited to, transforming growth factor-α ("TGF-α"), transforming growth factors ("TGF-β"), platelet-derived growth factors ("PDGF"), fibroblast growth factors ("FGF"), including FGF acidic isoforms 1 and 2, FGF basic form 2 and FGF 4, 8, 9 and 10, nerve growth factors ("NGF") including NGF 2.5s, NGF 7.0s and beta NGF and neurotrophins, brain derived neurotrophic factor, cartilage derived factor, bone growth factors (BGF), basic fibroblast growth factor, insulin-like growth factor (IGF), vascular endothelial growth factor (VEGF), granulocyte colony stimulating factor (G-CSF), insulin like growth factor (IGF) I and II, hepatocyte growth factor, glial neurotrophic growth factor (GDNF), stem cell factor (SCF), keratinocyte growth factor (KGF), transforming growth factors (TGF), including TGFs alpha, beta, beta1, beta2, beta3, skeletal growth factor, bone matrix derived growth factors, and bone derived growth factors and mixtures thereof.

Cytokines useful as therapeutic agents include, but are not limited to, cardiotrophin, stromal cell derived factor, macrophage derived chemokine (MDC), melanoma growth stimulatory activity (MGSA), macrophage inflammatory proteins 1 alpha (MIP-1alpha), 2, 3 alpha, 3 beta, 4 and 5, IL-1, IL-2, IL-3, IL-4, IL-5, IL-6, IL-7, IL-8, IL-9, IL-10, IL-11, IL-12, IL-13, TNF-α, and TNF-β. Immunoglobulins useful in the present disclosure include, but are not limited to, IgG, IgA, IgM, IgD, IgE, and mixtures thereof. Some preferred growth factors include VEGF (vascular endothelial growth factor), NGFs (nerve growth factors), PDGF-AA, PDGF-BB, PDGF-AB, FGFb, FGFa, and BGF.

Other molecules useful as therapeutic agents include but are not limited to growth hormones, leptin, leukemia inhibitory factor (LIF), tumor necrosis factor alpha and beta, endostatin, thrombospondin, osteogenic protein-1, bone morphogenetic proteins 2 and 7, osteonectin, somatomedin-like peptide, osteocalcin, , interferon alpha, interferon alpha A, interferon beta, interferon gamma, interferon 1 alpha, and interleukins 2, 3, 4, 5 6, 7, 8, 9, 10, 11, 12,13, 15, 16, 17 and 18.

In some embodiments, the therapeutic agent is present in the disclosed drug delivery composition in an amount (in µg of therapeutic agent per mg weight of the disclosed drug delivery composition) of about 10, about 20, about 30, about 40, about 45, about 50, about 55, about 60, about 65, about 70, about 75, about 80, about 85, about 90, about 95, about 100, about 110, about 120, about 130, about 140, about 150, about 160, about 170, about 180, about 190, about 200, about 210, about 220, about 230, about 240, about 250, about 260, about 270, about 280, about 290, about 300, about 310, about 320, about 330, about 340, about 350, about 360, about 370, about 380, about 390, about 400, about 410, about 420, about 430, about 440, about 450, about 460, about 470, about 480, about 490, or about 500; or an amount range of the therapeutic reagent bracketed by any of the foregoing values; or any combination of the foregoing values.

In some embodiments, the therapeutic agent exhibits near zero-order release kinetics over a period of at least 30 days, for example 45 days, 60 days, 3 months, 6 months, 9 months, 1 year, or more. The therapeutic agent may exhibit near zero-order release kinetics at the time of implanation of the drug delivery composition or after a period of time afterward, for example the therapeutic agent begins to exhibit near zero-order release kinetics about 30 days after implanation of the drug delivery composition. In other embodiments, the drug delivery composition may exhibit kinetics that deviate from zero-order kinetics.

### Methods of Preparing a Disclosed Drug Delivery Compositions

In various aspects, the disclosed drug delivery devices are prepared by methods disclosed herein below and as described in specific aspects in the representative Examples that follow.

Thus in one aspect, a method for preparing a drug delivery device described herein is provided comprising: forming a first layer comprising the first polymer on a conductive rod; and forming a second layer comprising the second polymer on the first layer.

In some embodiments, the forming a first layer comprises electrospinning using a solution of the first polymer and a voltage difference of about 10 kV to about 30 kV.

In some embodiments, the first polymer solution is about 1 w/v% to about 10 w/v% in at least one organic solvent. In some embodiments, the at least one organic solvent in the first polymer solution comprises trifluoroacetic acid, dichloromethane, hexafluoroisopropanol, or combinations thereof. In some embodiments, the trifluoroacetic acid and the dichloromethane are present in a ratio of about 1:10 to about 10:1, for example in a ratio of about 5:3 to about 10:3. In some embodiments, the trifluoroacetic acid and the dichloromethane are present in a ratio of about 7:3.

In some embodiments, the forming a second layer comprises electrospinning onto the formed first layer using a solution comprising the second polymer and optionally a porogen, wherein the voltage difference used for electrospinning is about 20 kV to about 30 kV.

In some embodiments, the solution comprising the second polymer and optionally the porogen is about 1 w/v% to about 10 w/v% based on the total weight of the second polymer and the porogen, for example from about 2.5 w/v% to about 10 w/v% or from about 5 w/v% to about 10 w/v%. In some embodiments, the solution comprising the second polymer and the porogen is a 1,1,1,3,3,3,-hexafluoropropan-2-ol solution.

In some embodiments, the weight ratio of the second polymer to the porogen is about 90:100 to about 100:1, for example from about 90:100 to 99.9:0.1, from 90:100 to about 95:5, or from 95:5 to about 99.9:0.1. In some embodiments, the weight ratio of the second polymer to the porogen is about 99:1, about 95:5, about 92.5:7.5, or about 90:10. In some embodiments, the weight ratio of the second polymer to the porogen ranges from about 50:50 to about 100:0.

A "porogen" as used herein refers to any material that can be used to create a porous material, e.g. porous polycaprolactone as described herein. In some embodiments, the porogen comprises a water-soluble compound, i.e. such that the porogen is substantially removed from the outer layer upon washing the drug delivery device with water. In some embodiments, the porogen comprises a compound selected from ([Tris(hydroxymethyl)methylamino]propanesulfonic acid) (TAPS), (2-(Bis(2-hydroxyethyl)amino)acetic acid) (Bicine), (Tris(hydroxymethyl)aminomethane) or, (2-Amino-2-(hydroxymethyl)propane-1,3-diol) (Tris), (N-[Tris(hydroxymethyl)methyl]glycine) (Tricine), (3-[N-Tris(hydroxymethyl)methylamino]-2-hydroxypropanesulfonic acid) (TAPSO), (4-(2-hydroxyethyl)-1-piperazineethanesulfonic acid) (HEPES), (2-[[1,3-dihydroxy-2-(hydroxymethyl)propan-2-yl]amino]ethanesulfonic acid) (TES), (3-(N-morpholino)propanesulfonic acid) (MOPS), (Piperazine-N,N'-bis(2-ethanesulfonic acid)) (PIPES), Dimethylarsenic acid, (2-(N-morpholino)ethanesulfonic acid) (MES), or salts thereof, such as the sodium salts thereof. In some embodiments, the porogen comprises HEPES sodium salt. In some embodiments, the porogen comprises a water soluble polymer such as polyethylene glycol, polyoxyethylene copolymer, an acrylate copolymer including quaternary ammonium groups, a polyacrylamide, a polyvinyl alcohol, hyaluronan, and polyvinylpyrrolidone. In other embodiments, the porogen comprises gelatin, polyethylene glycol (PEG), chitosan, polyvinylpyrrolidone (PVP), polyvinyl alcohol, or agarose.

In some embodiments, the method further comprises sintering the drug delivery device following forming the outer layer. In some embodiments, sintering comprises at a temperature from about 50 °C to about 150 °C, for example from about 90 °C to about 110 °C. In some embodiments, sintering comprises heating for a period from about 1 minute to about 6 hours, for example from about 30 minutes to about 6 hours.

In some embodiments, the method further comprises washing the drug delivery device following sintering. In some embodiments, the drug delivery device is washed with a saturated sodium bicarbonate solution followed by deionized water. In some embodiments, the porogen is substantially removed from the drug delivery device upon washing with deionized water.

In some embodiments, the method further comprises drying the drug delivery device following washing. In some embodiments, drying is *in vacuo.* In some embodiments, drying is at a temperature of about 50 °C to about 150 °C, for example from about 90 °C to 110 °C. In some embodiments, drying occurs for a period from about 1 minute to about 6 hours, for example from about 30 minutes to about 6 hours.

In other embodiments, the disclosed capsules may be manufactured by any appropriate method as would be readily understood by those of ordinary skill in the art. In some embodiments, the disclosed capsules may be manufactured by asymmetric membrane formation; a representative example of such methods are provided in Yen, C. et al. "Synthesis and characterization of nanoporous polycaprolactone membranes via thermally- and nonsolvent-induced phase separations for biomedical device application" Journal of Membrane Science 2009, 343 180-88. In some embodiments, the disclosed capsules may be manufactured using three-dimensional printing. In some embodiments, the disclosed capsules may be manufactured around methylcellulose which is subsequently removed to form the luminal compartment. In some embodiments, the disclosed capsules may be manufactured by a method described by Envisia Therapeutics in WO 2015/085251, WO 2016/144832, WO 2016/196365, WO 2017/015604, WO 2017/015616, or WO 2017/015675. In yet other embodiments, the disclosed capsules may be manufactured by methods similar to those used in the manufacturing of hollow fiber membranes, such as phase inversion including non-solvent induced phase inversion (NIPS), (solvent) evaporation-induced phase inversion (EIPS), vapor sorption-induced phase inversion (VIPS), and thermally induced phase inversion (TIPS) In some embodiments, the disclosed capsules may be manufacturing using a method similar to the methods described in US 2015/232506. In some embodiments, the pores may instead by formed by laser diffraction of the capsules.

The two ends of the tubular shape of the capsule are closed. The ends may be closed by any number of sealing techniques as would be appropriately selected by one of skill in the art. In some embodiments, the two ends are sealed using a high frequency tube sealing technique. In such techniques, a high frequency generates an eddy current in the wall, which heats up at least the polymer layers. When the temperature has reached the melting point of the polymer, clamps are closed and the melted polymer is cooled and formed. In some embodiments, the two ends are sealed using hot-jaw tube sealing, where heated jaws apply heat to the outside of the tubular shape to heat up the inside for sealing. In some embodiments, the two ends may be sealed using ultrasonic tube sealing. In such techniques, the polymer composition of the inner layers is heated and melted by high frequency friction force introduced form an ultrasonic horn. Clamps are then closed around the section intended to be sealed, cooled, and formed to seal the ends. In some embodiments, the two ends are sealed using hot air sealing, wherein the system heats the seal area inside the capsule with hot air and then subsequently presses and chills the ends in a subsequent station.

### Methods of Treatment Using a Disclosed Drug Delivery Device

Methods of treating a clinical condition by administration of a disclosed drug delivery composition are also provided herein. Any method of treatment disclosed herein should be read as relating to the composition of the invention for use in such a method. A clinical condition can be a clinical disorder, disease, dysfunction or other condition that can be ameliorated by a therapeutic composition.

The term "administering" or "administration" of a disclosed drug delivery device to a subject includes any route of introducing or delivering to a subject the device to perform its intended function. Administration can be carried out by any suitable route, including orally, intranasally, parenterally (intravenously, intramuscularly, intraperitoneally, or subcutaneously), or topically. Administration includes self-administration and the administration by another. In some instances, administration is via injection to the eye, including intraocular injection. In other instances, for example, in treatment of a cancer, administration can be via injection of a disclosed drug delivery composition within, abutting, adjacent, or proximal to a tumor or other mass of cancer cells.

It is also to be appreciated that the various modes of treatment or prevention of medical diseases and conditions as described are intended to mean "substantial," which includes total but also less than total treatment or prevention, and wherein some biologically or medically relevant result is achieved. The treatment may be a continuous prolonged treatment for a chronic disease or a single, or few time administrations for the treatment of an acute condition.

The term "separate" administration refers to an administration of at least two active ingredients at the same time or substantially the same time by different routes.

The term "sequential" administration refers to administration of at least two active ingredients at different times, the administration route being identical or different. More particularly, sequential use refers to the whole administration of one of the active ingredients before administration of the other or others commences. It is thus possible to administer one of the active ingredients over several minutes, hours, or days before administering the other active ingredient or ingredients. The term "sequential" therefore is different than "simultaneous" administration.

The term "simultaneous" administration refers to the administration of at least two active ingredients by the same route at the same time or at substantially the same time.

The term "therapeutic" as used herein means a treatment and/or prophylaxis. A therapeutic effect is obtained by suppression, remission, or eradication of a disease state.

The present disclosure further provides methods of treating an ophthalmological disease or disorder by administering a therapeutically effective amount of the compositions described herein. In some embodiments, the disclosed methods pertain to treatment of an ophthalmological disorder comprising injecting a therapeutically effective amount of the disclosed composition into the eye of a subject. The subject can be a patient; and the patient can have been diagnosed with an ophthalmological disorder. In some aspects, the method can further comprise diagnosing a subject with an ophthalmological disorder.

The ophthalmological disorder can be acute macular neuroretinopathy; Behcet's disease; neovascularization, including choroidal neovascularization; diabetic uveitis; histoplasmosis; infections, such as fungal or viral-caused infections; macular degeneration, such as acute macular degeneration (AMD), including wet AMD, non-exudative AMD and exudative AMD; edema, such as macular edema, cystoid macular edema and diabetic macular edema; multifocal choroiditis; ocular trauma which affects a posterior ocular site or location; ocular tumors; retinal disorders, such as central retinal vein occlusion, diabetic retinopathy (including proliferative diabetic retinopathy), proliferative vitreoretinopathy (PVR), retinal arterial occlusive disease, retinal detachment, uveitic retinal disease; sympathetic ophthalmia; Vogt Koyanagi-Harada (VKH) syndrome; uveal diffusion; a posterior ocular condition caused by or influenced by an ocular laser treatment; posterior ocular conditions caused by or influenced by a photodynamic therapy, photocoagulation, radiation retinopathy, epiretinal membrane disorders, branch retinal vein occlusion, anterior ischemic optic neuropathy, non-retinopathy diabetic retinal dysfunction, retinitis pigmentosa, a cancer, and glaucoma. In certain instances, the ophthalmological disorder is wet age-related macular degeneration (wet AMD), a cancer, neovascularization, macular edema, or edema. In a further particular aspect, the ophthalmological disorder is wet age-related macular degeneration (wet AMD).

In various aspects, the injection for treatment of an ophthalmological disorder can be injection to the vitreous chamber of the eye. In some cases, the injection is an intravitreal injection, a subconjunctival injection, a subtenon injection, a retrobulbar injection, or a suprachoroidal injection.

"Ocular region" or "ocular site" means any area of the ocular globe (eyeball), including the anterior and posterior segment of the eye, and which generally includes, but is not limited to, any functional (e.g., for vision) or structural tissues found in the eyeball, or tissues or cellular layers that partly or completely line the interior or exterior of the eyeball. Specific examples of areas of the eyeball in an ocular region include, but are not limited to, the anterior chamber, the posterior chamber, the vitreous cavity, the choroid, the suprachoroidal space, the conjunctiva, the subconjunctival space, the episcleral space, the intracorneal space, the subretinal space, sub-Tenon's space, the epicorneal space, the sclera, the pars plana, surgically-induced avascular regions, the macula, and the retina.

"Ophthalmological disorder" can mean a disease, ailment or condition which affects or involves the eye or one of the parts or regions of the eye. Broadly speaking, the eye includes the eyeball, including the cornea, and other tissues and fluids which constitute the eyeball, the periocular muscles (such as the oblique and rectus muscles) and the portion of the optic nerve which is within or adjacent to the eyeball.

"Glaucoma" means primary, secondary and/or congenital glaucoma. Primary glaucoma can include open angle and closed angle glaucoma. Secondary glaucoma can occur as a complication of a variety of other conditions, such as injury, inflammation, pigment dispersion, vascular disease and diabetes. The increased pressure of glaucoma causes blindness because it damages the optic nerve where it enters the eye. Thus, in one nonlimiting embodiment, by lowering reactive oxygen species, STC-1, or MSCs which express increased amounts of STC-1, may be employed in the treatment of glaucoma and prevent or delay the onset of blindness.

Inflammation-mediated" in relation to an ocular condition means any condition of the eye which can benefit from treatment with an anti-inflammatory agent, and is meant to include, but is not limited to, uveitis, macular edema, acute macular degeneration, retinal detachment, ocular tumors, fungal or viral infections, multifocal choroiditis, diabetic retinopathy, uveitis, proliferative vitreoretinopathy (PVR), sympathetic ophthalmia, Vogt-Koyanagi-Harada (VKH) syndrome, histoplasmosis, and uveal diffusion.

"Injury" or "damage" in relation to an ocular condition are interchangeable and refer to the cellular and morphological manifestations and symptoms resulting from an inflammatory-mediated condition, such as, for example, inflammation, as well as tissue injuries caused by means other than inflammation, such as chemical injury, including chemical burns, as well as injuries caused by infections, including but not limited to, bacterial, viral, or fungal infections.

"Intraocular" means within or under an ocular tissue. An intraocular administration of a drug delivery system includes administration of the drug delivery system to a sub-tenon, subconjunctival, suprachoroidal, subretinal, intravitreal, anterior chamber, and the like location. An intraocular administration of a drug delivery system excludes administration of the drug delivery system to a topical, systemic, intramuscular, subcutaneous, intraperitoneal, and the like location.

"Macular degeneration" refers to any of a number of disorders and conditions in which the macula degenerates or loses functional activity. The degeneration or loss of functional activity can arise as a result of, for example, cell death, decreased cell proliferation, loss of normal biological function, or a combination of the foregoing. Macular degeneration can lead to and/or manifest as alterations in the structural integrity of the cells and/or extracellular matrix of the macula, alteration in normal cellular and/or extracellular matrix architecture, and/or the loss of function of macular cells. The cells can be any cell type normally present in or near the macula including RPE cells, photoreceptors, and capillary endothelial cells. Age-related macular degeneration, or ARMD, is the major macular degeneration related condition, but a number of others are known including, but not limited to, Best macular dystrophy, Stargardt macular dystrophy, Sorsby fundus dystrophy, Mallatia Leventinese, Doyne honeycomb retinal dystrophy, and RPE pattern dystrophies. Age-related macular degeneration (AMD) is described as either "dry" or "wet." The wet, exudative, neovascular form of AMD affects about 10-20% of those with AMD and is characterized by abnormal blood vessels growing under or through the retinal pigment epithelium (RPE), resulting in hemorrhage, exudation, scarring, or serous retinal detachment. Eighty to ninety percent of AMD patients have the dry form characterized by atrophy of the retinal pigment epithelium and loss of macular photoreceptors. Drusen may or may not be present in the macula. There may also be geographic atrophy of retinal pigment epithelium in the macula accounting for vision loss. At present there is no cure for any form of AMD, although some success in attenuation of wet AMD has been obtained with photodynamic and especially anti-VEGF therapy.

"Drusen" is debris-like material that accumulates with age below the RPE. Drusen is observed using a funduscopic eye examination. Normal eyes may have maculas free of drusen, yet drusen may be abundant in the retinal periphery. The presence of soft drusen in the macula, in the absence of any loss of macular vision, is considered an early stage of AMD. Drusen contains a variety of lipids, polysaccharides, and glycosaminoglycans along with several proteins, modified proteins or protein adducts. There is no generally accepted therapeutic method that addresses drusen formation and thereby manages the progressive nature of AMD.

"Ocular neovascularization" (ONV) is used herein to refer to choroidal neovascularization or retinal neovascularization, or both.

"Retinal neovascularization" (RNV) refers to the abnormal development, proliferation, and/or growth of retinal blood vessels, e.g., on the retinal surface.

"Subretinal neovascularization" (SRNVM) refers to the abnormal development, proliferation, and/or growth of blood vessels beneath the surface of the retina.

"Cornea" refers to the transparent structure forming the anterior part of the fibrous tunic of the eye. It consists of five layers, specifically: 1) anterior corneal epithelium, continuous with the conjunctiva; 2) anterior limiting layer (Bowman's layer); 3) substantia propria, or stromal layer; 4) posterior limiting layer (Descemet's membrane); and 5) endothelium of the anterior chamber or keratoderma.

"Retina" refers to the innermost layer of the ocular globe surrounding the vitreous body and continuous posteriorly with the optic nerve. The retina is composed of layers including the: 1) internal limiting membrane; 2) nerve fiber layer; 3) layer of ganglion cells; 4) inner plexiform layer; 5) inner nuclear layer; 6) outer plexiform layer; 7) outer nuclear layer; 8) external limiting membrane; and 9) a layer of rods and cones.

"Retinal degeneration" refers to any hereditary or acquired degeneration of the retina and/or retinal pigment epithelium. Non-limiting examples include retinitis pigmentosa, Best's Disease, RPE pattern dystrophies, and age-related macular degeneration.

In various aspects, a method of treating an ophthalmological disorder may comprise treatment of various ocular diseases or conditions of the retina, including the following: maculopathies/retinal degeneration: macular degeneration, including age-related macular degeneration (ARMD), such as non-exudative age-related macular degeneration and exudative age-related macular degeneration; choroidal neovascularization; retinopathy, including diabetic retinopathy, acute and chronic macular neuroretinopathy, central serous chorioretinopathy; and macular edema, including cystoid macular edema, and diabetic macular edema. Uveitis/retinitis/choroiditis: acute multifocal placoid pigment epitheliopathy, Behcet's disease, birdshot retinochoroidopathy, infectious (syphilis, Lyme Disease, tuberculosis, toxoplasmosis), uveitis, including intermediate uveitis (pars planitis) and anterior uveitis, multifocal choroiditis, multiple evanescent white dot syndrome (MEWDS), ocular sarcoidosis, posterior scleritis, serpignous choroiditis, subretinal fibrosis, uveitis syndrome, and Vogt-Koyanagi-Harada syndrome. Vascular diseases/exudative diseases: retinal arterial occlusive disease, central retinal vein occlusion, disseminated intravascular coagulopathy, branch retinal vein occlusion, hypertensive fundus changes, ocular ischemic syndrome, retinal arterial microaneurysms, Coats disease, parafoveal telangiectasis, hemi-retinal vein occlusion, papillophlebitis, central retinal artery occlusion, branch retinal artery occlusion, carotid artery disease (CAD), frosted branch angitis, sickle cell retinopathy and other hemoglobinopathies, angioid streaks, familial exudative vitreoretinopathy, Eales disease, Traumatic/surgical diseases: sympathetic ophthalmia, uveitic retinal disease, retinal detachment, trauma, laser, PDT, photocoagulation, hypoperfusion during surgery, radiation retinopathy, bone marrow transplant retinopathy. Proliferative disorders: proliferative vitreal retinopathy and epiretinal membranes, proliferative diabetic retinopathy. Infectious disorders: ocular histoplasmosis, ocular toxocariasis, ocular histoplasmosis syndrome (OHS), endophthalmitis, toxoplasmosis, retinal diseases associated with HIV infection, choroidal disease associated with HIV infection, uveitic disease associated with HIV Infection, viral retinitis, acute retinal necrosis, progressive outer retinal necrosis, fungal retinal diseases, ocular syphilis, ocular tuberculosis, diffuse unilateral subacute neuroretinitis, and myiasis. Genetic disorders: retinitis pigmentosa, systemic disorders with associated retinal dystrophies, congenital stationary night blindness, cone dystrophies, Stargardt's disease and fundus flavimaculatus, Best's disease, pattern dystrophy of the retinal pigment epithelium, X-linked retinoschisis, Sorsby's fundus dystrophy, benign concentric maculopathy, Bietti's crystalline dystrophy, pseudoxanthoma elasticum. Retinal tears/holes: retinal detachment, macular hole, giant retinal tear. Tumors: retinal disease associated with tumors, congenital hypertrophy of the RPE, posterior uveal melanoma, choroidal hemangioma, choroidal osteoma, choroidal metastasis, combined hamartoma of the retina and retinal pigment epithelium, retinoblastoma, vasoproliferative tumors of the ocular fundus, retinal astrocytoma, intraocular lymphoid tumors. Miscellaneous: punctate inner choroidopathy, acute posterior multifocal placoid pigment epitheliopathy, myopic retinal degeneration, acute retinal pigment epithelitis and the like.

An anterior ocular condition is a disease, ailment or condition which affects or which involves an anterior (i.e., front of the eye) ocular region or site, such as a periocular muscle, an eyelid or an eyeball tissue or fluid which is located anterior to the posterior wall of the lens capsule or ciliary muscles. Thus, an anterior ocular condition primarily affects or involves the conjunctiva, the cornea, the anterior chamber, the iris, the posterior chamber (behind the iris but in front of the posterior wall of the lens capsule), the lens or the lens capsule and blood vessels and nerve which vascularize or innervate an anterior ocular region or site.

Thus, an anterior ocular condition can include a disease, ailment or condition, such as for example, aphakia; pseudophakia; astigmatism; blepharospasm; cataract; conjunctival diseases; conjunctivitis, including, but not limited to, atopic keratoconjunctivitis; corneal injuries, including, but not limited to, injury to the corneal stromal areas; corneal diseases; corneal ulcer; dry eye syndromes; eyelid diseases; lacrimal apparatus diseases; lacrimal duct obstruction; myopia; presbyopia; pupil disorders; refractive disorders and strabismus. Glaucoma can also be considered to be an anterior ocular condition because a clinical goal of glaucoma treatment can be to reduce a hypertension of aqueous fluid in the anterior chamber of the eye (i.e. reduce intraocular pressure).

Other diseases or disorders of the eye which may be treated in accordance with the present invention include, but are not limited to, ocular cicatricial pemphigoid (OCP), Stevens Johnson syndrome and cataracts.

A posterior ocular condition is a disease, ailment or condition which primarily affects or involves a posterior ocular region or site such as choroid or sclera (in a position posterior to a plane through the posterior wall of the lens capsule), vitreous, vitreous chamber, retina, optic nerve (i.e., the optic disc), and blood vessels and nerves which vascularize or innervate a posterior ocular region or site. Thus, a posterior ocular condition can include a disease, ailment or condition, such as for example, acute macular neuroretinopathy; Behcet's disease; choroidal neovascularization; diabetic retinopathy; uveitis; ocular histoplasmosis; infections, such as fungal or viral-caused infections; macular degeneration, such as acute macular degeneration, non-exudative age-related macular degeneration and exudative age-related macular degeneration; edema, such as macular edema, cystoid macular edema and diabetic macular edema; multifocal choroiditis; ocular trauma which affects a posterior ocular site or location; ocular tumors; retinal disorders, such as central retinal vein occlusion, diabetic retinopathy (including proliferative diabetic retinopathy), proliferative vitreoretinopathy (PVR), retinal arterial or venous occlusive disease, retinal detachment, uveitic retinal disease; sympathetic ophthalmia; Vogt-Koyanagi-Harada (VKH) syndrome; uveal diffusion; a posterior ocular condition caused by or influenced by an ocular laser treatment; posterior ocular conditions caused by or influenced by a photodynamic therapy, photocoagulation, radiation retinopathy, epiretinal membrane disorders, branch retinal vein occlusion, anterior ischemic optic neuropathy, non-retinopathy diabetic retinal dysfunction, retinitis pigmentosa, and glaucoma. Glaucoma can be considered a posterior ocular condition because the therapeutic goal is to prevent the loss of or reduce the occurrence of loss of vision due to damage to or loss of retinal ganglion cells or retinal nerve fibers (i.e., neuroprotection).

In some embodiments, the ophthalmic disorder is ocular inflammation resulting from, e.g., iritis, conjunctivitis, seasonal allergic conjunctivitis, acute and chronic endophthalmitis, anterior uveitis, uveitis associated with systemic diseases, posterior segment uveitis, chorioretinitis, pars planitis, masquerade syndromes including ocular lymphoma, pemphigoid, scleritis, keratitis, severe ocular allergy, corneal abrasion and blood-aqueous barrier disruption. In yet another embodiment, the ophthalmic disorder is post-operative ocular inflammation resulting from, for example, photorefractive keratectomy, cataract removal surgery, intraocular lens implantation, vitrectomy, corneal transplantation, forms of lamellar keratectomy (DSEK, etc), and radial keratotomy.

In various aspects, the injection for treatment of an ophthalmological disorder can be injection to the vitreous chamber of the eye. In some cases, the injection is an intravitreal injection, a subconjunctival injection, a subtenon injection, a retrobulbar injection, or a suprachoroidal injection.

In various aspects, the method for treatment of an ophthalmological disorder comprises administration of a disclosed drug delivery device containing an amount, e.g., via injection of about 0.01 mg to about 25 mg of therapeutic agent; or about 1 mg to about 15 mg of therapeutic agent. In some embodiments, the drug delivery composition may release an amount of drug that maintains a concentration within the vitreous of the eye from about 10 picomolar to about 500 picomolar over a period from about 10 days to about 12 months. The quantity of therapeutic in the drug delivery composition would be dependent on the quantity of therapeutic agent that can reside in the one or more capsules as well as the amount necessary to achieve the desired therapeutic effect.

In some embodiments, the disclosed drug delivery may protect the bioactivity of the enclosed therapeutic over a period up to 12 months. The level of protection of bioactivity will be dependent upon both the therapeutic agent used as well as the selected composition of the disclosed capsules, but may be quantified by such methods as HPLC (for determining quantity and forms of drugs present in eye), cellular assays of activity against a positive control (such as use of the therapeutic agent alone), as well as ELISA to characterize the forms of other therapeutics or to assess changes in biological activity such as transcription factor expression.

### Kits

The present disclosure also pertains to kits comprising one of: (a) the drug delivery composition as described herein; (b) the drug delivery composition as described herein in a sterile package; or (c) a pre-filled syringe or needle comprising the drug delivery composition as described herein; and instructions for administering the drug delivery composition as described herein to treat a clinical condition or pathology.

In a further aspect, the disclosed kits can be packaged in a daily dosing regimen (e.g., packaged on cards, packaged with dosing cards, packaged on blisters or blow-molded plastics, etc.). Such packaging promotes products and increases ease of use for administration by a health care profession. Such packaging can also reduce potential medical errors. The present invention also features such kits further containing instructions for use.

In a further aspect, the present disclosure also provides a pharmaceutical pack or kit comprising one or more packages comprising fthe disclosed drug delivery composition. Associated with such packages can be a notice in the form prescribed by a governmental agency regulating the manufacture, use or sale of pharmaceuticals or biological products, which notice reflects approval by the agency of manufacture, use or sale for human administration.

In various aspects, the disclosed kits can also comprise further therapeutic agents, compounds and/or products co-packaged, co-formulated, and/or co-delivered with other components. For example, a drug manufacturer, a drug reseller, a physician, a compounding shop, or a pharmacist can provide a kit comprising a disclosed drug delivery composition and another component for delivery to a patient.

It is contemplated that the disclosed kits can be used in connection with the disclosed methods of making, the disclosed methods of using or treating, and/or the disclosed compositions.

From the foregoing, it will be seen that aspects herein are well adapted to attain all the ends and objects hereinabove set forth together with other advantages which are obvious and which are inherent to the structure.

Now having described the aspects of the present disclosure, in general, the following Examples describe some additional aspects of the present disclosure. While aspects of the present disclosure are described in connection with the following examples and the corresponding text and figures, there is no intent to limit aspects of the present disclosure to this description.

### EXAMPLES

The following examples are put forth so as to provide those of ordinary skill in the art with a complete disclosure and description of how the compounds, compositions, articles, devices and/or methods claimed herein are made and evaluated, and are intended to be purely exemplary of the disclosure and are not intended to limit the scope of what the inventors regard as their disclosure. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### Materials

Chitosan (DD >75%, Mw 310,000-375,000 Da), polycaprolactone (Mn 80,000), trifluoroacetic acid (TFA), 4-(2-hydroxyethyl)piperazine-1-ethanesulfonic acid (HEPES) sodium salts and tween 20 were purchased from Sigma-Aldrich Inc. (St. Louis, MO). 1,1,1,3,3,3-hexafluoro-2-propanol (HFP) was purchased from Oakwood Products Inc. (Estill, SC). Dichloromethane (DCM), chromatographically purified bovine serum albumin (BSA) and VEGF recombinant human protein were purchased from Fisher Scientific International Inc. (Hampton, NH). Bevacizumab (Avastin) was purchased from Genentech, Inc. (San Francisco, CA). The bicinchoninic acid (BCA) protein assay kit and colorimetric 3-(4,5-dimethylthiazol-2-yl)-5-(3-carboxymethoxyphenyl)-2-(4-sulfophenyl)-2H-tetrazolium (MTS) assay, horseradish peroxidase (HRP) conjugate goat anti-human immunoglobulin G (IgG) fragment crystallizable (Fc) secondary antibody, and 3,3',5,5'-tetramethylbenzidine (TMB) were purchased from Thermo Fisher Scientific Inc. (Waltham, MA). Human retinal pigment epithelial cell line (ARPE-19 cells, CRL2302) and DMEM:F-12 medium were purchased from American Type Culture Collection (Rockville, MD). Human umbilical vein endothelial cells (HUVECs), medium 200PRF, low serum growth supplement, and lactose dehydrogenase elevating virus (LDEV)-free reduced growth factor basement membrane matrix were purchased from Thermo Fisher Scientific Inc. (Waltham, MA). All other reagents used were analytical grade.

### Capsule Fabrication

Two sizes of capsules with different inner diameters (260 µm and 1645 µm) were fabricated in this study. The 1.645 mm sized capsule mainly served as a preliminary model for the smaller capsules to optimize processing conditions. The 260 µm sized capsules were used for subsequent studies.

The capsule fabrication process is shown in FIG. 1A. The chitosan fibrous layer was prepared via electrospinning based on previous studies with modifications (see Gu, B.K., et al., Fabrication of sonicated chitosan nanofiber mat with enlarged porosity for use as hemostatic materials. 2013. 97(1): p. 65-73). Briefly, 5.0% (w/v) chitosan solution prepared in a mixture of TFA and DCM at a 7:3 volume ratio was extruded through a 20-gauge stainless steel needle that was connected to the cathode of a high-voltage DC generator. The ground was attached to a rotating drum collector at a speed of 500 rpm, where electrospun fibers were deposited. To obtain capsules with the two different inner diameters, either a 1.645 mm or 260 µm diameter 315 stainless steel rod was used for fiber collection. The solution was continuously supplied with a feeding rate of 3.0 mL/h for the 1.645 mm drum collector and 1.0 mL/h for the 260 µm drum collector at a voltage of 25.0 kV. The humidity during electrospinning was controlled at 30% using a nitrogen-filled glove box.

To prepare the PCL nanoporous layer (see Cipitria, A., et al., Design, fabrication and characterization of PCL electrospun scaffolds-a review. 2011. 21(26): p. 9419-9453; Chaparro, F.J., et al., Sintered electrospun polycaprolactone for controlled model drug delivery. 2019; Nam, J., et al., Modulation of embryonic mesenchymal progenitor cell differentiation via control over pure mechanical modulus in electrospun nanofibers. 2011. 7(4): p. 1516-1524; and Chaparro, F.J., et al., Sintered electrospun poly(ε-caprolactone)-poly(ethylene terephthalate) for drug delivery. Journal of Applied Polymer Science, 2019. 0(0): p. 47731), 0.5 g of a combination of PCL and HEPES sodium salts were dissolved in 10.0 g HFP, and the solution was continuously stirred at 40°C overnight. Five mass ratios of PCL to HEPES sodium salt (100:0; 99:1; 95:5; 92.5:7.5; 90:10) were studied to assess the impact of the salt-induced porous structure of the PCL film on drug release. The PCL solution was continuously supplied with a feeding rate of 3.0 mL/h for the 1.645 mm drum collector and 1.0 mL/h for the 260 µm drum collector using a syringe pump. The high-voltage DC generator was set to 24.0 kV to produce PCL nanofibers depositing on 1.645 mm and 260 µm diameter 315 stainless steel rods with or without the as-spun chitosan layer to form a bi-layered film and a mono-layered film, respectively.

The electrospun capsules were sintered under vacuum at 100°C for 3 hours to remove the surface porosity using an AccuTemp digital vacuum oven, and then the capsules were gently removed from the rod (see Chaparro, F.J., et al., Sintered electrospun polycaprolactone for controlled model drug delivery. 2019). The samples were washed with the saturated sodium bicarbonate solution to neutralize TFA, then deionized water to dissolve and remove HEPES sodium salts. Capsules were vacuum dried overnight. The outer diameter of the capsules prepared using the 1.645 mm rod before and after sintering was measured using a digital micrometer (Keyence). The thickness of the film was calculated as [sintered outer diameter of capsule - 1.645 mm]/2. A light microscope (Cole-Parmer) was used to acquire the images of capsules prepared using the 260 µm diameter rod. The images were analyzed by Motic Image Plus to determine the outer diameter of the capsule. The thickness of the film was calculated as [sintered outer diameter of capsule - 260 µm]/2.

### Capsule Characterization

The morphological characteristics of the capsules were examined by scanning electron microscopy (SEM) (FEI, Quanta 200). The chitosan fibrous layer, PCL fibrous layer, and the cross-section of bi-layered films and mono-layered films before and after salt leaching were attached on carbon tape placed on aluminum stub mounts and sputter-coated a layer of gold-palladium. Capsules were immersed and fractured in liquid nitrogen to acquire the cross-section for imaging. The average fiber sizes and pore sizes of the PCL layer and chitosan layer were characterized and quantified from SEM images of three samples using ImageJ (NIH).

Surface chemical analysis of the electrospun samples was performed using a Fourier-transform infrared (FTIR) spectrometer (Thermo Scientific, Nicolet Nexus 670) in the attenuated total reflectance (ATR) mode. A germanium crystal was placed in contact with samples, and 100 scans were collected at 8 cm⁻¹ resolution. Standard peak positions at 1727 cm⁻¹ and 1590 cm⁻¹ were used to identify PCL (carbonyl peak) and chitosan (amine band), respectively (see Elzein, T., et al., FTIR study of polycaprolactone chain organization at interfaces. Journal of Colloid and Interface Science, 2004. 273(2): p. 381-387; and Osman, Z. and A.K. Arof, FTIR studies of chitosan acetate based polymer electrolytes. Electrochimica Acta, 2003. 48(8): p. 993-999).

### Drug Release Profile and Loading/Encapsulation Efficacy

Hollow bi-layered capsules with two open ends were obtained by removing the drum collectors. For the 1.645 mm inner diameter capsule, 2.0 mg BSA powders (model protein) or 2.0 mg lyophilized bevacizumab powders (Avastin, anti-VEGF) dissolved in phosphate buffered saline (PBS) at a concentration of 0.1 mg/µL was loaded to the capsule which was sealed at the ends using a tube sealer (Doug Care Equipment, TTS-8C) (see Chaparro, F.J., et al., Sintered electrospun polycaprolactone for controlled model drug delivery. 2019; and Bernards, D.A., et al., Nanostructured thin film polymer devices for constant-rate protein delivery. 2012. 12(10): p. 5355-5361). For the 260 µm inner diameter capsules, concentrated 1.0 mg BSA or 1.0 mg bevacizumab slurry at a concentration of 1.0 mg/µL was loaded into the capsules using a 31-gauge needle considering the limited volume inside the capsules.

*In vitro* BSA release profiles from PCL mono-layered capsules (not according to the invention) and PCL-chitosan bi-layered capsules were acquired as described in the following steps. Capsules were immersed in 1 mL PBS in a 1.5 mL low binding centrifuge tube to reduce the centrifuge tube binding to the eluted protein. The centrifuge tube with an immersed capsule was incubated at 37°C to simulate physiological conditions. At 1 h, 3 h, 6 h, 12 h, 24 h, 3 days, 1 week, 2 weeks, 1 month, and monthly thereafter, the eluant was collected (see Sousa, F., et al., A new paradigm for antiangiogenic therapy through controlled release of bevacizumab from PLGA nanoparticles. 2017. 7(1): p. 3736; Yandrapu, S.K., et al., Nanoparticles in Porous Microparticles Prepared by Supercritical Infusion and Pressure Quench Technology for Sustained Delivery of Bevacizumab. Molecular Pharmaceutics, 2013. 10(12): p. 4676-4686; and Tyagi, P., et al., Light-activated, in situ forming gelfor sustained suprachoroidal delivery of bevacizumab. Molecular pharmaceutics, 2013. 10(8): p. 2858-2867). Then, fresh 1.0 mL PBS was added and kept under incubation. The BSA release profile was acquired by determining the absorption of eluted BSA by BCA assay and quantifying the concentration using a BSA protein-based standard curve. For *in vitro* bevacizumab release from PCL mono-layered capsules and PCL-chitosan bi-layered capsules, the same protocol was applied to acquiring the bevacizumab eluant. The characteristic absorbance of bevacizumab was identified at 277 nm by UV-Vis spectroscopy (Agilent, Cary 100 UV-Vis), and the release rate of bevacizumab from capsules was determined by micro-plate reader (BioTek, Synergy HT) at 277 nm and quantified based on the standard curve of the stock bevacizumab solution at different concentrations (see Li, F., et al., Controlled release of bevacizumab through nanospheres for extended treatment of age-related macular degeneration. 2012. 6: p. 54). The experiments were done in triplicate.

To determine the release rate of reactive bevacizumab from the 260 µm inner diameter capsules, the enzyme-linked immunosorbent assay (ELISA) was conducted as reported (see Tyagi, P., et al., Light-activated, in situ forming gel for sustained suprachoroidal delivery of bevacizumab. Molecular pharmaceutics, 2013. 10(8): p. 2858-2867; and Varshochian, R., et al., Albuminated PLGA nanoparticles containing bevacizumab intended for ocular neovascularization treatment. Journal of Biomedical Materials Research Part A, 2015. 103(10): p. 3148-3156). Briefly, 100 µL of 1 µg/mL VEGF recombinant human protein in pH 9.6 sodium carbonate buffer solution was immobilized on the 96-well Nunc maxisorp plate (Thermo Fisher Scientific) at 4°C overnight. The plate was blocked by 200 µL 2% BSA solution in PBS/T (0.05% v/v tween 20 in pH 7.4 PBS) for 2h at room temperature and washed with 300 µL PBS/T three times. Then, the eluted bevacizumab from the capsules was diluted between 0 ng/mL to 10 ng/mL (determined by the standard curve) in 0.1% BSA-PBS/T solution, and 100 µL sample was added to each well and incubated at room temperature for another 2 hours. Later, the plate was washed with PBS/T three times, and 100 µL HRP goat anti-human IgG Fc secondary antibody PBS/T solution (1:1000) was added to each well. The whole plate was incubated in the dark at room temperature for 1 hour and washed with PBS/T five times. The color was shown by adding 100 µL TMB and stopped by 100 µL 1N sulfuric acid. The concentration of active bevacizumab in each test sample was determined by comparing the absorbance at 450 nm with the standard curve.

The drug payload was determined by breaking three BSA and bevacizumab loaded mono-layered and bi-layered capsules of different sizes in PBS. Briefly, three BSA and bevacizumab loaded mono-layered and bi-layered capsules were broken and immersed in 1 mL PBS solution. The device was vigorously washed by 1 mL PBS five times using a vortex mixer. Each washing took at least ten minutes. The collected eluents of BSA and reactive bevacizumab were determined by BCA assay, UV-Vis spectroscopy, and ELISA. The drug encapsulation efficiency was calculated as free drug in the eluent / total amount of drug *100%. The drug loading efficiency was calculated as drug payload/ capsule weight *100%. The cumulative release % was calculated as the cumulative amount of eluted drug from the capsule / [drug payload * encapsulation efficiency] *100%.

### Capsule Biodegradation

The *in vitro* degradation and erosion of 260 µm inner diameter PCL mono-layered capsule and the PCL-chitosan bi-layered capsule over a long-term period were determined by morphology changes. Briefly, the capsules with two sealed ends and bi-layered capsules with two open ends incubated in PBS at physiological temperature over 9 months and three weeks, respectively, were retrieved and then vacuum dried for characterization. The PCL outer-layer, chitosan inner-layer, and cross-section of both mono-layered and bi-layered capsules were examined using SEM. The large break and tear were assessed, and the average pore sizes of the PCL layer prepared with different ratios of HEPES sodium salt were quantified by analyzing three different images using Image J and compared with the initial pore size of capsules before incubation by one-way ANOVA with post-hoc Tukey test at a significance level of 0.05. Data are presented as mean ± standard deviation.

### Cytotoxicity

*In vitro* cytotoxicity of the PCL mono-layered capsule and the PCL-chitosan bi-layered capsule were assessed by MTS assay conducted with human retinal pigmented epithelial (ARPE-19) cells (see Sur, A., et al., Pharmacological protection of retinal pigmented epithelial cells by sulindac involves PPAR-α. 2014. 111(47): p. 16754-16759; Andrés-Guerrero, V., et al., Novel biodegradable polyesteramide microspheres for controlled drug delivery in ophthalmology. 2015. 211: p. 105-117; and Huhtala, A., et al., In vitro biocompatibility of degradable biopolymers in cell line cultures from various ocular tissues: extraction studies. Journal of Materials Science: Materials in Medicine, 2008. 19(2): p. 645-649). ARPE-19 cells were seeded in 48-well plates at a density of 4×10⁴ cells/well for all experiments. The cytotoxicity assay was performed by both the direct contact method and extract exposure method. For the direct contact method, 1 cm PCL mono-layered capsule or PCL-chitosan bi-layered capsule were placed in the cell-seeded well plate for 24 hours. For the extract exposure method, the PCL mono-layered capsule or PCL-chitosan bi-layered capsule were immersed in 1 mL fresh media for 1 day, 3 days, 1 week, 2 weeks, and 1 month. At each time point, the capsule-conditioned media was transferred to the ARPE-19 cell culture, and measurements were performed with incubation times of each sample with the cells for 24 hours. To perform the cytotoxicity assay, the cell culture media were mixed with 20 µL MTS reagent followed by 3h incubation at 37 °C. The absorbance measurements of the supernatants were obtained using a microplate reader at 490 nm. Cell viabilities of the experimental group were normalized to the control group (no treatment). All experiments were repeated in triplicate, and data were analyzed by one-way ANOVA with post-hoc Tukey test at a significance level of 0.05. Data are presented as mean ± standard deviation.

### Aggregation and Anti-angiogenic Activity Assessment

The stability of bevacizumab was determined by ultra-high-performance liquid chromatography (UHPLC) 3000 system (Thermo Fisher Scientific Inc., Waltham, MA) using a SEC-1000 column. To determine the bevacizumab stability during the lyophilization process, 500 µL 25 mg/mL bevacizumab (Avastin) was freeze-dried by lyophilizer (Labconco), and the powders were re-diluted in 500 µL PBS. The instability of concentrated bevacizumab was also assessed by diluting the bevacizumab slurry from the device in PBS to 25 mg/mL. The free native bevacizumab before and after lyophilization, concentrated bevacizumab, and eluted bevacizumab from mono-layered and bi-layered capsules at specific time points were filtered through 0.2 µm Whatman SPARTAN HPLC Syringe Filter (VWR International, Radnor, PA) before injection. The fractions native bevacizumab monomer, aggregate, and fragment were analyzed by HPLC spectral deconvolution into separative individual elution peaks. The integral areas of monomer, aggregate, and fragment were normalized to the total area of the HPLC peak to obtain the percentage of each component. The average molecular weight was then calculated from the fraction % and molecular weight of each component.

The anti-angiogenic activity of released bevacizumab from the PCL mono-layered capsule and PCL-chitosan bi-layered capsules were further assessed using a capillary-like tubule formation assay (see Elsaid, N., et al., PLGA microparticles entrapping chitosan-based nanoparticles for the ocular delivery of ranibizumab. 2016. 13(9): p. 2923-2940; Arnaoutova, I. and H.K.J.N.p. Kleinman, In vitro angiogenesis: endothelial cell tube formation on gelled basement membrane extract. 2010. 5(4): p. 628; and DeCicco-Skinner, K.L., et al., Endothelial cell tube formation assay for the in vitro study of angiogenesis. 2014(91)). More specifically, HUVECs were exposed to VEGF (5 ng/mL), angiogenesis promoter, mixed with i) 10 µg/mL native bevacizumab; ii) 10 µg/mL bevacizumab released from PCL mono-layered capsule; and iii) 10 µg/mL bevacizumab released from PCL-chitosan bi-layered capsule at 1 week, 2 weeks, 1 month, 3 months, 6 months, and 9 months. After 6 hours, calcein AM was added to the cells followed by incubation for 30 min. Cells were then visualized directly using a fluorescent microscope (Nikon, Eclipse TS100) equipped with a digital camera (Qimaging). Three images were analyzed using Image J to quantify the lengths of formed capillary structure. The total tubular lengths from the experimental groups were normalized to the VEGF-treated control group for each sample and all experiments were repeated three times. Data were analyzed by one-way ANOVA with post-hoc Tukey test with a significance level of 0.05. Data are presented as mean ± standard deviation.

### Injection Feasibility

Fresh porcine eyes obtained from a local abattoir (Delaware Meats, Delaware, Ohio) were used for assessment of device injection feasibility (see Hoshi, S., et al., In Vivo and In Vitro Feasibility Studies of Intraocular Use of Polyethylene Glycol-Based Synthetic Sealant to Close Retinal Breaks in Porcine and Rabbit Eyes. 2015. 56(8): p. 4705-4711). The capsule was preloaded into a 21-gauge hypodermic needle, which was connected to a 1 mL syringe. The 21-gauge needle used in this study has a similar inner diameter to the commercialized intraocular implant injector, Ozurdex applicator (see Lee, S.S., et al., Biodegradable implants for sustained drug release in the eye. 2010. 27(10): p. 2043-2053). The intraocular injection was placed 3 mm posterior to the limbus using the syringe needle. A small volume of PBS (100 µL) used to push the capsule into the porcine vitreous humor and reduce the effect of IOP elevation. After injection, the needle was removed, and the sclera was cut around the middle of the eye to check the placement of the capsule in the vitreous humor.

### Electrospinning Chitosan and PCL Nanofibers as Building Blocks for IBB Capsules

The disclosed strategy of fabricating the IBB capsules is based on two-step coating of films of chitosan and PCL on a rod-shaped template followed by removal from the template. To create the porous central hollowed bi-layered structure, electrospinning was used, which can offer a high surface area to volume ratio for protein chemoadsorption and tunable porosity for drug diffusion to obtain the desired function. Electrospinning as a method for nanofiber fabrication is based on using electric force to draw charged polymer solution to nanosized fibers. To synthesize the chitosan nanofibers, processing parameters were optimized, including humidity and voltage. For example, either high humidity (above 30%) or low voltage (below 24 kV) caused a significant loss of charge from the spinning head and prevented threads of chitosan solution from forming fibers. Therefore, low humidity and relatively high voltage were used. Meanwhile, the chitosan precursor was dissolved in TFA and DCM, as the addition of TFA better dissolved chitosan while the DCM allowed timely evaporation of the solvent, both of which are required for electrospinning. To place the chitosan nanofiber onto the steel rod templates, the chitosan nanofibers were collected on a steel rod under rotation directly. From the SEM images shown in FIG. 2, the diameter of chitosan fibers was 331.61 ± 186.19 nm, and these fibers were highly interconnected, forming a highly porous structure to allow efficient drug diffusion. However, the chitosan fibrous mat was found to be fragile, which is consistent with the reports on its low mechanical flexibility (see Jayakumar, R., et al., Biomedical applications of chitin and chitosan based nanomaterials-A short review. 2010. 82(2): p. 227-232). To this end, a second layer of PCL was added, which not only provided physical entrapment of drugs, but also imparted improved flexibility. More specifically, on top of the chitosan nanofibers, PCL nanofibers with a diameter of 932.57 ± 399.42 nm were coated (see Baker, S.R., et al., Determining the mechanical properties of electrospun poly-ε-caprolactone (PCL) nanofibers using AFM and a novel fiber anchoring technique. 2016. 59: p. 203-212). To this end, nanofiber-based cylinders that have a high surface area, high mechanical flexibility, and strong adhesion between different layers were constructed as building blocks for IBB capsules.

### Synthesis and Characterization of the Injectable and Bi-Layers Microcapsules

Utilizing the two nanofiber layers as building blocks, the hollow capsule structure was formed by directly removing the steel rod template after electrospinning as shown in FIG. 3. While the bi-layered PCL-chitosan nanofibrous structure could provide significant physical and electrostatic interactions with the protein therapeutics, burst release could still take place given the significantly larger sizes of the continuous porous structures of nanofibers compared to the size of proteins (see Chaparro, F.J., et al., Sintered electrospun polycaprolactone for controlled model drug delivery. 2019). Sintering was used to melt the PCL nanofiber layer to reduce its porosity and reduce the burst release of the drug. On the other hand, directly coating the PCL layer without starting with the initial nanofibrous structure made it difficult to achieve the thin layered structure on top of chitosan, which was critical for making small capsules that could be injected. The mechanism of sintering-based formation of the bi-layered structure was based on the relatively low melting point of PCL at 60°C compared to the chitosan nanofibers at 220°C. Therefore, while PCL became mostly non-porous during the process to physically hold the drug, the chitosan remained porous for binding the drug electrostatically. In addition, this process also better integrated the two layers during the melting process. From the SEM images shown in FIG. 3, the chitosan fibrous layer adhered to the PCL outer layer, which stabilized the bi-layered structure, as melting the PCL nanofibers increases the adhesion between the two layers. However, the framework composed of large fibers can still be observed on the surface of the PCL after sintering. During the sintering process, the thickness of the film decreased by 80% due to compression and an increase in density, so the capsule size can be controlled by modulating the thickness of the chitosan and PCL fibrous layers during the electrospinning process.

After sintering the chitosan and PCL fibrous film, the bi-layered microcapsules with hollow structures were generated by a templating strategy and by taking advantage of the mechanical robustness of the PCL outer layer. By controlling the shape and size of the template rods, the sizes and structures of capsules could be effectively controlled. As a proof-of-concept, two sizes of the mono-layered PCL and bi-layered chitosan-PCL capsules were prepared: one with a larger inner diameter of 1.645 mm (pre-model) which could be transplanted as a scaffold and one with a smaller inner diameter of 260 µm (final model) which is injectable through a 21-gauge needle. While the steel-rod-templated hollow structure mainly allowed a high volume for drug loading, the bi-layered membrane provided physical trapping and chemical non-covalent bonding to achieve sustainable release for a long time.

In the devices used for the drug release studies, the outer diameter of the 1.645 mm inner diameter capsule was approximately 1.815 mm with a wall thickness of 89.36 ± 11.52 µm. Similarly, the outer diameter of the 260 µm inner diameter capsule was approximately 430 µm with 89.85 ± 4.27 µm membrane thickness, which was designed to be injectable via a 21-gauge needle. The increased thickness of the capsules enhanced the mechanical properties of the capsule, which prevented fracture during the injection. However, increasing the size of the capsule could potentially impede intravitreal injection. Therefore, 80-90 µm was determined as the wall thickness that balanced mechanical robustness as well as injection feasibility. Also, the membrane thickness is closely related to the diffusion rate of drug, so the thickness difference between the mono-layered and bi-layered capsules was controlled and minimized to reduce the impact of thickness on drug release.

### Modulating the Nanoporous Structure of the Bi-Layers Membrane

After sintering, PCL layers became non-porous and the drug release rate was significantly limited (see Chaparro, F.J., et al., Sintered electrospun polycaprolactone for controlled model drug delivery. 2019). However, without sintering, the bi-layered capsule would be highly porous resulting in an undesirable high drug release rate. As such, a salt leaching method was employed to precisely modulate the 3D porous structure of the bi-layered membrane to enable the long-term sustainable release of drugs. More specifically, varying amounts of water-soluble salts (HEPES) were mixed into the nanofibers during electrospinning. Incubation of capsules in water prior to drug loading led to the dissolution of HEPES inside the films, thereby forming a porous structure again on the bi-layered membrane. By modulating the concentrations of HEPES in the PCL nanofibers, the porosities could be effectively controlled. As shown in FIG. 4, the pore size and distribution in the PCL sintered films were highly dependent on the mass ratio of salt to PCL. For example, the pores tended to be smaller and more dispersed throughout the surface of films at lower salt concentrations. However, low amounts of salt also hindered the generation of interconnecting pores for diffusion and release of large molecules. Table 1 shows the analytical pore size results for different ratios of PCL to HEPES sodium salt. To this end, PCL films prepared with salt concentrations above 5.0% were used in capsule manufacturing and drug release studies because of the interconnected porous structures observed in their cross-sections via SEM.

**TABLE 1. Porosity and pore size of PCL membranes prepared with different ratios of PCL to HEPES sodium salt.**

| Sample name | Pore diameter (nm) | Porous channel |
|---|---|---|
| 0.0 % HEPES salt | None | No |
| 1.0 % HEPES salt | 237.26 ± 96.93 | No |
| 5.0 % HEPES salt | 371.65 ± 156.77 | Yes |
| 7.5 % HEPES salt | 582.21 ± 302.17 | Yes |
| 10 % HEPES salt | 608.55 ± 273.90 | Yes |

To assess the changes in the bi-layered structure before and after salt leaching, SEM imaging was used to observe the inner surface, the outer surface, and the cross-section of the bi-layered capsules. Before salt leaching, the PCL sintered film was rough with some HEPES sodium salt crystals embedded in it. After salt leaching, the porous structure appeared in the PCL layer, and the chitosan layer lost its fibrous structure and formed a porous layer. The average pore sizes of the chitosan layer were 802.47 ± 501.02 nm, which allowed for 3D diffusion of and interactions with protein for sustainable release by maximizing its interactions with the protein through electrostatic interactions. The inner chitosan layer showed a more nanoporous structure with a thickness of 25 µm, which could be due to the relatively high melting point of chitosan. In contrast, the outer PCL layer had a more compact structure with nanochannels passing through and a total thickness of 65 µm to support protein diffusion while physically trapping the drugs. These results are consistent with morphologies collected on the individual layers of PCL and chitosan.

To further confirm the chemical property of the bi-layered capsules after sintering and washing, FTIR spectroscopy was performed on the final capsule, shown in FIG. 5. In the spectrum shown, a significant peak at 1727 cm⁻¹ was assigned to the carbonyl group in PCL. Peaks at 2963 cm⁻¹ and 2995 cm⁻¹ were C-H stretches in the backbone of PCL. A broad group could be observed at 3478 cm⁻¹ which was attributed to the O-H stretching vibrations from the hydroxyl groups which are abundant in the backbone of chitosan. Moreover, a characteristic peak for chitosan at 1571 cm⁻¹ was assigned to N-H stretching. Such peaks provide strong evidence of the chemical property of the chitosan layer and PCL layer even after exposure to sintering. Therefore, the cationic chitosan remains active and is capable of chemically non-covalent binding the anionic protein, bevacizumab. With SEM and FTIR, the feasibility of the disclosed bottom-up approach for synthesizing hybrid nano-microstructured capsules that have widely tunable pore sizes, aspect ratios, dimensions and can provide optimal physical and chemical properties for drug loading and controlled release of protein therapeutics was proved.

### High Payloads of Bevacizumab and Long-Term Sustainable Drug Release

To confirm the high payloads of protein drugs enabled by the hollow structure of the capsules, drug encapsulation efficacy was determined by breaking the drug-loaded capsules and assessing the amount of BSA and bevacizumab leaking from the capsules, respectively. BSA was used as a model protein drug, and bevacizumab is a clinically used anti-VEGF therapeutic for treating AMD. Considering the BSA and bevacizumab may adsorb to the chitosan layer of bi-layered capsule, both mono-layered capsule and bi-layered capsule were used to assess the drug payload. No significant difference in drug payload was found between both capsules. Based on the study, BSA encapsulation efficacy of the three large capsules and three small capsules were 100.39 ± 6.46% and 69.64 ± 7.15%, respectively. Lower encapsulation efficacy was observed in loading bevacizumab in both large and small capsules, which was 52.66 ± 6.47% assessed by UV-Vis spectroscopy, a commonly used instrument to determine the concentration of protein having characteristic absorption around 280 nm, shown in FIG. 12. However, a higher amount of reactive bevacizumab, 729.02 ± 84.67 µg, was quantified by ELISA, which gave approximately 70% bevacizumab encapsulation efficacy. The lower encapsulation efficiency could be attributed to the decreased sensitivity of UV-Vis spectroscopy to the bevacizumab at a lower concentration and cumulative release which can be effectively detected by ELISA. The loading capacity of the capsule is approximately 26.60 ± 1.90 % w/w, which is higher than most reported devices with a loading capacity of 10-15% (see Li, F., et al., Controlled release of bevacizumab through nanospheres for extended treatment of age-related macular degeneration. 2012. 6: p. 54; and Badiee, P., et al., Ocular implant containing bevacizumab-loaded chitosan nanoparticles intended for choroidal neovascularization treatment. Journal of Biomedical Materials Research Part A, 2018. 106(8): p. 2261-2271).

Using the model drug BSA the ability of the exemplary capsule for modulating the drug release profile by altering the surface morphology and porosity of the capsules was showcased, as shown in FIG. 6. As a proof-of-concept, both single-layered and bi-layered capsules with inner diameters of 1.645 mm and 260 µm were prepared, and samples prepared with salt leaching conditions of 5%, 7.5%, and 10% salt were also investigated. FIG. 6 shows the BSA release profile of mono-layered and bi-layered capsules for both sizes. As expected, a burst release occurred in the 1.645 mm inner diameter PCL mono-layered capsules in the first month, and more than 75% of the loaded BSA was eluted from the capsules, which significantly limited the duration of drug release. Due to the increased ratio of surface area to volume, the period of burst release was shortened to two weeks in the 260 µm inner diameter capsules, and the cumulative release percentage is similar to the one of 1.645 mm inner diameter capsule during this period. As such, the drug diffusion rate of the small capsule was promoted. However, a steady release was followed by burst release in both capsule sizes. The maximum drug release period of the mono-layered capsule was approximately five months for 1.645 mm inner diameter capsule and three months for the 260 µm inner diameter capsules prepared with 10% salt. The effect of HEPES sodium salt was also investigated. A higher salt concentration resulted in a faster release rate due to the increased interconnectivity of pores. The burst release was slowed down but still uncontrollable in the capsules with lower salt concentration.

In contrast, the PCL-chitosan bi-layered capsules did not show obvious evidence of burst release. The bi-layered capsules significantly slowed BSA release. The release profiles of the bi-layered capsule showed high linearity, which are summarized in FIG. 7. After one month, the 1.645 mm inner diameter bi-layered capsule showed a higher ability to retain the BSA inside the device, with approximately 15% of the loaded BSA was released, which was 60% less release in the same period as compared to the mono-layered PCL capsule. Similarly, 260 µm inner diameter bi-layered capsules significantly reduced the burst release. Only 25% of the BSA was eluted from the 260 µm inner diameter bi-layered capsules, which was higher than the 1.645 mm capsules due to the relatively greater area to volume for diffusion. The chitosan layer was effective in limiting drug diffusion, and the porosity of the PCL shell did not play a vital role in controlling BSA release. There was no significant difference between the diameters of the mono-layered and bi-layered capsules (p>0.05), so the effect of thickness on drug release was negligible in these studies. Theoretically, the bi-layered structure has the potential to control drug release over at least one year for the capsules in both sizes based on the cumulative release data.

After the porous structures of bi-layered capsules was comprehensively investigated and optimized, the capsules with a bi-layered structure were used and leached with 10% HEPES for loading and release of bevacizumab, the target drug for clinical AMD treatment. Consistent with the BSA drug release experiments, a sustainable release profile over one year and nine months without obvious initial burst release was successfully achieved in both the 1.645 mm capsules and 260 µm capsules, respectively. Interestingly, there was a further reduction of burst release of bevacizumab compared to BSA during the bi-layered capsule-based drug release. This could be due to the increased molecular weight and lower effective charge of bevacizumab as compared to BSA. At this condition, the pore size dominates the diffusion rate of bevacizumab. The bevacizumab with higher molecular weight may be difficult to be eluted from the capsule with the limited porous channels. This explains why the total release of both mono-layered capsules and bi-layered capsules prepared with 5% HEPES salt have similar release kinetics. Also, the capsules prepared by 5% HEPES salt have the lowest release rate as compared to the other two capsules with larger pores inside the membrane. It is noticeable that the nearly zero-order release kinetics was achieved with the 260 µm inner diameter bi-layered capsule loaded with bevacizumab after the burst release, shown in FIG. 7 (p<0.05).

Simple UV absorption was used to assess and quantify the amount of bevacizumab, but it could not specify the reactive bevacizumab and differentiate the background of broken polymers from the capsules over time. Therefore, eluted bevacizumab from 260 µm capsules was re-determined by ELISA. FIG. 13 shows that the general trend of bevacizumab releasing was consistent with the previous results determined by the UV-Vis. For instance, the long-term cumulative release of bevacizumab from the mono-layered capsules made by 5 % HEPES salt was approximately 160 µg assessed by UV-Vis which was the same as that characterized by ELISA over nine months. Therefore, by comparing the release result from UV-Vis and ELISA, the release profile acquired by the UV-Vis was reliable, which could provide a general trend of bevacizumab release from both mono-layered and bi-layered capsules. Similarly, the capsules prepared with 5% salt had a relatively slower release rate as compared to the ones with 7.5% and 10% salt over nine months. Based on these considerations, the bi-layered capsules with higher (7.5% and 10%) HEPES salt concentrations were then identified and used for the long-term release of anti-VEGF. Meanwhile, the high drug loading capacity and stable drug release profiles over periods of nine months strongly indicate the potential of the exemplary bi-layered capsules as a versatile platform for delivering anti-VEGF therapeutics.

### Capsule Biodegradation

The *in vitro* degradation of PCL mono-layered capsule and chitosan-PCL bi-layered capsule were studied. By placing the capsules with two closed ends in PBS at 37°C over nine months, the capsules were retrieved and characterized by SEM. The mechanical integrity of the whole device is mainly determined by the PCL layer which slowly undergoes hydrolysis at ester linkages (see Darwis, D., et al., Enzymatic degradation of radiation crosslinked poly (ε-caprolactone). Polymer Degradation and Stability, 1998. 62(2): p. 259-265). Therefore, it is critical to assess the erosion and degradation of the PCL layer over time. From the characteristic SEM images shown in FIG. 11, both the mono-layered capsule and bi-layered capsule were remaining intact after nine-month incubation. However, the pores on the surface of the PCL membrane became larger and more dispersed after nine months as compared to the initial capsule, which indicated the slow degradation of the PCL membrane. Table 2 summarizes the analytical pore size of capsules at different conditions of HEPES salt. The pores on the PCL surface significantly increased by approximately 180 nm in diameter on average (p ≤ 0.05), but the whole device kept integrity without any obvious cracks and breaks. The bi-layered capsule was also characterized. After nine months, the chitosan layer was still tightly adhered on the PCL layer, and fibers were still well-defined and intact. The fibrous framework of the chitosan surface layer was still obvious without any significant changes. Moreover, the membrane thickness of both the mono-layered capsule and bi-layered capsule was in the range of 80 µm to 90 µm, which is similar to its original thickness before the incubation. However, the significant thickness decrease and loss of chitosan fibers were observed when immersing the capsules with two opened ends in PBS at the physiological temperature over three weeks, as shown in FIG. 15. This probably is caused by the slow degradation of chitosan when directly exposing to water in the long term (see Kean, T. and M. Thanou, Biodegradation, biodistribution and toxicity of chitosan. Advanced Drug Delivery Reviews, 2010. 62(1): p. 3-11; and Onishi, H. and Y. Machida, Biodegradation and distribution of water-soluble chitosan in mice. Biomaterials, 1999. 20(2): p. 175-182). Moreover, due to chitosan's weak mechanical properties, the inner chitosan fibrous membrane can become fragile under shear forces during long-term incubation in water and result in a significant loss of chitosan (see Sangsanoh, P. and P.J.B. Supaphol, Stability improvement of electrospun chitosan nanofibrous membranes in neutral or weak basic aqueous solutions. 2006. 7(10): p. 2710-2714; and Chen, Z., et al., Mechanical properties of electrospun collagen-chitosan complex single fibers and membrane. Materials Science and Engineering: C, 2009. 29(8): p. 2428-2435). Therefore, the hydrophobic PCL layer is able to protect the inner chitosan layer from breaking down and further reduce the deterioration of the whole device.

**TABLE 2. Porosity and pore size of PCL membranes prepared with different ratios of PCL to HEPES sodium salt after one-year incubation. A significant increase of pore size of PCL at different salt concentrations was found over nine-month incubation (p ≤ 0.05).**

| Sample name | Pore diameter (nm) | Increase (nm) |
|---|---|---|
| 5.0 % HEPES salt | 550.83 ± 243.33 | 179.81 ± 16.71 |
| 7.5 % HEPES salt | 662.00 ± 238.43 | 79.79 ± 22.26 |
| 10 % HEPES salt | 935.17 ± 331.41 | 326.62 ± 24.82 |

### Cytotoxicity

One of the most critical properties for any drug delivery device is acceptable biocompatibility in the presence of the target cells or tissues both in the short term and long term. To this end, the cytotoxicity of the exemplary bi-layered capsules was investigated using retinal pigment epithelial (ARPE-19) cells, as they are among the most prevalent cells in the retina and are highly sensitive to toxic and exogenous materials by both direct contact and extract exposure methods. By using a standard mitochondria activity measurement assay, the cell viability of RPE cells with and without treatment of both monolayered and bi-layered capsules was measured. From the results shown in FIG. 8, both the PCL mono-layered capsule and PCL-chitosan bi-layered capsule showed negligible toxicity to the RPE cells during 24-hour direct incubation. Both PCL and chitosan were reported for their good biocompatibility in intraocular applications (see Kim, J., et al., Long-term intraocular pressure reduction with intracameral polycaprolactone glaucoma devices that deliver a novel anti-glaucoma agent. 2018. 269: p. 45-51; and Wassmer, S., et al., Chitosan microparticles for delivery of proteins to the retina. Acta Biomaterialia, 2013. 9(8): p. 7855-7864). Similarly, the extracts of both capsules also did not influence the cell viability over a month shown in FIG. 8. Over 95% viability for the bi-layered capsules even at time points as long as one month was also found. These results collectively indicate negligible cytotoxicity to the retinal pigmented epithelial cells and suggest the potential of the bi-layered capsules for preclinical evaluation in ophthalmic models in future studies.

### Stability of Released VEGF-Inhibitors and Therapeutic Effects on Anti-Angiogenesis

One main issue preventing the development of long term sustained protein delivery systems is the aggregation and degradation of proteins in aqueous environments. Bevacizumab is unstable under physiological conditions and is prone to degradation and aggregation in the body over time (see Courtois, F., et al., Rational design of therapeutic mAbs against aggregation through protein engineering and incorporation of glycosylation motifs applied to bevacizumab. mAbs, 2016. 8(1): p. 99-112; Oliva, A., M. Llabrés, and J.B. Fariña, Capability measurement of size-exclusion chromatography with a light-scattering detection method in a stability study of bevacizumab using the process capability indices. Journal of Chromatography A, 2014. 1353: p. 89-98; Latypov, R.F., et al., Elucidation of acid-induced unfolding and aggregation of human immunoglobulin IgG1 and IgG2 Fc. Journal of Biological Chemistry, 2012. 287(2): p. 1381-1396; and Bakri, S.J., et al., Six-month stability of bevacizumab (Avastin) binding to vascular endothelial growth factor after withdrawal into a syringe and refrigeration or freezing. Retina, 2006. 26(5): p. 519-522). Moreover, the device is loaded with high concentrated bevacizumab acquired from freeze-drying. The aggregation and loss of activity may happen at high concentrations or during the lyophilization process (see Varshochian, R., et al., The protective effect of albumin on bevacizumab activity and stability in PLGA nanoparticles intended for retinal and choroidal neovascularization treatments. European Journal of Pharmaceutical Sciences, 2013. 50(3): p. 341-352). It becomes critical as the bevacizumab aggregates may not be released from the implant at the same rate as the monomer. Therefore, the bevacizumab stability study was required to assess the aggregation and fragmentation of bevacizumab during the device fabrication and device incubation over time using HPLC. The analytical aggregation and fragmentation of bevacizumab are summarized in Table 3, and the HPLC spectrum is shown in FIG. 14. To confirm the stability of bevacizumab during the lyophilization, the HPLC spectrum of lyophilized bevacizumab was compared with that of the commercial bevacizumab, Avastin. 16% aggregates formed in the free native bevacizumab and a slight increase of bevacizumab aggregates were observed during the lyophilization cycle. Also, the aggregation in concentrated solutions was assessed by diluting in PBS immediately followed by HPLC characterization. However, no changes of bevacizumab aggregates were observed, which indicated that the proteins are quite stable in concentrated solutions and further proved that the exemplary device can slow down the release of bevacizumab monomer. The potency of the bevacizumab released from the mono-layered capsule and bi-layered capsule in the long-term was also assessed. The percentage of aggregates ranged between 11% to 16% for both capsules over three months, and it was also found that the bevacizumab underwent fragmentation during long-term incubation. However, the potency of bevacizumab was still maintained at a high level over this period, shown in Table 3. The monomer of bevacizumab eluted from 260 µm inner diameter PCL mono-layered capsule took up 84% at one month, and this number slightly decreased to 79% at three months. Similarly, the released bevacizumab monomer from 260 µm inner diameter chitosan-PCL bi-layered capsule was 82% over the first three months. This enhanced stability could be due to the adhesion to chitosan by ionically binding to glycoprotein and increasing its bioavailability. Also, the hydrophobic PCL layer slowed down the process of fragmentation by reducing the fluid exchange across the capsule. As such, the potency of the bevacizumab released in the long-term is well preserved, suggesting the good potential of the exemplary capsules for the treatment of AMD without frequent injections.

**TABLE 3. Analytical aggregation and fragmentation of free native bevacizumab before and after lyophilization and eluted bevacizumab from both mono-layered capsule and bi-layered capsule at 1 month and 3 months.**

| **Sample name** | **Light Chain** | **Heavy Chain** | **Heavy +Light** | **Avastin -Heavv** | **Avastin - Light** | **Avastin** | **Aggregate** | **MW (kDa)** |
|---|---|---|---|---|---|---|---|---|
| **Native bevacizumab** | 0% | 0% | 0% | 0% | 0% | 84% | 16% | 161 |
| **Lyophilized bevacizumab** | 0% | 0% | 0% | 0% | 0% | 81% | 19% | 162 |
| **Bevacizumab in the device** | 0% | 0% | 0% | 0% | 0% | 81% | 19% | 165 |
| **PCL_1 month** | 0% | 0% | 4% | 0% | 0% | 84% | 11% | 155 |
| **PCL_3 month** | 0% | 0% | 5% | 0% | 0% | 79% | 16% | 157 |
| **Ch-PCL_1 month** | 0% | 3% | 2% | 3% | 0% | 82% | 11% | 154 |
| **Ch-PCL_3 month** | 0% | 0% | 6% | 0% | 0% | 83% | 11% | 154 |

Whereas HPLC provides clear information on the bevacizumab monomer and aggregation delivered by the exemplary capsules, ELISA characterizes the potency and amount of the reactive bevacizumab to VEGF released in the long-term to ensure its effects on angiogenesis. Therefore, bevacizumab ELISA was conducted to determine the reactive bevacizumab released from the 260 µm inner diameter capsule over time. After one month, the release rate of active bevacizumab was maintained at around 20 µg/mL per month, which is similar to the amount of bevacizumab determined by UV-Vis. Moreover, the bioactivity percentage of eluted bevacizumab was also calculated from comparing the cumulative release percentage measured by ELISA to that determined by UV/Vis. From the result, the bevacizumab released from mono-layered capsules could maintain its bioactivity over 90% during the nine-month period, which indicates its potential in protecting the protein. A fluctuation of bioactivity was observed in the bi-layered capsule, which was maintained around 80%. The lower bioactive percentage could be caused by the increased background of the UV-VIS absorbance effect by the slow biodegradation of the inner layer over a long-term period of incubation as aforementioned. However, both results strongly support the high bioactivity of protein protected by the mono-layered capsule and bi-layered capsule. In this regard, the exemplary hollow bi-layered capsule that physically protects the drug has the potential to overcome this barrier to sustained release.

Also, bevacizumab eluted from PCL mono-layered and PCL-chitosan bi-layered capsules was assessed for its inhibitory effect on VEGF-induced tubule growth in a tube formation assay using HUVECs, as shown in FIG. 9. At a concentration of 10 µg/mL, the positive control native bevacizumab caused 93.15 ± 1.49 % tubule length inhibition. The eluted bevacizumab from both mono-layered and bi-layered capsules after one month led to an approximately 13.33 ± 6.51 % and 12.33 ± 4.63 % tube formation of 260 µm inner diameter capsule and 1.645 mm inner diameter capsule, respectively, which was more effective as compared to the conventional injection of native bevacizumab. A slight increase in tube length formation appeared after three months due to the bioactivity loss caused by long-term incubation at physiological temperature. However, there was no significant difference in the anti-angiogenetic properties of eluted bevacizumab from the bi-layered capsule and the one eluted from the mono-layered capsule (p>0.05). It is expected that the slow drug diffusion delayed the process of bevacizumab decomposition by enzymes which significantly protected the protein inside the capsule. Overall, the anti-angiogenic bioactivity was well maintained at a high level over nine months, suggesting the potential protective effects of the capsule toward long-term drug release.

### Injection Feasibility

In addition to the high drug loading capacity, sustainable release of protein therapeutics, and maintaining the bioactivity of anti-VEGF, these capsules can also be made injectable. To demonstrate this, injection feasibility tests were conducted by delivery of capsules of 10 mm length into *ex vivo* porcine vitreous humor via a 21-gauge needle through the sclera, shown in FIG. 10. The capsules with outer diameter of 430 µm were used in this study because they are of similar size to the commercialized intraocular implant, Ozurdex with 460 µm in diameter and 6 mm in length. The Ozurdex applicator is equipped with 22 gauge TSK needle (see Chan, A., L.-S. Leung, and M.S. Blumenkranz, Critical appraisal of the clinical utility of the dexamethasone intravitreal implant (Ozurdex®) for the treatment of macular edema related to branch retinal vein occlusion or central retinal vein occlusion. Clinical Ophthalmology (Auckland, NZ), 2011. 5: p. 1043; Arcinue, C.A., O.M. Cerón, and C.S. Foster, A comparison between the fluocinolone acetonide (Retisert) and dexamethasone (Ozurdex) intravitreal implants in uveitis. Journal of ocular pharmacology and therapeutics, 2013. 29(5): p. 501-507; and Querques, L., et al., Repeated intravitreal dexamethasone implant (Ozurdex®) for retinal vein occlusion. Ophthalmologica, 2013. 229(1): p. 21-25). The inner diameter of the needle is approximately 500 µm, which could fit the exemplary capsule (see Meyer, C.H., et al., Penetration force, geometry, and cutting profile of the novel and old Ozurdex needle: the MONO study. Journal of Ocular Pharmacology and Therapeutics, 2014. 30(5): p. 387-391). More specifically, in clinical applications, the anti-VEGF loaded capsule could be typically delivered by the similar applicator intravitreally which can avoid invasive open surgery. Therefore, the advanced drug delivery system based on the exemplary bi-layered capsules could be quite compatible with the currently used clinical approach.

In this study, to address the critical challenges of long-term therapy for wet AMD treatment, a polymer-based microstructured delivery platform was designed and developed to achieve sustainable release of anti-VEGF *in vitro.* Sustainable protein release was achieved by designing and optimizing the structures of chitosan-PCL bi-layered microcapsules by using a combined materials chemistry and engineering approach. Critical features of these chitosan-PCL microcapsules included: size, porosity of PCL shell, and hollow structures for simple and neat drug loading.

PCL-chitosan microcapsules were synthesized by a novel combination of electrospinning, sintering, and salt leaching. In preliminary studies, it was noted that chitosan fibers lost their structure after salt leaching. It was thought that the formation of trifluoroacetate salts during fiber preparation accelerated the process of dissolution of chitosan while using TFA and DCM as solvents, so a necessary step of neutralization with sodium bicarbonate solution was required during washing to reduce the effect of acidic salts on the bioactivity of bevacizumab (see Sangsanoh, P. and P.J.B. Supaphol, Stability improvement of electrospun chitosan nanofibrous membranes in neutral or weak basic aqueous solutions. 2006. 7(10): p. 2710-2714).

Membrane thickness was correlated to the drug release period. Theoretically, a thicker membrane resulted in slower diffusion of the drug. Even though increasing the size of the capsule could potentially help with achieving a slower drug release, the increased size of microcapsules that would preclude injection through a small gauge needle. Therefore, to make the capsule injectable for clinical application, a thinner membrane was required. A chitosan layer was added to address this problem. In this study, all the capsules had a thickness between 80-95 µm, which minimized the influence of thickness in exploring the relationship between drug release rate and the chitosan-PCL composite.

After optimizing these important factors to control drug release, the performance of the microcapsule was evaluated and optimized for sustainable release of anti-VEGF. Bevacizumab has been used clinically in the treatment of wet AMD since 2004 (see Michels, S., et al., Systemic bevacizumab (Avastin) therapy for neovascular age-related macular degeneration: twelve-week results of an uncontrolled open-label clinical study. 2005. 112(6): p. 1035-1047. e9). Theoretically, the isoelectric point (pI) of bevacizumab is 7.8 (see Nomoto, H., et al., Pharmacokinetics of bevacizumab after topical, subconjunctival, and intravitreal administration in rabbits. 2009. 50(10): p. 4807-4813). Its net charge calculated from the pI should be slightly positive at pH 7.4 which had been reported by numerous studies. However, the protein aggregates in water and other organic solvents typically used during device manufacturing have the potential to reduce bioactivity and cause undesirable side effects (see Varshochian, R., et al., Albuminated PLGA nanoparticles containing bevacizumab intended for ocular neovascularization treatment. Journal of Biomedical Materials Research Part A, 2015. 103(10): p. 3148-3156; Courtois, F., et al., Rational design of therapeutic mAbs against aggregation through protein engineering and incorporation of glycosylation motifs applied to bevacizumab. mAbs, 2016. 8(1): p. 99-112; and Varshochian, R., et al., The protective effect of albumin on bevacizumab activity and stability in PLGA nanoparticles intended for retinal and choroidal neovascularization treatments. European Journal of Pharmaceutical Sciences, 2013. 50(3): p. 341-352). Therefore, PBS is widely used to suspend bevacizumab to maintain its stability and bioactivity. It has been reported that bevacizumab is net negatively charged in PBS at pH 7.4, which suggests a binding to chitosan and may provide a more sustainable release from the exemplary capsule (see Li, S.K., et al., Effective electrophoretic mobilities and charges of anti-VEGF proteins determined by capillary zone electrophoresis. 2011. 55(3): p. 603-607; and Garcia-Quintanilla, L., et al., Pharmacokinetics of Intravitreal Anti-VEGF Drugs in Age-Related Macular Degeneration. Pharmaceutics, 2019. 11(8): p. 365). The binding of buffer ions in PBS to bevacizumab increases its hydrophilicity which further increases its stability and causes the difference between the theoretical and experimental net charge of the protein (see Li, S.K., et al., Effective electrophoretic mobilities and charges of anti-VEGF proteins determined by capillary zone electrophoresis. 2011. 55(3): p. 603-607; and Chopra, P., J. Hao, and S.K.J.I.j.o.p. Li, Iontophoretic transport of charged macromolecules across human sclera. 2010. 388(1-2): p. 107-113). Accordingly, bevacizumab has a negative charge in the vitreous body and capsule; therefore, it is hypothesized that this protein could be retained by positively charged chitosan via electrostatic attraction. Similarly, BSA is a negatively charged protein in water, with an isoelectric point around 4.7. BSA could bind to cationic ions and raise its surface charge under physiological conditions (in PBS). However, the BSA still remains negatively charged in PBS since these ions have less effect on the charge of BSA, as previously reported (see Li, S.K., et al., Effective electrophoretic mobilities and charges of anti-VEGF proteins determined by capillary zone electrophoresis. 2011. 55(3): p. 603-607; and alis, A., et al., Measurements and Theoretical Interpretation of Points of Zero Charge/Potential of BSA Protein. Langmuir, 2011. 27(18): p. 11597-11604). By providing a combined electrostatic interaction between the bevacizumab or BSA and chitosan, and a protective effect from the PCL shell, a desirable sustained drug release profile could be achieved.

In this study, the release rate for a similar payload of bevacizumab was also significantly improved. The average payload of *reported* devices ranged from 500 µg to 1000 µg (see Li, F., et al., Controlled release of bevacizumab through nanospheres for extended treatment of age-related macular degeneration. 2012. 6: p. 54; and Varshochian, R., et al., Albuminated PLGA nanoparticles containing bevacizumab intended for ocular neovascularization treatment. Journal of Biomedical Materials Research Part A, 2015. 103(10): p. 3148-3156). However, these devices had limitations including not being injectable or not sustaining release over three months. The drug loading capacity of these devices was not as expected. Also, the bioactivity of anti-VEGF may be influenced during the fabrication process in these devices due to the interaction of the therapeutic with solvents or high temperatures. However, the drug loading was processed after the device was fabricated, which avoided drug loss and deactivation which commonly occurs using conventional preparation methods such as emulsion. Therefore, the capsules designed herein ensured a drug payload of 700 µg bevacizumab because of the confined space in the injectable capsule and large molecular weight of bevacizumab. The template rod may be selectively increased to enlarge the inner space and enhance drug loading. Also, in the pharmaceutical, the dry powders of bevacizumab could be replaced and loaded precisely under the microscope and further enhance the drug loading efficiency and bevacizumab stability. In addition, other therapeutics with a lower molecular weight comparable to the model drug BSA may be evaluated using the exemplary device and have the potential to further increase the drug payload significantly (see Rosenfeld, P.J., et al., Optical coherence tomography findings after an intravitreal injection of bevacizumab (Avastin®) for neovascular age-related macular degeneration. 2005. 36(4): p. 331-335).

Bi-layered capsules can efficiently control the drug release rate by utilizing the electrostatic interaction between the protein therapeutics and polymers, which can address many of the current problems associated with the clinical treatment of wet AMD. It also provides an alternative method for some diseases which require long-term treatment with protein therapeutics such as colorectal and breast cancers, as well as some brain tumors. However, device manufacturing methods may still need to be optimized for requirements of different protein therapeutics, which could have great potential for ophthalmic, cancer, and other biomedical applications.

In conclusion, a polymer-based delivery platform has been developed for controlled release of anti-VEGF, which is based on a bi-layered microstructure that synergistically combines the electrostatic binding between chitosan and anti-VEGF with a protective hydrophobic layer of PCL, to provide an effective route to modulate polymer-protein interactions for controlled therapeutic release. The bi-layered structure was characterized in detail and further determined capsule performance for protein delivery. Most importantly, the exemplary designed delivery platform significantly improved the long-term release of anti-VEGF *in vitro* compared to most current devices, supporting its potential for treating AMD. In future studies, evaluating and re-optimizing the therapeutic effect of anti-VEGF-loaded devices in an *in vivo* AMD model is required.

## Claims

1. A drug delivery composition comprising:
one or more capsules each having a tubular shape with two ends that are closed, wherein each of the one or more capsules independently comprises a bi-layered wall and at least one luminal compartment; and
one or more therapeutic agents each present within one or more of the at least one luminal compartment;
wherein each bi-layered wall comprises an inner layer and an outer layer;
wherein the inner layer comprises chitosan; and
wherein the outer layer independently comprises a poly(ε-caprolactone) (PCL); and
wherein the one or more therapeutic agents have a net negative charge at any pH within pH 6.0 to pH 7.4.

2. The drug delivery composition of claim 1, wherein the composition is intended for injection into the eye of a subject, preferably wherein the injection is into the vitreous chamber of the eye or wherein the injection is an intravitreal injection, a subconjunctival injection, a subtenon injection, a retrobulbar injection, or a suprachoroidal injection.

3. The drug delivery composition of any one of claims 1-2, wherein each of the one or more capsules has a length from 0.1 cm to 5 cm, preferably from 0.5 cm to 3 cm or from 1 cm to 3 cm.

4. The drug delivery composition of any one of claims 1-3, wherein each of the one or more capsules has an inner diameter from 100 µm to 2000 µm, preferably from 100 µm to 500 µm or from 100 µm to 300 µm.

5. The drug delivery composition of any one of claims 1-4, wherein the bi-layered wall has a wall thickness from 25 µm to 150 µm, preferably from 70 µm to 100 µm, from 75 µm to 95 µm, or from 80 µm to 90 µm.

6. The drug delivery composition of any one of claims 1-5, wherein the outer layer further comprises pores having a pore diameter from 100 nm to 10000 nm, preferably from 350 nm to 650 nm.

7. The drug delivery composition of any one of claims 1-6, wherein the outer layer comprises fibers having a diameter from 100 nm to 2000 nm, preferably from a 500 nm to 1000 nm.

8. The drug delivery composition of any one of claims 1-7, wherein the inner layer comprises fibers having a diameter from 50 nm to 1000 nm, preferably from 100 nm to 400 nm.

9. The drug delivery composition of any one of claims 1-8, wherein the therapeutic agent is an anti-VEGF therapeutic agent, preferably wherein the anti-VEGF therapeutic agent is a therapeutic antibody such as bevacizumab, ranibizumab, or IBI305, a therapeutic protein such as a VEGF decoy receptor such as aflibercept, a tyrosine kinase inhibitor such as lapatinib, sunitinib, sorafenib, axitinib, or pazopanib, or an antisense nucleic acid targeting VEGF or the VEGF receptor.

10. The drug delivery composition of any one of claims 1-9, wherein the therapeutic agent is present in an amount from 0.01 mg to 3 mg, preferably from 0.5 mg to 2 mg or from 0.5 mg to 1.5 mg.

11. The drug delivery composition of any one of claims 1-10, wherein the therapeutic agent exhibits near zero-order release kinetics over a period of at least 30 days, preferably at least 3 months, at least 6 months, or at least 9 months.

12. A drug delivery composition of any one of claims 1-11 for use in therapy, preferably for use in treating an ophthalmological disorder in a subject in need thereof, preferably wherein in treating the ophthalmological disorder a therapeutically effective amount of the drug delivery composition is injected into an eye of the subject.

## Patentansprüche

1. Zusammensetzung zur Medikamentenabgabe, umfassend:
eine oder mehrere Kapseln, die jeweils eine röhrenförmige Form mit zwei verschlossenen Enden aufweisen, wobei jede der einen oder mehreren Kapseln unabhängig voneinander eine zweischichtige Wand und mindestens ein Luminalfach aufweist; und
ein oder mehrere therapeutische Mittel, die sich jeweils in einem oder mehreren der mindestens einen Luminalfächer befinden;
wobei jede zweischichtige Wand eine innere Schicht und eine äußere Schicht umfasst;
wobei die innere Schicht Chitosan umfasst; und
wobei die äußere Schicht unabhängig ein Poly(ε - caprolacton) (PCL) umfasst; und wobei das eine oder die mehreren therapeutischen Mittel eine negative Nettoladung bei einem pH-Wert zwischen 6,0 und 7,4 aufweisen.

2. Zusammensetzung zur Medikamentenabgabe nach Anspruch 1, wobei die Zusammensetzung zur Injektion in das Auge eines Subjekts bestimmt ist, vorzugsweise wobei die Injektion in die Glaskammer des Auges erfolgt oder wobei die Injektion eine intravitreale Injektion, eine subkonjunktivale Injektion, eine Subtenoninjektion, eine retrobulbäre Injektion oder eine suprachoroidale Injektion ist.

3. Zusammensetzung zur Medikamentenabgabe nach einem der Ansprüche 1 bis 2, wobei jede der einen oder mehreren Kapseln eine Länge von 0,1 cm bis 5 cm, vorzugsweise von 0,5 cm bis 3 cm oder von 1 cm bis 3 cm aufweist.

4. Zusammensetzung zur Medikamentenabgabe nach einem der Ansprüche 1 bis 3, wobei jede der einen oder mehreren Kapseln einen Innendurchmesser von 100 µm bis 2000 µm, vorzugsweise von 100 µm bis 500 µm oder von 100 µm bis 300 µm aufweist.

5. Zusammensetzung zur Medikamentenabgabe nach einem der Ansprüche 1 bis 4, wobei die zweischichtige Wand eine Wandstärke von 25 µm bis 150 µm, vorzugsweise von 70 µm bis 100 µm, von 75 µm bis 95 µm oder von 80 µm bis 90 µm aufweist.

6. Zusammensetzung zur Medikamentenabgabe nach einem der Ansprüche 1 bis 5, wobei die äußere Schicht ferner Poren mit einem Porendurchmesser von 100 nm bis 10000 nm, vorzugsweise von 350 nm bis 650 nm, umfasst.

7. Zusammensetzung zur Medikamentenabgabe nach einem der Ansprüche 1 bis 6, wobei die äußere Schicht Fasern mit einem Durchmesser von 100 nm bis 2000 nm, vorzugsweise von 500 nm bis 1000 nm, umfasst.

8. Zusammensetzung zur Medikamentenabgabe nach einem der Ansprüche 1 bis 7, wobei die innere Schicht Fasern mit einem Durchmesser von 50 nm bis 1000 nm, vorzugsweise von 100 nm bis 400 nm, umfasst.

9. Zusammensetzung zur Medikamentenabgabe nach einem der Ansprüche 1 bis 8, wobei das therapeutische Mittel ein therapeutisches Anti-VEGF-Mittel ist, vorzugsweise wobei das therapeutische Anti-VEGF-Mittel ein therapeutischer Antikörper wie Bevacizumab, Ranibizumab oder IBI305 ist, ein therapeutisches Protein wie ein VEGF-Decoy-Rezeptor wie Aflibercept, ein Tyrosinkinase-Inhibitor wie Lapatinib, Sunitinib, Sorafenib, Axitinib oder Pazopanib oder eine Antisense-Nukleinsäure, die auf VEGF oder den VEGF-Rezeptor zielt.

10. Zusammensetzung zur Medikamentenabgabe nach einem der Ansprüche 1 bis 9, wobei das therapeutische Mittel in einer Menge von 0,01 mg bis 3 mg, vorzugsweise von 0,5 mg bis 2 mg oder von 0,5 mg bis 1,5 mg vorhanden ist.

11. Zusammensetzung zur Medikamentenabgabe nach einem der Ansprüche 1 bis 10, wobei das therapeutische Mittel eine Freisetzungskinetik nahe der Nullordnung über einen Zeitraum von mindestens 30 Tagen, vorzugsweise mindestens 3 Monaten, mindestens 6 Monaten oder mindestens 9 Monaten aufweist.

12. Zusammensetzung zur Medikamentenabgabe nach einem der Ansprüche 1 bis 11 zur Verwendung in der Therapie, vorzugsweise zur Verwendung bei der Behandlung einer ophthalmologischen Störung bei einem Subjekt, das diese benötigt, wobei bei der Behandlung der ophthalmologischen Störung eine therapeutisch wirksame Menge der Zusammensetzung zur Medikamentenabgabe in ein Auge des Subjekts injiziert wird.

## Revendications

1. Composition d'administration de médicament comprenant :
une ou plusieurs capsules présentant chacune une forme tubulaire avec deux extrémités qui sont fermées, dans laquelle chacune des une ou plusieurs capsules comprend indépendamment une paroi bilamellaire et au moins un compartiment luminal ; et
un ou plusieurs agents thérapeutiques présentent chacun au sein d'un ou plusieurs de l'au moins un compartiment luminal ;
dans lequel chaque paroi bilamellaire comprend une couche interne et une couche externe ;
dans laquelle la couche interne comprend du chitosane ; et
dans lequel la couche externe comprend indépendamment un poly(s-caprolactone) (PCL) ; et dans laquelle les un ou plusieurs agents thérapeutiques présentent une charge négative nette à un quelconque pH compris entre pH 6,0 et pH 7,4.

2. Composition d'administration de médicament selon la revendication 1, dans laquelle la composition est destinée à être injectée dans l'œil d'un sujet, de préférence dans laquelle l'injection est dans la chambre vitrée de l'œil ou dans laquelle l'injection est une injection intravitréenne, une injection sous-conjonctivale, une injection sous-ténonienne, une injection rétrobulbaire, ou une injection suprachoroïdienne.

3. Composition d'administration de médicament selon l'une quelconque des revendications 1 et 2, dans laquelle chacune des une ou plusieurs capsules présente une longueur de 0,1 cm à 5 cm, de préférence de 0,5 cm à 3 cm ou de 1 cm à 3 cm.

4. Composition d'administration de médicament selon l'une quelconque des revendications 1 à 3, dans laquelle chacune des une ou plusieurs capsules présente un diamètre interne de 100 µm à 2 000 µm, de préférence de 100 µm à 500 µm ou de 100 µm à 300 µm.

5. Composition d'administration de médicament selon l'une quelconque des revendications 1 à 4, dans laquelle la paroi bilamellaire présente une épaisseur de paroi de 25 µm à 150 µm, de préférence de 70 µm à 100 µm, de 75 µm à 95 µm, ou de 80 µm à 90 µm.

6. Composition d'administration de médicament selon l'une quelconque des revendications 1 à 5, dans laquelle la couche externe comprend en outre des pores présentant un diamètre de pore de 100 nm à 10 000 nm, de préférence de 350 nm à 650 nm.

7. Composition d'administration de médicament selon l'une quelconque des revendications 1 à 6, dans laquelle la couche externe comprend des fibres présentant un diamètre de 100 nm à 2 000 nm, de préférence de 500 nm à 1 000 nm.

8. Composition d'administration de médicament selon l'une quelconque des revendications 1 à 7, dans laquelle la couche interne comprend des fibres présentant un diamètre de 50 nm à 1 000 nm, de préférence de 100 nm à 400 nm.

9. Composition d'administration de médicament selon l'une quelconque des revendications 1 à 8, dans laquelle l'agent thérapeutique est un agent thérapeutique anti-VEGF, de préférence dans lequel l'agent thérapeutique anti-VEGF est un anticorps thérapeutique tel que le bevacizumab, le ranibizumab, ou l'IBI305, une protéine thérapeutique telle qu'un récepteur leurre du VEGF tel que l'aflibercept, un inhibiteur de la tyrosine kinase tel que le lapatinib, le sunitinib, le sorafenib, l'axitinib, ou le pazopanib, ou un acide nucléique antisens ciblant le VEGF ou le récepteur du VEGF.

10. Composition d'administration de médicament selon l'une quelconque des revendications 1 à 9, dans laquelle l'agent thérapeutique est présent en une quantité de 0,01 mg à 3 mg, de préférence de 0,5 mg à 2 mg ou de 0,5 mg à 1,5 mg.

11. Composition d'administration de médicament selon l'une quelconque des revendications 1 à 10, dans laquelle l'agent thérapeutique a une cinétique de libération d'ordre quasi nulle sur une période d'au moins 30 jours, de préférence d'au moins 3 mois, d'au moins 6 mois ou d'au moins 9 mois.

12. Composition d'administration de médicament selon l'une quelconque des revendications 1 à 11 destinée à être utilisée en thérapie, de préférence destinée à être utilisée dans le traitement d'un trouble ophtalmologique chez un sujet en ayant besoin, de préférence dans laquelle pour traiter le trouble ophtalmologique une quantité thérapeutiquement efficace de la composition d'administration de médicament est injectée dans un œil du sujet.
